(19) **Europäisches Patentamt / European Patent Office / Office européen des brevets**

(11) **EP 1 558 760 B1**

(12) # EUROPEAN PATENT SPECIFICATION

(45) Date of publication and mention
of the grant of the patent:
**22.12.2010 Bulletin 2010/51**

(21) Application number: **03758054.5**

(22) Date of filing: **23.10.2003**

(51) Int Cl.:
*C12Q 1/68* (2006.01)    *C07K 14/575* (2006.01)

(86) International application number:
**PCT/EP2003/011792**

(87) International publication number:
**WO 2004/039837 (13.05.2004 Gazette 2004/20)**

(54) **CRH RESPONSIVE GENES IN CNS**

GENE DES ZNS, DIE AUF CRH REAGIEREN

GENES REAGISSANT A L'HORMONE LIBERATRICE DE LA CORTICOTROPINE DANS LE SYSTEME NERVEUX CENTRAL

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **31.10.2002 PCT/EP02/12274**

(43) Date of publication of application:
**03.08.2005 Bulletin 2005/31**

(73) Proprietor: **Janssen Pharmaceutica N.V.
2340 Beerse (BE)**

(72) Inventors:
- **PEETERS, Pieter Johan,**
  **Janssen Pharmaceutica N.V.**
  **2340 Beerse (BE)**
- **GÖHLMANN, Hinrich W.H.,**
  **Janssen Pharmaceutica N.V.**
  **2340 Beerse (BE)**
- **SWAGEMAKERS, Sigrid, Maria, Alice**
  **4562 AR Hulst (NL)**
- **KASS, Stefan Ulrich,**
  **Janssen Pharmaceutica N.V.**
  **2340 Beerse (BE)**
- **STECKLER, Thomas H.W.**
  **Janssen Pharmaceutica N.V.**
  **2340 Beerse (BE)**
- **FIERENS, Frederik, L.P.**
  **Janssen Pharmaceutica N.V.**
  **2340 Beerse (BE)**

(56) References cited:
**WO-A2-02/02797**

- **RAJAN VIDYA ET AL: "Cloning, sequencing and tissue-distribution of mouse 11-beta-hydroxysteroid dehydrogenase-1 cDNA" JOURNAL OF STEROID BIOCHEMISTRY AND MOLECULAR BIOLOGY, vol. 52, no. 2, 1995, pages 141-147, XP002407924 ISSN: 0960-0760**
- **OPPERMANN UDO C T ET AL: "Cloning and primary structure of murine 11-beta-hydroxysteroid dehydrogenase/microsomal carbonyl reductase" EUROPEAN JOURNAL OF BIOCHEMISTRY, vol. 227, no. 1-2, 1995, pages 202-208, XP002407925 ISSN: 0014-2956**
- **AGARWAL A K ET AL: "CLONING AND EXPRESSION OF RAT COMPLEMENTARY DNA ENCODING CORTICOSTEROID 11-BETA DEHYDROGENASE" JOURNAL OF BIOLOGICAL CHEMISTRY, vol. 264, no. 32, 1989, pages 18939-18943, XP002407926 ISSN: 0021-9258**
- **KROZOWSKI Z ET AL: "TISSUE-SPECIFIC EXPRESSION OF AN 11-BETA HYDROXYSTEROID DEHYDROGENASE WITH A TRUNCATED N-TERMINAL DOMAIN A POTENTIAL MECHANISM FOR DIFFERENTIAL INTRACELLULAR LOCALIZATION WITHIN MINERALOCORTICOID TARGET CELLS" JOURNAL OF BIOLOGICAL CHEMISTRY, vol. 267, no. 4, 1992, pages 2569-2574, XP002407927 ISSN: 0021-9258**
- **DATABASE EMBL [Online] 16 July 1999 (1999-07-16), XP002249222 retrieved from EBI Database accession no. AI842377**

- BRUNSON K L ET AL: "Corticotropin releasing hormone downregulates CRF1 binding and induces CRF1 gene expression in hippocampus and cortex of the immature rat." SOCIETY FOR NEUROSCIENCE ABSTRACTS, vol. 26, no. 1-2, 2000, pages Abstract No.-807.1, XP001153651 30th Annual Meeting of the Society of Neuroscience;New Orleans, LA, USA; November 04-09, 2000 ISSN: 0190-5295
- DA COSTA A P C ET AL: "Region-specific immediate-early gene expression following the administration of corticotropin-releasing hormone in virgin and lactating rats." BRAIN RESEARCH, vol. 770, no. 1-2, 1997, pages 151-162, XP001153650 ISSN: 0006-8993
- BAKSHI VAISHALI P ET AL: "Corticotropin-releasing hormone and animal models of anxiety: Gene-environment interactions" BIOLOGICAL PSYCHIATRY, ELSEVIER SCIENCE, NEW YORK, NY, US, vol. 48, no. 12, 15 December 2000 (2000-12-15), pages 1175-1198, XP001104031 ISSN: 0006-3223
- VAN GAALEN MARCEL M ET AL: "Effects of transgenic overproduction of CRH on anxiety-like behaviour." EUROPEAN JOURNAL OF NEUROSCIENCE, vol. 15, no. 12, June 2002 (2002-06), pages 2007-2015, XP002249220 June, 2002 ISSN: 0953-816X
- HOLSBOER FLORIAN: "The corticosteroid receptor hypothesis of depression." NEUROPSYCHOPHARMACOLOGY, vol. 23, no. 5, November 2000 (2000-11), pages 477-501, XP002249221 ISSN: 0893-133X

**Description**

The present invention relates generally to therapy and diagnosis of depression. In particular this invention relates to the polypeptides as well as to the polynucleotides encoding these polypeptides, wherein said polypeptides are shown to play a central role in mediating the cellular response to corticotropin releasing hormone. These polypeptides and polynucleotides are useful in the diagnosis, treatment and/or prevention of depression.

BACKGROUND OF THE INVENTION

Recent socioeconomic analyses found that depression is a leading cause of disability and a major risk factor for development of other diseases. Moreover, on a world-wide scale depression is underdiagnosed and undertreated. Current antidepressant drugs have proven to be effective, but are burdened with slow onset of action and side effects. Above this, it is still unclear by which pharmacological mode of action they exert their clinical effects. Hypothesis-driven research based upon the corticosteroid receptor hypothesis of depression has led to a novel concept focusing on brain neuropeptide receptors, specifically the corticotropin-releasing hormone (CRH) receptor as drug target.

Corticotropin releasing hormone (CRH), a 41-amino acid polypeptide plays a central role in the regulation of the hypothalamic-pituitary-adrenal axis, mediating the endocrine responses to various stressors. Hypothalamic neurons release CRH into the hypophyseal portal system in response to stress, stimulating the secretion and biosynthesis of pituitary adrenocorticotropin (ACTH) leading to increased adrenal glucocorticoid production (1). Several clinical and preclinical studies point towards a causal role for alterations in the CRH system in the development of depression (2). The first studies with CRH in humans showed that the ACTH response to CRH is blunted in depressed patients, reflecting a CRH receptor desensitization secondary to continuously increased hypothalamic CRH secretion (3;4). In support of blunted ACTH response as consequence of increased CRH release is the finding of elevated CRH levels in cerebrospinal fluid of patients with depression. Other findings strengthening this notion of CRH hypersecretion in the depressed state are an increased number of CRH secreting neurons and a decreased number of CRH receptors in suicide victims who suffered from depression (5;6).

For CRH two high affinity receptors have been described, CRH-R1 and CRH-R2, both of which exist in several splice variant forms. Activation of these receptors by CRH results in $G_S$-mediated stimulation of adenyl cyclase leading to increased levels of intracellular cAMP. This in itself will activate cAMP dependent protein kinase A (PKA) and ultimately result in increased cytosolic levels of cAMP and $Ca^{2+}$. The increased levels of cAMP and $Ca^{2+}$ lead to the activation of several other additional kinases such as $Ca^{2+}$/calmodulin-dependent kinase II (CAMKII) and p42/p44 mitogen activated kinases (MAPK). As a result the $Ca^{2+}$/cAMP response element binding protein (CREB) is phosporylated and this in turn will regulate the transcription of genes containing cAMP response elements (CRE) in their promoter region. Examples of such genes shown to be involved in the modulation of CRH signaling include c-*fos*, macrophage migration-inhibitory factor gene *Mif*, orphan nuclear receptors *Nurr77* and *Nurr1*.

In order to develop an animal model for chronic pituitary-adrenal activation, CRF overexpressing mice (CRF-OE) have been generated (Stenzel-Poore et al., (1992) Endocrinology 130:3378-3386). These transgenic mice present a Cushingoid phenotype due to lifelong overproduction of ACTH and corticosterone as a result of global overexpression of CRF in these animals. In keeping with the role of brain CRF in mediating endocrine, autonomic and behavioral responses to stress, CRF-OE mice exhibit a spontaneous state of heightened emotionality, are hyper-reactive to stressors and central administration of a CRF receptor antagonist reverses these anxiogenic-like behaviors (Stenzel-Poore et al., (1994) J.Neurosci. 14:2579-2584). These CRF-OE allow the investigation of the long-term effects of CRF on the central nervous system and have been used as a genetic model of anxiety and stress related behavior. Notwithstanding the fact that the downstream pathways for CRH activated receptors were extensively studied and led to the identification of a number of genes involved in the signaling cascade, a major area is unexplored. It was thus an object of the present invention to explore the transcriptional response to CRH stimulation at a genome wide level in order to identify further genes involved in the corticotropin-releasing hormone receptor activated gene network. The polypeptides thus identified and the polynucleotides encoding said polypeptides provide new chances for drug development as drug targets through screening techniques, or are useful in the diagnosis, prevention and/or treatment of depression.

SUMMARY OF THE INVENTION

The present application describes a number of genes that were hitherto not associated with CRH signaling and accordingly useful in methods to identify compounds, which modulate CHR signaling response in a cell or in diagnostic methods to identify CRH induced depression in an individual.

In one embodiment the method to identify a compound capable to alter the CRH signaling response in a cell, in particular the murine pituitary corticotroph-derived adenoma cell line AtT-20 comprises, contacting said cell with CRH in the presence and absence of said compound and determine the expression level of a polynucleotide comprising a nucleic acid sequence selected from the group consisting of SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID No.36, SEQ ID No.38 and SEQ ID No.40. In this screening method the expression levels are typically assessed using an oligonucleotide probe that binds to the aforementioned polynucleotides, preferably using array technology methods. Accordingly in a particular embodiment a method is described to identify compounds that modulate the CRH signaling response in a cell said method comprising, contacting said cell with CRH in the presence and absence of said compound; and determine the expression level of the polynucleotides having the nucleic acid sequences SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO. 36, SEQ ID No.38 and SEQ ID No.40, wherein a change in expression profile of these sequences is indicative for a compound capable to alter the CRH signaling response in said cell.

BRIEF DESCRIPTION OF THE DRAWING

Table 1 : Table of genes shown to be important mediators of CRH induced changes in the CNS.

Table 2 : Sequences of oligonucleotides used for RT-PCR

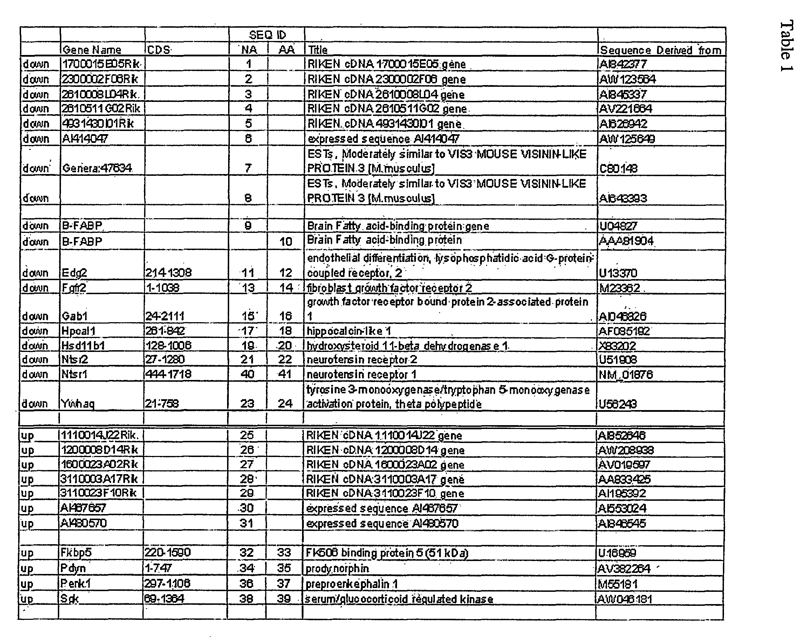

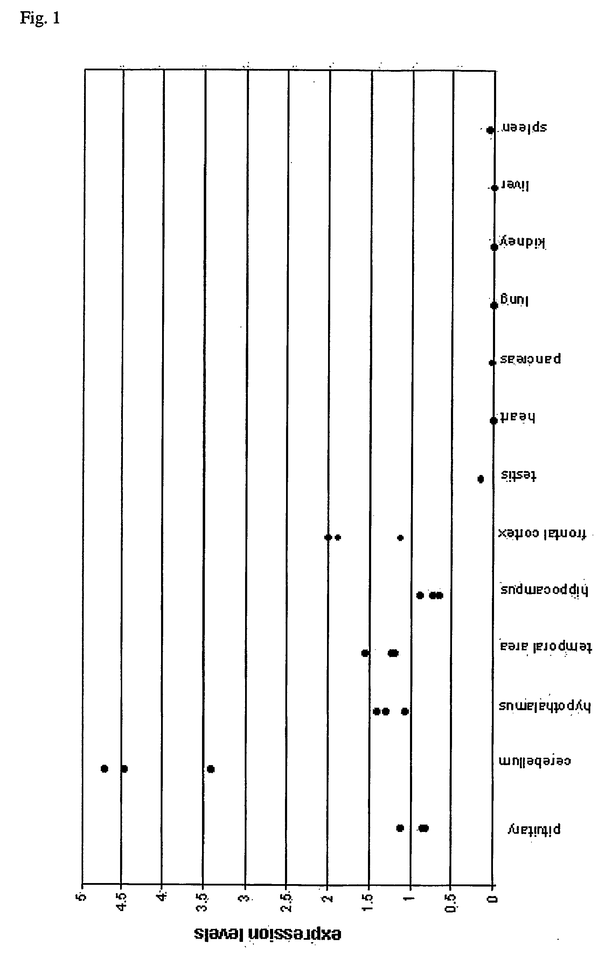

Figure 1: Expression levels of CRH-R1 in different mouse tissues. Quantitative RT-PCR was applied on tissues derived from three different animals for the brain regions.

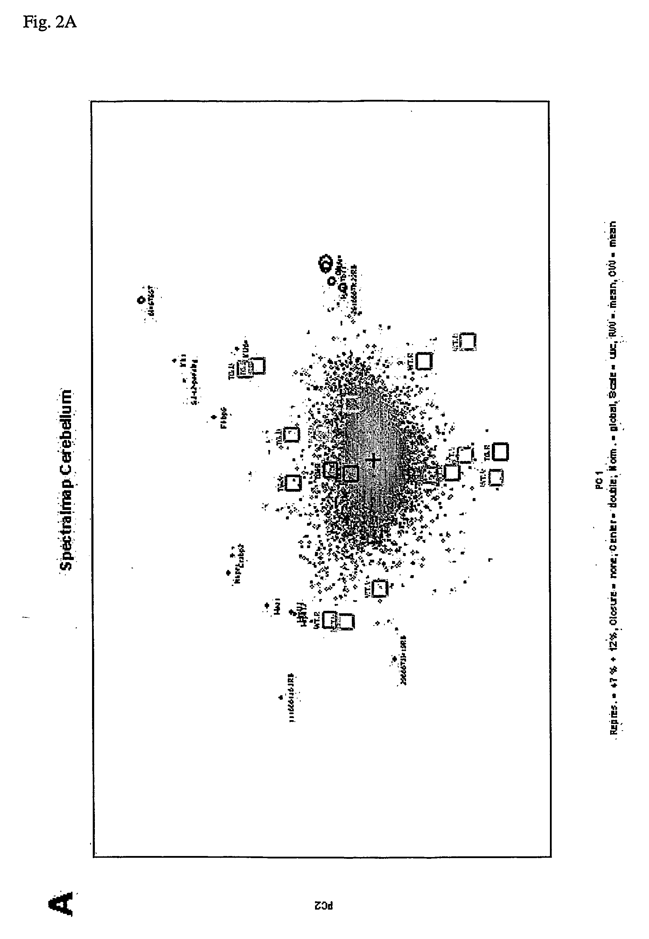

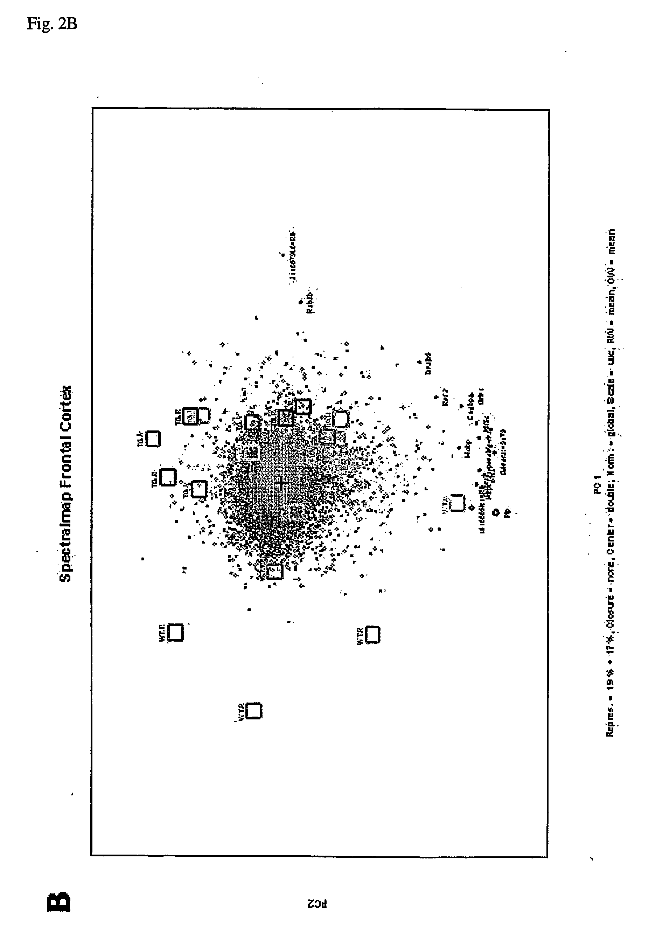

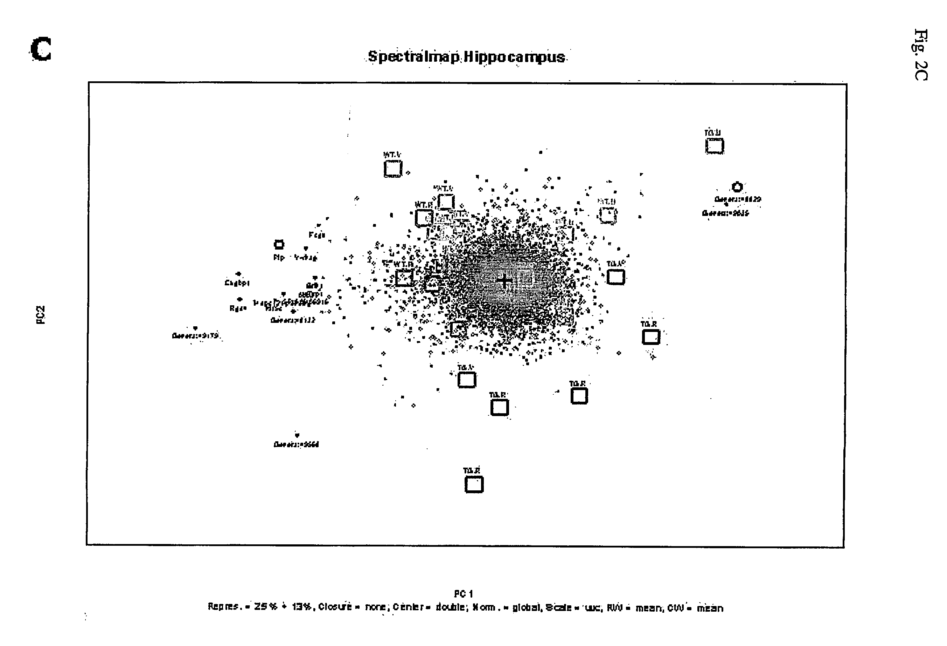

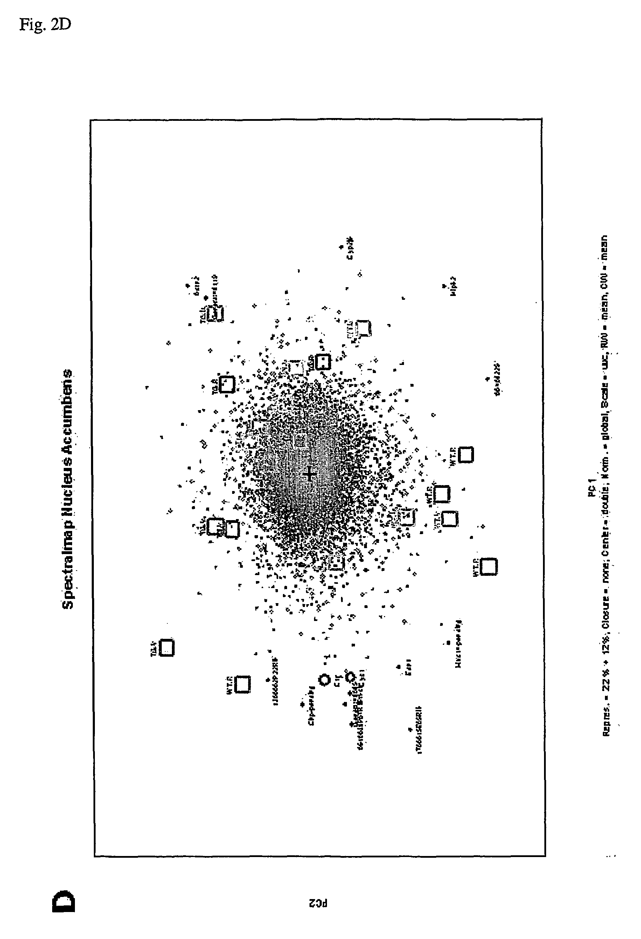

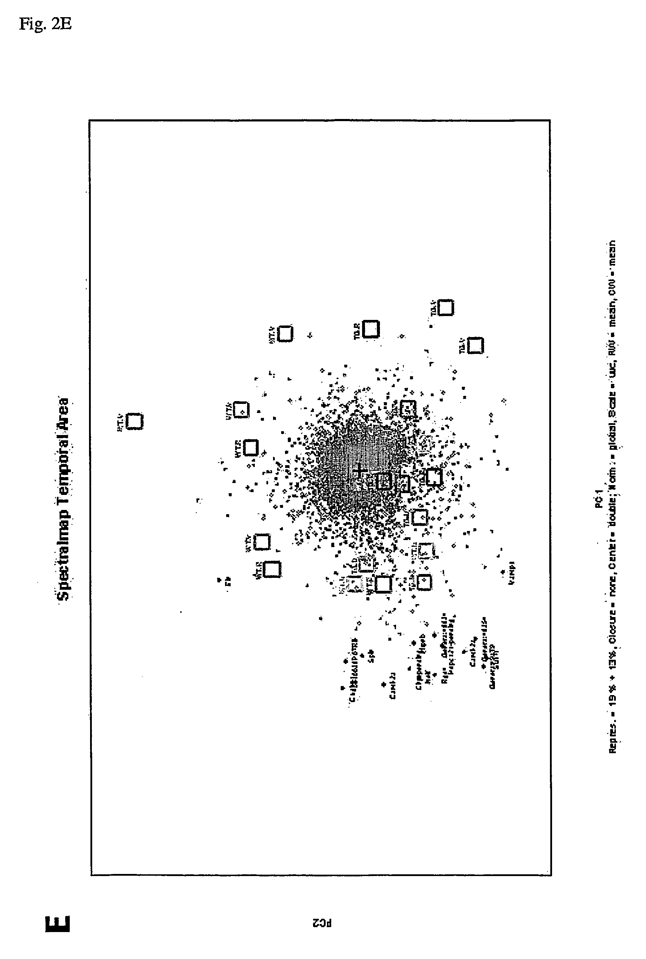

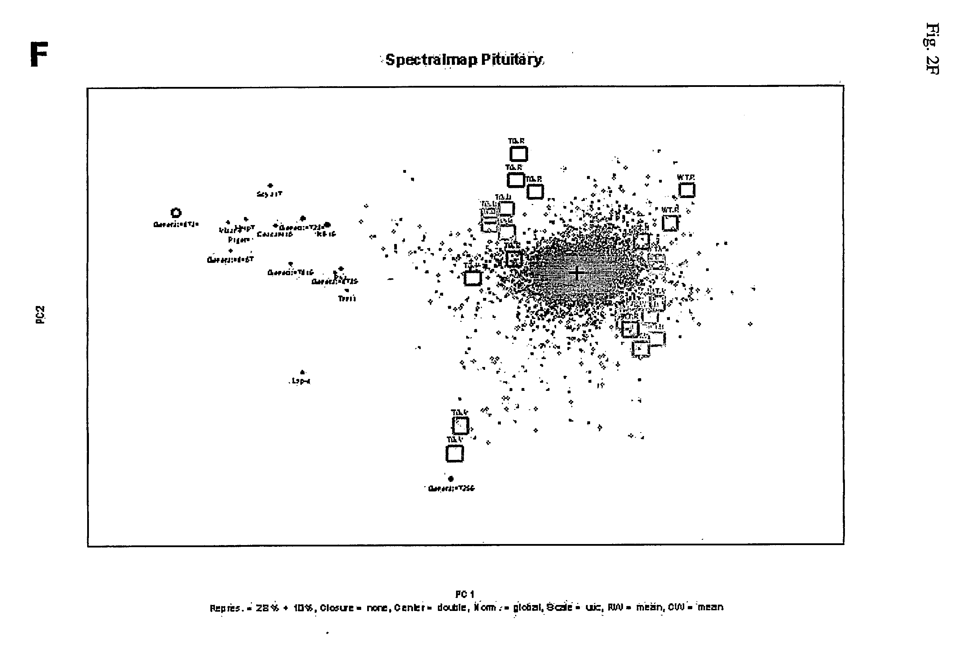

Figure 2: Spectral map analysis of microarray data obtained on all investigated brain areas. Squares depict different samples whereas circles depict genes. The diameter of the circle corresponds to the average intensity of a gene. Distances between squares are a measure for similarity between samples. A positive association of a gene with a given sample (i.e. an upregulation of that gene in that particular sample) results in the positioning of the gene and sample on a common line through the centroid (indicated by a cross). Different areas are shown in the following order (A) cerebellum, (B) frontal cortex, (C) hippocampus, (D) nucleus accumbens, (E) temporal area, (F) pituitary.

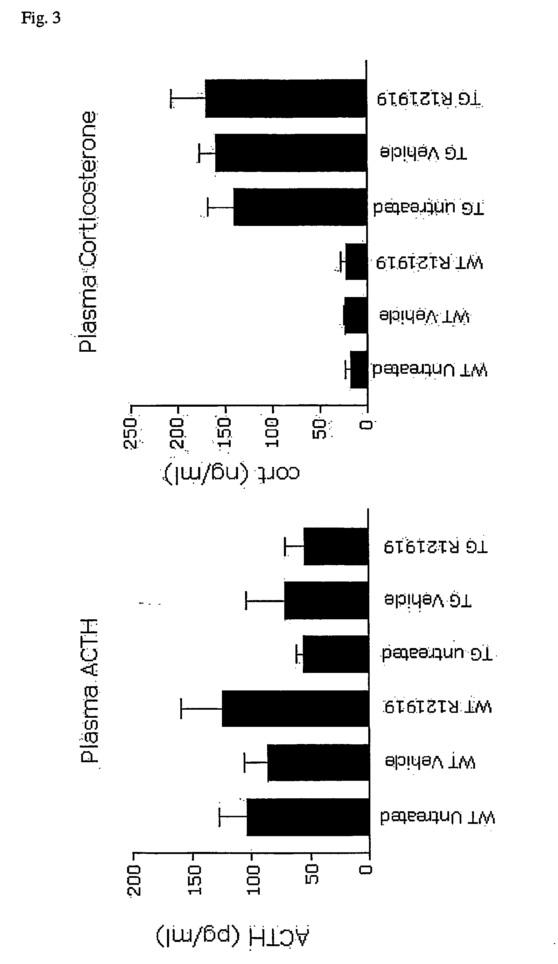

Figure 3: SPA axis parameters in animals used for microarray analysis. Plasma CORT levels are 6 to 8 times higher in CRH overexpressors compared to wild-type animals. No significant differences were observed in ACTH levels.

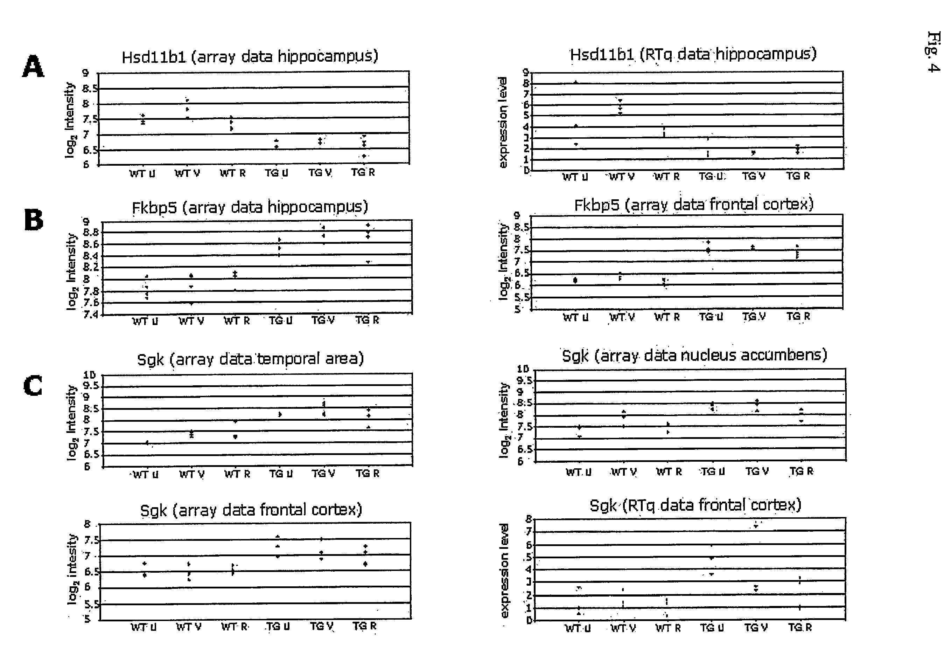

Figure 4: Microarray and quantitative RT-PCR data for genes implicated in glucocorticoid signaling. (A) Array data showing a downregulation of 11β-hydroxysteroid dehydrogenase type 1 (HSD11b1) in hippocampus were confirmed using quantitative RT-PCR (levels normalized against β-actin). (B) Array data showing a significant upregulation in hippocampus and frontal cortex of FK506 binding protein 5 (Fkbp5), a modulator of glucocorticoid receptor activation. (C) Array data on serum/glucocorticoid regulated kinase (Sgk) were confirmed by quantitative RT-PCR in frontal cortex.

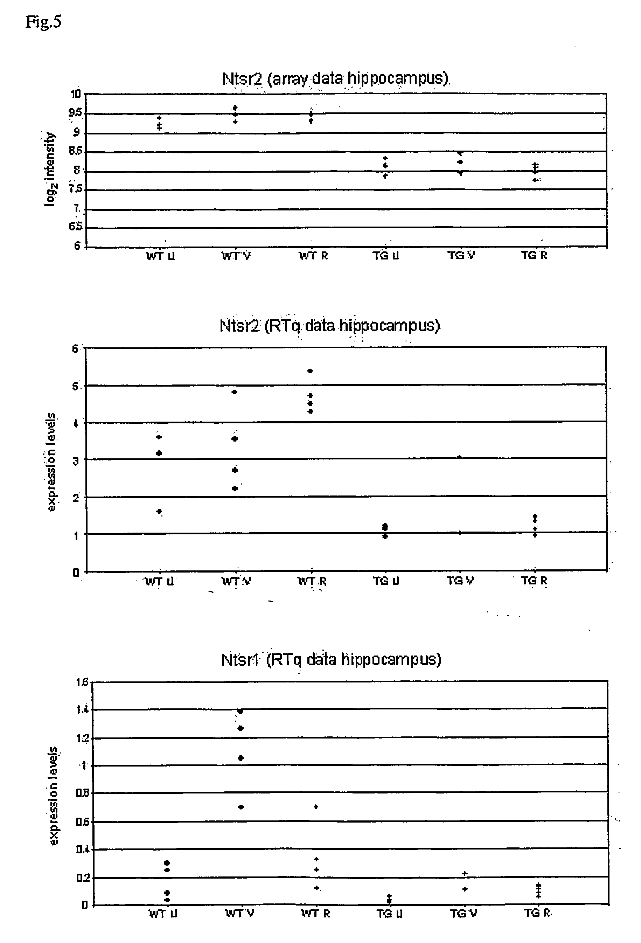

Figure 5: Microarray and quantitative RT-PCR data for neurotensin receptor 1 and 2. (A) Downregulation of neurotensin receptor 2 (Ntsr2) in CRH overexpressing mice observed in hippocampus was established using quantitative RT-PCR. Levels of neurotensin receptor 1 (Ntsr1) were below detection limit in microarray analysis. (B) Quantitative RT-PCR however established a similar downregulation of Ntsr1 in CRH overexpressors. In addition two-way ANOVA on qRT-PCR data shows a significant effect of vehicle treatment which apparently is abolished by treatment with R121919.

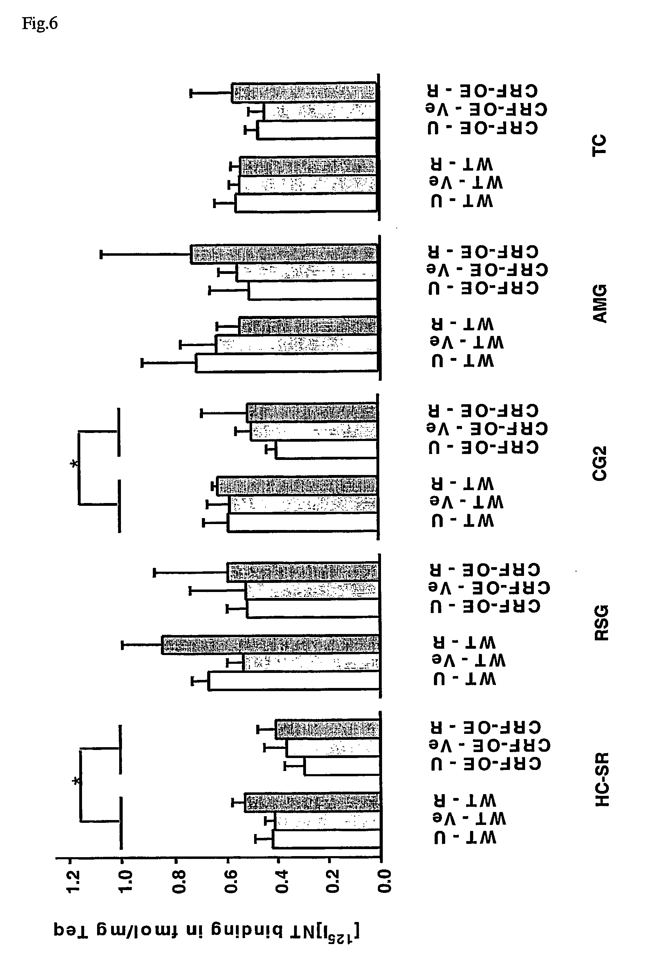

Figure 6: [$^{125}$I]NT binding (0.1 nM, 30 min, RT) to all NTS-sites determined via quantitative autoradiography on frozen glass-mounted brain tissue sections. Data are represented as the average IOD ± SEM of three animals determined on at least three different Bregma sections each.

(*) : difference between the genetic groups with p< 0.05.

AMG, amygdala including ACe, MeA, BMA, BLA; CG2, anterior cingulate cortex; HC-SR, hippocampus - *stratum, radiatum*; RSG, retrosplenial granular cortex; TC, temporal/ parietal cortex; U, untreated; Ve, vehicle-treated; R, R121919-treated.

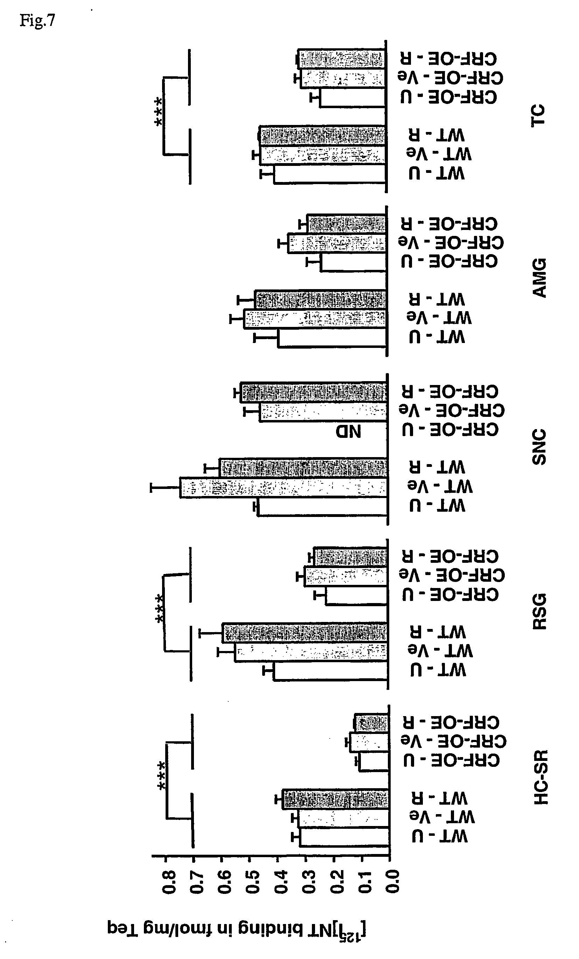

Figure 7: [$^{125}$I]NT binding to NTS1&3-sites on structures within the hippocampal sections with Nts2 sites blocked by Levocabastine. Binding of 0.1 nM [$^{125}$I]NT (30 min, RT) was determined via quantitative autoradiography on frozen glass-mounted brain tissue sections. Data are represented as the average IOD ± SEM of three animals determined on at least three different Bregma sections each.

(***) : difference between the genetic groups with p< 0.001

SNC, *substantia nigra* pars compacta; other abbreviations, see legend Fig. 5.

DETAILED DESCRIPTION

As used herein, the term "compound" or "agent" means a biological or chemical compound such as a simple or complex organic molecule, a peptide, a protein or an oligonucleotide. A "test compound" as used herein, refers to a "compound" or "agent" used in a method according to the invention to assess whether said compounds modulates CRH signalling activity.

"CRH signaling" as used herein refers to the cellular changes in gene transcription after activation of the corticotropin releasing hormone receptor by CRH in said cell. It thus induces a CRH specific gene expression profile. Changes at the transcriptional level can be assessed either at the protein level or at the gene, RNA level.

"CRH response activity" as used herein refers in general to the change of a detectable cellular parameter as a result of the exposure of said cell to CRH. Detectable cellular parameters include amongst others, changes in membrane potential, changes in enzyme activity of an enzyme that modulates CRH signalling a said cell, changes in expression levels of a protein according to the invention or changes in the amount of second messengers such as cGMP, cAMP, $Ca^{2+}$ or $IP_3$.

The term "analog" or "functional analog" refers to a modified form of mammalian purine permeases in which at least one amino acid substitution has been made such that said analog retains substantially the same biological activity as the unmodified mammalian purine permease *in vivo* and/or *in vitro.*

"Sample" or "Biological sample" as used herein refers to cells, cell extracts, body fluids or tissue samples such as from, blood, urine, saliva, tissue biopsy or autopsy material.

The term "functional analog" is intended to include the "fragments," "variants," "degenerate variants," "analogs" and "homologues" or to "chemical derivatives" of the polypeptides according to the invention. Useful chemical derivatives of polypeptide are well known in the art and include, for example covalent modification of reactive organic site contained within the polypeptide with a secondary chemical moiety. Well known cross-linking reagents are useful to react to amino, carboxyl, or aldehyde residues to introduce, for example an affinity tag such as biotin, a fluorescent dye, or to conjugate the polypeptide to a solid phase surface (for example to create an affinity resin)

Variant(s) of polynucleotides or polypeptides, as the term is used herein, are polynucleotides or polypeptides that differ from a reference polynucleotide or polypeptide, respectively. A variant of the polynucleotide may be a naturally occurring variant such as a naturally occurring allelic variant, or it may be a variant that is not known to occur naturally. (1) A polynucleotide that differs in nucleotide sequence from another, reference polynucleotide. Generally, differences are limited so that the nucleotide sequences of the reference and the variant are closely similar overall and, in many regions, identical. As noted below, changes in the nucleotide sequence of the variant may be silent. That is, they may not alter the amino acids encoded by the polynucleotide. Where alterations are limited to silent changes of this type a variant will encode a polypeptide with the same amino acid sequence as the reference. Also as noted below, changes in the nucleotide sequence of the variant may alter the amino acid sequence of a polypeptide encoded by the reference polynucleotide. Such nucleotide changes may result in amino acid substitutions, additions, deletions, fusions and truncations in the polypeptide encoded by the reference sequence, as discussed above. (2) A polypeptide that differs in amino acid sequence from another, reference polypeptide. Generally, differences are limited so that the sequences of the reference and the variant are closely similar overall and, in many regions, identical. A variant and reference polypeptide may differ in amino acid sequence by one or more substitutions, additions, deletions, fusions and truncations, which may be present in any combination.

The terms "complementary" or "complementarity" as used herein refer to the capacity of purine and pyrimidine nucleotides to associate through hydrogen bonding to form double-stranded nucleic acid molecules. The following base pairs are related by complementarity: guanine and cytosine; adenine and thymine; and adenine and uracil. As used herein "complementary" means that the aforementioned relationship applies to substantially all base pairs comprising two single-stranded nucleic acid molecules over the entire length of said molecules. "Partially complementary" refers to the aforementioned relationship in which one of the two single-stranded nucleic acid molecules is shorter in length than the other such that a portion of one of the molecules remains single-stranded.

The term "conservative substitution" or "conservative amino acid substitution" refers to a replacement of one or more amino acid residue(s) in a parent protein without affecting the biological activity of the parent molecule based on the art recognized substitutability of certain amino acids (See e.g. M. Dayhoff, In Atlas of Protein Sequence and Structure, Vol. 5, Supp. 3, pgs 345-352, 1978).

"Fragment thereof" refers to a fragment, piece, or sub-region of a nucleic acid or protein molecule whose sequence is disclosed herein, such that said fragment comprises 5 or more amino acids, or 10 or more nucleotides that are contiguous in the parent protein or nucleic acid molecule.

"Functional fragment" as used herein, refers to an isolated sub-region, or fragment of a protein disclosed herein, or sequence of amino acids that, for example, comprises a functionally distinct region such as an active site for a receptor. Functional fragments may be produced by cloning technology, or as the natural products of alternative splicing mechanims.

The term "homolog" or "homologous" describes the relationship between different nucleic acid molecules or amino acid sequences in which said sequences or molecules are related by partial identity or similarity at one or more blocks or regions within said molecules or sequences. "Isolated nucleic acid compound" refers to any RNA or DNA sequence, however construed or synthesized, which is locationally distinct from its natural location.

A "nucleic acid probe" or "probe" as used herein is a labeled nucleic acid compound which hybridizes with another nucleic acid compound. "Nucleic acid probe" means a single stranded nucleic acid sequence that will hybridize with a single stranded target nucleic acid sequence. A nucleic acid probe may be an oligonucleotide or a nucleotide polymer. A "probe" will usually contain a detectable moiety which may be attached to the end(s) of the probe or be internal to the sequence of the probe.

The term "primer" is a nucleic acid fragment which functions as an initiating substrate for enzymatic or synthetic elongation of, for example, a nucleic acid molecule.

The term "hybridization" as used herein refers to a process in which a single-stranded nucleic acid molecule joints with a complementary strand through nucleotide base pairing.

The term "stringency" refers to hybridization conditions. High stringency conditions disfavor non-homologous base pairing. Low stringency conditions have the opposite effect. Stringency may be altered, for example, by temperature and salt concentration. "Stringent conditions" refers to an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 $\mu$g/ml denatured, sheared salmon sperm DNA, followed by washing the filters in 0.1 x SSC at about 65°C. Further suitable hybridization conditions are described in the examples.

"Lower stringency conditions" include an overnight incubation at 37°C in a solution comprising 6X SSPE (20X SSPE = 3M NaCl; 0.2M NaH$_2$PO4; 0.02M EDTA, pH 7.4), 0.5% SDS, 30% formamide, 100 $\mu$g/ml salmon sperm blocking DNA; followed by washes at 50°C with 1 X SSPE, 0.1% SDS. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include Denhardt's reagent, BLOT-TO, heparin, denatured salmon sperm DNA, and commercially available proprietary formulations. The inclusion of specific blocking reagents may require modification of the hybridization conditions described above, due to problems with compatibility.

The term "fusion protein" as used herein refers to protein constructs that are the result of combining multiple protein domains or linker regions for the purpose of gaining the combined functions of the domains or linker regions. This is may be accomplished by molecular cloning of the nucleotide sequences encoding such domains to produce a new polynucleotide sequence that encodes the desired fusion protein. Alternatively, creation of a fusion protein may be accomplished by chemically joining two proteins.

The term "linker region" or "linker domain" or similar such descriptive terms as used herein refers to polynucleotide or polypeptide sequence that are used in the construction of a cloning vector or fusion protein. Functions of a linker region can include introduction of cloning sites into the nucleotide sequence, introduction of a flexible component or space-creating region between two protein domains, or creation of an affinity tag for specific molecule interaction. A linker region may be introduced into a fusion protein resulting from choices made during polypeptide or nucleotide sequence construction.

*Screening methods*

The present invention relates to screening methods to identify compounds that modulate corticotropin-releasing hormone (CRH) induced depression and stress. It is based on the identification of a number of genes as downstream modulators of the CRH activated CRH receptors. In particular this invention provides a method for identifying a compound capable to alter the CRH signalling response in a cell, said method comprising;

a) contacting said cell with CRH in the presence and absence of said compound;

b) determine the change at transcriptional level of at least one protein that modulates corticotropin releasing hormone (CRH) signaling in said cell; and

c) compare the transcriptional level of said protein in the presence and absence of said compound;

whereby the protein that modulates corticotropin releasing hormone (CRH) signaling is SEQ ID NO.20.

To determine the change of transcription at the protein level one could determine the amount of said protein using art known techniques. For example using separation techniques such as isoelectric focusing or SDS-page in combination with protein staining techniques such as coomassie or silver staining. Alternatively, for proteins that are enzymes, the amount in a given solution or tissue extract can be measured or assayed in terms of the catalytic effect the enzyme produces, that is the conversion of its substrate into reaction product. For example, for kinases one may assess the kinase activity using a substrate comprising the kinase specific phosphorylation site and by measuring the phosphorylation of the substrate by incorporation of radioactive phosphate into the substrate. This assay may be perfomed both in the presence and absence of the compound to be tested. For proteins that are not enzymes, other quantification methods are required. For example transport proteins can be assayed by their binding to the molecule they transport and hormones and toxins by the biological effect they produce.

To assess changes in transcription at the gene level, RNA or cDNA may be used directly for detection or may be amplified enzymatically by using PCR or other amplification techniques prior to analysis. Preferably said analysis method comprises the use of a labelled oligonucleotide probe targeted to a suitable region of the gene encoding a protein that modulates CRH signalling in said cell. Accordingly, in a preferred embodiment the level of gene transcription is assessed using a probe which binds to a polynucleotide encoding the amino acid sequence SEQ ID NO.20.

Also described is an array of oligonucleotides probes comprising a nucleotide sequence encoding a protein that modulates CRH signalling or fragments thereof can be constructed to conduct efficient screening of gene expression. Array technology methods are well known and have general applicability and can be used to address a variety of questions in molecular genetics including gene expression, genetic linkage, and genetic variability (see for example: M.Chee et al., Science, Vol 274, pp 610-613 (1996)).

Also described is a method for identifying a compound capable to alter the CRH signalling response in a cell, comprising;

a) contacting said cell with CRH in the presence and absence of said compound; and
b) determine the expression level of a polynucleotide comprising a nucleic acid sequence selected from the group consisting of SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No. 30, SEQ ID No.31, SEQ ID NO.32, SEQ ID NO.34, SEQ ID NO.36, SEQ ID No.38 and SEQ ID No.40.

To assess changes in expression levels, RNA or cDNA may be used directly for detection or may be amplified enzymatically by using PCR or other amplification techniques prior to analysis. Preferably said analysis method comprises the use of a labelled oligonucleotide probe targeted to a suitable region of the polynucleotiode. Accordingly, in an embodiment the level of gene transcription is assessed using a probe which binds to a polynucleotide comprising a nucleic acid sequence selected from the group consisting of SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO.36, SEQ ID No.38 and SEQ ID No.40.

In another embodiment, an array of oligonucleotides probes comprising a nucleotide sequence encoding a protein that modulates CRH signalling or fragments thereof can be constructed to conduct efficient screening of gene expression. In this embodiment the invention provides a method for identifying a compound capable to alter the CRH signaling response in a cell, said method comprising, contacting said cell with CRH in the presence and absence of said compound; and determine the expression level of the poynucleotides having the nucleic acid sequences SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO.36, SEQ ID No.38 and SEQ ID No.40. In particular, using an array of oligonucleotide probes that bind to the polynucleotides having the nucleic acid sequences SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO. 15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO.36, SEQ ID No. 38 and SEQ ID No.40. Array technology methods are well known and have general applicability and can be used to address a variety of questions in molecular genetics including gene expression, genetic linkage and genetic variability (see for example: M.Chee et al., Science, Vol.274, pp 610-613 (1996)).

In case the genomic DNA is not used directly, mRNA may be isolated, and a first strand cDNA synthesis carried out. A second round of DNA synthesis can be carried out for the production of the second strand. Subsequently by the specific PCR amplification an isolated cDNA can be obtained. If desired the double-stranded cDNA can be cloned into any suitable vector, for example, a plasmid, thereby forming a cDNA libary. In analogy to the above, it is possible to screen cDNA libraries constructed in a bacteriophage or plasmid shuttle vector with a labeled oligonucleotide probe targeted to any suitable region of the gene encoding a protein that modulates CRH signalling. See e.g. PCR Protocols: A Guide to Method and Application, Ed. M. Innis et al., Academic Press (1990)..

Methods for constructing cDNA libraries in a suitable vector such as a plasmid or phage for propagation in prokaryotic or eukaryotic cells are well known to those skilled in the art. [See e.g. Maniatis *et al. Supra*]. Suitable cloning vectors are well known and are widely available.

In a further embodiment changes in gene transcription are determined at mRNA level. Decreased or increased expression can be measured at the RNA level using any of the methods well known in the art for the quantitation of polynucleotides, such as, for example; nucleic acid amplification, for instance via PCR, RT-PCR; RNase protection; Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays. Assay techniques that can be used to determine the presence of protein derivatives or variants comprise amongst others mass spectrometry.

It is thus an object of the present invention to provide a method for identifying a compound capable to alter the CRH signalling response in a cell, said method comprising;

a) contacting said cell with CRH in the presence and absence of said compound;

b) determine the amount of at least one protein that modulates corticotropin releasing hormone (CRH) signaling in said cell; and

c) compare the amount of said protein in the presence and absence of said compound;

whereby the protein that modulates corticotropin releasing hormone (CRH) signaling is SEQ ID NO.20.

Preferably, the method to assay the amount of protein that modulates CRH signaling is using an antibody which binds to a polypeptide comprising the amino acid sequence SEQ ID NO.20.

Thus in another embodiment, this invention provides a monospecific antibody immunologically reactive with a protein that modulates CRH signalling said protein being SEQ ID NO.20.

Antibodies generated against polypeptides of the present invention may be obtained by administering the polypeptides or epitope-bearing fragments, analogs or cells expressing these to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975)256:495-497), the trioma technique, the human B-cell hybridoma technique (Kozbor et al., Immunology Today (1983)4:72) and the EBV-hybridoma technique (Cole et al., MONOCLONAL ANTIBODIES AND CANCER THERAPY, pp.77-96, Alan R. Liss, Inc., 1985).

Techniques for the production of single chain antibodies, such as those described in U.S. Patent No.4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography.

Antibodies against polypeptides of the present invention may also be employed to treat the CRH metabolism related disorders such as CRH induced stress or depression amongst others.

To determine the amount of protein that modulates CRH signalling, the antibodies according to the invention are used in conventional immunological techniques. Suitable immunological techniques are well known to those skilled in the art and include for example, ELISA, Western Blot analysis, competitive or sandwich immunoassays and the like, as is otherwise well known they all depend on the formation of an antigen-antibody immune complex wherein for the purpose of the assay, the antibody can be detectable labeled with, e.g. radio, enzyme or fluorescent labels or it can be immobilized on insoluble carriers.

For example in an ELISA screening format the antibody is added to a solid phase (for example the bottom of a microplate) which is coated with either the protein or a peptide fragment thereof coupled to a carrier (such as BSA), and then, adding an anti-immunoglobin antibody (for example when the immunization is performed in mice, an anti-mouse immunoglobulin antibody is used, e.g. sheep-anti-mouse immunoglobulin (Ig)) conjugated with a detectable label such as an enzyme, preferably horseradish peroxidase, or a radioactive isotope such as $^{125}$I.

It is thus an object of the invention to provide **immunoassays** for the determination or detection of proteins that modulate CRH signalling in a sample, the method comprising contacting the sample with an antibody to the proteins according to the invention and determining whether an immune complex is formed between the antibody and said protein. These methods can either be performed on **tissue samples** or **body fluid** samples and generally comprise obtaining a sample from the body of a subject; contacting said sample with an imaging effective amount of a detectably labeled antibody according to the invention; and detecting the label to establish the presence of proteins that modulate CRH signalling in the sample.

The measuring methods using the antibodies of the present invention are not particularly limited. Any measuring method may be used as long as the amount of antibodies, antigens or the antigens-antibody complexes corresponding to the amount of the antigens to be measured is detected by chemical or physical means, and calculated from standard curves prepared by the use of standard solutions containing the antigens in known amounts. For example, nephelometry, competitive methods, immunometric methods and sandwich methods are suitably used. With respect to sensitivity and specificity, it is particularly preferred to use sandwich methods described below.

In measuring methods using **labelling substances,** radioisotopes, enzymes, fluorescent substances, luminous substances, etc. are used as labelling agents. Examples of the radioisotopes include $^{125}$I, $^{131}$I, $^{3}$H and $^{14}$C. Enzymes are usually made detectable by conjugation of an appropriate substrate that, in turn catalyzes a detectable reaction. Examples thereof include, for example, beta-galactosidase, beta-glucosidase, alkaline phosphatase, peroxidase and malate deydrogenase, preferably horseradish peroxidase. The luminous substances include, for example, luminol, luminol derivatives, luciferin, aequorin and luciferase. Further, the avidin-biotin systems can also be used for labelling the antibodies and immunogens of the present invention.

Accordingly, in a further aspect, the present invention provides for a method of identifying and obtaining compounds that alter the CRH signalling response activity in a cell, comprising:

a) contacting a cell which expresses at least one protein comprising the amino acid sequence SEQ ID NO.20, with said test compound; and

b) compare the CRH response activity of said cell in the presence and absence of said compound.

Changes in membrane potential can be measured using conventional electrophysiological techniques and when they become available, using novel high throughput methods currently under development. Since the change in membrane potential are normally the result of ion fluxes, as an alternative approach, changes in membrane potential can be measured indirectly through the change in intracellular ion concentrations using ion-sensitive fluorescent dyes, including fluo-3, fluo-4, fluo-5N, fura red, Sodium Green, SBFI and other similar probes from suppliers including Molecular Probes. Other fluorescent dyes, from suppliers including Molecular Probes, such as DIBAC $_{4\ (3)}$ or Di-4-Anepps can detect membrane potential changes. For example calcium and sodium ion fluxes can thus be characterised in real time, using fluorometric and fluorescence imaging techniques, including fluorescence microscopy with or without laser confocal methods combined with image analysis algorithms.

In a preferred embodiment this assay is based around an instrument called a **FL**uorescence **I**maging **P**late **R**eader ((FLIPR), Molecular Devices Corporation). In its most common configuration, it excites and measures fluorescence emitted by fluorescein-based dyes. It uses an argon-ion laser to produce high power excitation at 488 nm of a fluorophore, a system of optics to rapidly scan the over the bottom of a 96-/384-well plate and a sensitive, cooled CCD camera to capture the emitted fluorescence. It also contains a 96-/384-well pipetting head allowing the instrument to deliver solutions of test agents into the wells of a 96-/384-well plate. The FLIPR assay is designed to measure fluorescence signals from populations of cells before, during and after addition of compounds, in real time, from all 96-/384-wells simultaneously.

It is thus an object of the present invention to provide a FLIPR assay used to screen for and characterise compounds functionally active in modulating CRH response in cells, said cells expressing the protein SEQ ID NO.20.

In an alternative embodiment, the activity of the cell may be assessed using electrophysiological methods. Therefore, proteins modulating CRH signalling in a cell can be characterised using whole cell and single channel electrophysiology.

It is thus a further object of this invention to provide a screening method to identify compounds which modulate CRH signalling response activity in a cell, said method comprising;

(a) contacting a host cell expressing the protein SEQ ID NO.20, with a compound to be tested;
(b) measuring the effect of the test compound on the membrane potential of said cell using electrophysiological techniques; and
(c) compare the CRH response activity of said cell in the presence and absence of said compound.

In a preferred embodiment the host cells are Xenopus oocytes and the electrophysiological measurement consists of measuring the membrane current using the voltage clamp technique at distinct membrane potentials. Changes in enzyme activity of an enzyme that modulates CRH signalling a said cell, can generally be measured or assayed in terms of the catalytic effect the enzyme produces, that is the conversion of its substrate into reaction product. For example, for kinases one may assess the kinase activity using a substrate comprising the kinase specific phosphorylation site and by measuring the phosphorylation of the substrate. Similarly for phosphatases one may assess the phosphatase activity using a phosphorylated substrate and by measuring the dephosphorylation of the substrate. These assays may be performed both in the presence and absence of the compound to be tested.

It is thus an object of the present invention to provide a method for identifying a compound capable to alter the CRH signalling response activity in a cell, said method comprising;

a) contacting a mixture comprising the kinase SEQ ID NO.20, a source of phosphate and a suitable kinase substrate;
b) incubating said mixture in the presence or absence of a said compound and ;

measuring the level of phosphorylation of said substrate in the presence of said compound compared to the level of phosphorylation of said substrate in the absence of said test compound.

In the assay of the invention, the kinase may be provided as a protein or it may be provided in the assay mixture as an mRNA encoding said kinase. When the assay comprises cell-free components, the kinase is provided as the protein. When the assay is conducted in the milieu of a cell, the kinase may be provided as either the protein or as an mRNA encoding said kinase, wherein, in order that the kinase be available in the assay, the mRNA is translated and kinase protein is thereby produced. It will be apparent from the Examples provided herein that it is a simple matter to obtain mRNA specifying the kinase and inject the mRNA into a cell for production of the kinase protein. The kinase may also be provided by expression of a plasmid, which encodes the kinase protein. Standard molecular biology techniques may be used to construct operable plasmids encoding the kinase protein and to express the plasmid in cells s (Sambrook, et al., 1989, In: Molecular Cloning: A Laboratory Manual, Cold Spring Harbor Laboratory, New York).

As discussed herein, the method of identifying a kinase modulator may be performed either in vitro wherein the assay mixture is cell-free, in vitro wherein live cells are included in the assay, or in vivo in an animal. Thus, in one aspect of the invention, the mixture is contained within a eukaryotic cell and the method of the invention may be performed wherein some of the components of the assay mixture may be provided exogenously to a cell my microinjection of the components therein, and some of the components may be endogenous in the cell.

The term "endogenous in the cell" as used herein, means that the component is naturally produced in the subject cell.

The term "exogenous to the cell" as used herein, means that the component is not found naturally in the subject cell, or is found therein at a low level, and is added thereto. When the method of the invention is performed using a eukaryotic cell, one or more of the kinase protein, the kinase substrate and the test compound may be injected into the eukaryotic cell prior to the incubation. The cell so injected is then incubated under conditions that facilitate protein kinase activity and the level of protein kinase activity is subsequently measured following the incubation period using the assays described herein.

The eukaryotic cell that is useful in the methods of the invention may be any one of a Xenopus laevis oocyte, a Xenopus laevis embryo cell, a mammalian cell (such as a I OTI/2 cell), a Drosophila melanogaster S2 cell, a Dictyostefium discoideum cell and a yeast cell. Still more preferably, the eukaryotic cell is the murine pituitary corticotroph-derived adenoma cell line cell AtT-20.

The source of phosphate for use in the methods of the invention may be any common source of phosphate, including, but not limited to, a nucleotide triphosphates such as, but not limited to, ATP or GTP. In a preferred embodiment, the phosphate source has bound thereon a detectable label which label is transferred with the phosphate group to the kinase substrate during the reaction. In this manner, phosphorylated kinase substrate may be distinguished from non-phosphorylated kinase substrate in that the phosphorylated substrate will contain the detectable label whereas the non-phosphorylated substrate will not contain the label. In another embodiment, the phosphate source does not have bound thereon a detectable label; instead, phosphorylated kinase substrate may be distinguished from non- phosphorylated kinase substrate, for instance by recognition of one form of the substrate, but not the other, by an antibody.

The detectable label which is useful in the methods of the invention may include any known or heretofore unknown detectable label which is transferred to the kinase substrate upon transfer of a phosphate group thereto as a result of protein kinase activity. Labels which are useful include, but are not limited to, radioactive labels, such as $\gamma^{32}P$, $^{31}S$, and non-radioactive labels, such as biotin and the like.

Preferably the kinase assay is performed in a cell and comprising;

a) contacting a cell comprising the kinase SEQ ID NO.20, with a source of phosphate and a suitable kinase substrate;
b) incubating said mixture in the presence or absence of a said compound and ;
c) compare the CRH response activity of said cell in the presence and absence of said compound.

Similarly, a change in CRH signaling response may be assessed using a phosphatase assay, said assay comprising;

a) contacting a mixture comprising the phosphatase SEQ ID NO.20, and a suitable phosphorylated substrate;
b) incubating said mixture in the presence or absence of a said compound and ;

measuring the level of phosphorylation of said substrate in the presence of said compound compared to the level of phosphorylation of said substrate in the absence of said test compound.

As for the kinase assay, the phosphatase in the assay of the invention, may be provided as a protein or it may be provided in the assay mixture as an mRNA encoding said phosphatase. The phosphorylated substrate is typically labeled with a detectable phosphate residue. Labels which are useful include, but are not limited to, radioactive labels, such as $\gamma^{32}$P, $^{31}$S, and non-radioactive labels, such as biotin and the like. For use in a phosphatase activity assay, the substrate preferably consists of a peptide substrate, phosphorylated at a tyrosine or serine residue, typically labeled with $\gamma^{32}$P. In general, phosphorylation may be accomplished in a variety of ways. Typically, a protein tyrosine kinase is used. For example, a soluble EGF-receptor kinase in combination with sup.32 P-labeled ATP may be used to phosphorylate a tyrosine residue on a peptide of the present invention. Such a phosphorylation reaction is typically allowed to proceed for about 2 hours at 30.degree. C. or overnight at room temperature. Phosphorylated peptide, hereinafter referred to as "phosphopeptide", is then purified from a phosphorylation reaction mixture. For example, peptide may be separated from a reaction mixture by addition of trichloroacetic acid and centrifugation, whereby the peptide remains in the supernate. The peptide is generally further purified by column chromatography, e.g. , on C18. Purified phosphorylated peptide may be lyophilized and stored at - 20.degree. C. prior to use.

Following incubation, phosphopeptide which is not dephosphorylated ("non-dephosphorylated phosphopeptide") is separated from radioactivity released by dephosphorylation of phosphopeptide (i.e., from free radioactive phosphorus released by dephosphorylation). As used herein, the term "radioactive phosphorous" includes all forms in which a radioactive phosphorus atom may be present on a tyrosine residue and removed by dephosphorylation, e.g., as a phosphate group. Typically, separation of non-dephosphorylated phosphopeptide from free radioactive phosphorus released by dephosphorylation of phosphopeptide is effected by centrifugation, following termination of the dephosphorylation reaction by the addition of substances including nonradioactive phosphates and charcoal. Radioactivity in the supernate is determined by means well known to those of ordinary skill in the art. Based upon the amount of radioactivity added to the assay mixture initially via the phosphopeptide and the amount of radioactivity detected at the end of the assay as radioactivity released by dephosphorylation, the phosphatase enzymatic activity of the sample assayed may be calculated.

Preferably the phosphatase assay is performed in a cell and comprising;

  a) contacting a cell comprising the phosphatase SEQ ID NO.20, with a suitable phosphorylated substrate;
  b) incubating said mixture in the presence or absence of a said compound and ;
  c) compare the CRH response activity of said cell in the presence and absence of said compound, wherein the CRH response activity of said cell is determined by the change in phosphorylation level of the substrate.

It is also an embodiment of the present invention to provide a method for identifying a compound capable to alter the CRH signalling response activity in a cell, said method comprising;

  a) contacting a cell which expresses at least one protein comprising the amino acid SEQ ID NO.20 with said test compound; and
  b) compare the levels of a second messenger, such as cAMP, cGMP, Ca$^{2+}$ or IP$_3$ in said cell, in the presence and absence of said compound.

Levels of second messengers can be determined using art known techniques either in whole cells or cellular extracts comprising one of the aforementioned proteins.

A further method to identify a compound capable to alter CRH signalling in a cell is based on the use of a gene, such as a reporter gene, operably linked to a gene promoter or regulatory sequence element thereof characterized in that said gene promoter or regulatory sequence element comprises a transcription factor binding site, wherein said transcription factor is capable of modulating CRH signalling in a cell. In a preferred embodiment the transcription factor capable of modulating CRH signalling in a cell is SEQ ID NO.20.

Accordingly, the present invention provides a recombinant DNA molecule comprising the gene promoter region as defined above. In the said recombinant DNA molecule, the promoter region can be operably linked to a nucleic acid molecule encoding a detectable product, such as a reporter gene. The term "operably linked", as used herein, means functionally fusing a promoter with a gene in the proper frame to express the gene under control of the promoter. As used herein, the term "reporter gene" means a gene encoding a gene product that can be identified using simple, inexpensive methods or reagents and that can be operably linked to the promoter region or an active fragment thereof. Reporter genes such as, for example, a firefly luciferase, β-galactosidase, alkaline phosphatase, the bacterial chloramphenicol acetyl transferase or green fluorescent protein reporter gene, can be used to determine transcriptional activity in screening assays according to the invention (see, for example, Goeddel (ed.), Methods Enzymol., Vol. 185, San Diego:Academic Press, Inc. (1990); see also Sambrook, supra). In a preferred embodiment, the reporter gene is the firefly luciferase gene. The invention also provides a vector comprising the recombinant DNA molecule as defined above, as well as a host cell stably transformed with such a vector, or generally with the recombinant DNA molecule according to the invention. The term "vector" refers to any carrier of exogenous DNA that is useful for transferring the DNA into a host cell for replication and/or appropriate expression of the exogenous DNA by the host cell. Accordingly, in a specific embodiment said vector is an expression vector such as the commercially available pGL3luc expression vector.

*Identification of a CRH induced gene expression profile.*

In another aspect the present invention relates to the use of the isolated and purified nucleic acid molecules which were shown to be either up or down-regulated upon prolonged exposure to elevated levels of CRH, wherein said nucleic acid molecule is either RNA, DNA, cDNA or genomic DNA, as a marker of CRH signaling in a cell

In particular, the present application describes an isolated and purified nucleic acid molecule comprising a member selected from a group consisting of:

a) a nucleotide sequence which has at least 70% identity, preferably at least 80% identity, more preferably at least 90% identity, more preferably at least 90% identity, Yet more preferably at least 90% identity, yet more preferably at least 95% identity, even more preferably at least 97-99% identity, to a polynucleotide selected from the group consisting of SEQ ID No.1, SEQ ID No.2, SEQ ID No.3, SEQ ID No.4, SEQ ID No.5, SEQ ID No.6, SEQ ID No.7, SEQ D No.8, SEQ ID No.25, SEQ ID No.26, SEQ ID No.27, SEQ ID No.28, SEQ ID No.29, SEQ ID No.30 and SEQ ID Neo.31, over the entire length of said polynucleotide;

b) a nucleotide sequence which has at least 70% identity, preferably at least 80% identity, more preferably at least 90% identity, more preferably at least 90% identity, Yet more preferably at least 90% identity, yet more preferably at least 95% identity, even more preferably at least 97-99% identity, to a polynucleotide selected from the group consisting of SEQ ID No.1, SEQ ID No.2, SEQ ID No.3, SEQ ID No.4, SEQ ID No.5, SEQ ID No.6, SEQ ID No.7, SEQ D No.8, SEQ ID No.25, SEQ ID No.26, SEQ ID No.27, SEQ ID No.28, SEQ ID No.29, SEQ ID No.30 and SEQ ID No.31, over the entire coding region of said polynucleotide;

c) a nucleotide sequence polynucleotide selected from the group consisting of SEQ ID No.1, SEQ ID No.2, SEQ ID No.3, SEQ ID No.4, SEQ ID No.5, SEQ ID No.6, SEQ ID No.7, SEQ D No.8, SEQ ID No.25, SEQ ID No.26, SEQ ID No.27, SEQ ID No.28, SEQ ID No.29, SEQ ID No.30 and SEQ ID No.31;

Identity or similarity, as known in the art, are relationships between two or more polypeptide sequences or two or more polynucleotide sequences, as determined by comparing the sequences. In the art, identity also means the degree of sequence relatedness between polypeptide or polynucleotide sequences, as the case may be, as determined by the match between strings of such sequences. Both identity and similarity can be readily calculated (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity and similarity between two polynucleotide or two polypeptide sequences, both terms are well known to skilled artisans (Sequence Analysis Primer, Gribskov, M. and Devereux, J., eds., M Stockton Press, New York, 1991; and Carillo, H., and Lipman, D., (1988) SIAM J. Applied Math., 48, 1073. Methods commonly employed to determine identity or similarity between sequences include, but are not limited to those disclosed in Carillo, H., and Lipman, D., (1988) SIAM J. Applied Math., 48, 1073. Preferred methods to determine identity are designed to give the largest match between the sequences tested. Methods to determine identity and similarity are codified in computer programs. Preferred computer program methods to determine identity and similarity between two sequences include, but are not limited to, GCG program package (Devereux, J., et al., (1984) Nucleic Acids Research 12(1), 387), BLASTP, BLASTN, and FASTA (Atschul, S. F. et al., (1990) J. Molec. Biol. 215, 403).

The nucleic acid sequence encoding a protein capable of modulating CRH activity, or fragment thereof, can be isolated from a tissue in which said gene is expressed, such as but not limited to, brain, hart, kidney, pancreas, liver and skin. Said sequence can also be isolated from mammals other than human and mouse. Selection of suitable cells may be done by screening for CRH modulating activity in cell extracts or in whole cell assays, as described herein. Cells that possess CRH modulating activity in any one of these assays may be suitable for the isolation of nucleic acid sequences according to the invention.

Any of a variety of procedures known in the art may be used to molecularly clone DNA encoding a protein according to the invention. In one method, mRNA is isolated, and first strand cDNA synthesis is carried out. A second round of DNA synthesis can be carried out for the production of the second strand. Subsequently by the specific PCR amplification of DNA fragments through the design of degenerate oligonucleotide primers from the amino acid sequence of the purified protein that modulates CRH signalling, an isolated cDNA can be obtained. If desired the double-stranded cDNA can be cloned into any suitable vector, for example, a plasmid, thereby forming a cDNA libary. Another method is to screen cDNA libraries constructed in a bacteriophage or plasmid shuttle vector with a labeled oligonucleotide probe targeted to any suitable region of SEQ ID NO: 1, SEQ ID NO 7 or SEQ ID NO:3. See e.g. PCR Protocols: A Guide to Method and Application, Ed. M. Innis et al., Academic Press (1990)..

Methods for constructing cDNA libraries in a suitable vector such as a plasmid or phage for propagation in prokaryotic or eukaryotic cells are well known to those skilled in the art. [See e.g. Maniatis *et al. Supra*]. Suitable cloning vectors are well known and are widely available.

It is readily apparent to those skilled in the art that other types of libraries, as well as libraries constructed from other cells or cell types, may be useful for isolating the nucleic acid sequences according to the invention. Other types of libraries include, but are not limited to, cDNA libraries derived from other cells, from organisms other than human and mouse, and genomic DNA libraries that include YAC (yeast artificial chromosome) and cosmid libraries. Construction of genomic DNA libraries can be performed by standard techniques well known in the art. Well known genomic DNA library construction techniques can be found in T. Maniatis et al. Molecular Cloning: A Laboratory Manual, 2d Ed. Chap. 14 (1989).

The skilled artisan will appreciate that, in many cases, an isolated cDNA sequence will be incomplete, in that the region coding for the polypeptide is short at the 5' end of the cDNA. This is a consequence of reverse transcriptase, an enzyme with inherently low 'processivity' (a measure of the ability of the enzyme to remain attached to the template during the polymerisation reaction), failing to complete a DNA copy of the mRNA template during the 1st strand cDNA synthesis.

There are several methods available and well known to those skilled in the art to obtain full-length cDNAs, or extend short cDNAs, for example those based on the method of Rapid Amplification of cDNA ends (RACE) (Frohman et al., 1988, PNAS USA 85, 8998-9002), or recent modifications of this technique, exemplified by the Marathon™ technology (Clontech Laboratories Inc.).

*polypeptides*

In a further embodiment this application describes a polypeptide in a substantially pure form which modulate CRH signalling wherein said polypeptide is encoded by an isolated and purified nucleic acid molecule according to the invention. In a preferred embodiment the polypeptide has the amino acid sequence selected from the group consisting of SEQ ID NO.10, SEQ ID 12, SEQ ID NO.14, SEQ ID NO.16, SEQ ID NO.18, SEQ ID NO.20, SEQ ID NO.22, SEQ ID NO.24, SEQ ID NO.33, SEQ ID NO.35, SEQ ID NO.37, SEQ ID No.39 and SEQ ID No.41 and functional analogs thereof

The protein according to the invention includes all possible amino acid variants encoded by the nucleic acid according to the invention including a polypeptide encoded by said molecule and having conservative amino acid changes.

Those skilled in the art will recognize that the protein which modulate CRH signalling could be obtained by a plurality of recombinant DNA techniques including, for example, hybridization, polymerase chain reaction (PCR) amplification, or *de novo* DNA synthesis (See e.g., T. Maniatis et al. Molecular Cloning: A Laboratory Manual, 2d Ed. Chap. 14 (1989)).

Purified biologically active protein that modulates CRH signallings may have several different physical forms. The polypeptides according to the invention may exist as full-length nascent or unprocessed polypeptides, or as partially processed polypeptides or combinations of processed polypeptides. The full-length nascent polypeptide may be post-translationally modified, amongst other, by specific proteolytic cleavage events that result in the formation of fragments of the full-length nascent polypeptide. A fragment, or physical association of fragments may have the full biological activity associated with proteins according to the invention; however, the degree of CRH modulating activity may vary between individual fragments.

Also preferred in this aspect of the invention are fragments characterized by structural or functional attributes of the polypeptide. Preferred embodiments of the invention in this regard include fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, high antigenic index regions of the polypeptide of the invention, and combinations of such fragments. Preferred regions are those that mediate activities of the polypeptides of the invention. Most highly preferred in this regard are fragments that have a chemical, biological or other activity of the response regulator polypeptide of the invention, including those with a similar activity or an improved activity, or with a decreased undesirable activity.

*Recombinant expression of polynucleotides encoding protein which modulate CRH activity*

In another embodiment polynucleotides according to the invention may be recombinantly expressed by molecular cloning into an expression vector containing a suitable promoter and other appropriate transcription regulatory elements, and transferred into prokaryotic or eukaryotic host cells to produce a protein that modulates CRH signalling. Techniques for such manipulations are fully described in Maniatis, T, et al., supra, and are well known in the art.

Therefore, in a further aspect this application describes an expression vector for expression of a protein that modulates CRH signalling in a recombinant host, wherein said vector contains a nucleic acid sequence encoding a protein that modulates CRH signalling and functional analogs thereof. In a more preferred aspect of this invention this expression vector contains a nucleic acid molecule encoding a protein that modulates CRH signalling, having a nucleotide sequence selected from a group consisting of: SEQ ID NO.9, SEQ ID 11, SEQ ID NO.13, SEQ ID NO.15, SEQ ID NO. 17, SEQ ID NO.19, SEQ ID NO.21, SEQ ID NO.23, SEQ ID NO.32, SEQ ID NO.34, SEQ ID NO.36, SEQ ID No.38 and SEQ ID No.40 and functional analogs thereof or contains genomic DNA encoding a protein that modulates CRH signalling.

Expression vectors are defined herein as DNA sequences that are required for the transcription of cloned copies of genes and the translation of their mRNAs in an appropriate host. Such vectors can be used to express eukaryotic genes in a variety of hosts such as bacteria including E. coli, cyanobacteria, plant cells, insect cells, amphibian cells, fungal cells including yeast cells, and animal cells.

Specifically designed vectors allow the shuttling of DNA between hosts such as bacteria-yeast or bacteria-animal cells or bacteria-fungal cells or bacteria-invertebrate cells. An appropriately constructed expression vector may contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one that causes mRNAs to be initiated at high frequency. Expression vectors may include, but are not limited to, cloning vectors, modified cloning vectors, specifically designed plasmids or viruses.

The isolated and purified nucleic acid molecules, according to the invention, encoding a protein which modulates CRH signalling may be cloned into an expression vector for expression in a recombinant host cell. Recombinant host cells may be prokaryotic or eukaryotic, including but not limited to bacteria such as E. coli, fungal cells such as yeast, amphibian cells such as Xenopus oocytes, mammalian cells including but not limited to cell lines of human, bovine, porcine, monkey and rodent origin, and insect cells including but not limited to Drosophila- and silkworm-derived cell lines. Cell lines derived from mammalian species which may be suitable and which are commercially available, include but are not limited to, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, neuroblastoma, glial cells and HEK-293 (ATCC CRL1573).

Therefore, in a further embodiment this application describes a recombinant host cell containing a recombinantly cloned nucleic acid molecule encoding a protein that modulates CRH signalling or functional analog thereof. In a further aspect the recombinant host cell according to the invention contains a nucleic acid molecule which is either genomic DNA or has a nucleotide sequence selected from a group consisting of: SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID NO.32, SEQ ID NO.34, SEQ ID NO. 36, SEQ ID No.38 and SEQ ID No.40; and functional analogs thereof.

The expression vector may be introduced into host cells via any one of a number of techniques including but not limited to transformation, transfection, protoplast fusion, lipofection, and electroporation. The expression vector-containing cells are clonally propagated and analyzed to determine whether they produce a protein that modulates CRH signalling. Identification of permeases expressing host cell clones may be done by several means, including but not limited to immunological reactivity with antibodies directed against the polypeptides according to the invention, and the presence of host cell-associated mammalian purine permease activity.

Thus, the present invention also relates to a process for expression of protein that modulates CRH signalling in a recombinant host cell, comprising culturing the host cells according to the invention under conditions which allow expression of the protein that modulates CRH signalling from the expression vector as outlined herein. The proteins of this invention may be synthesized either by direct expression or as a fusion protein comprising the protein of interest as a translational fusion with another protein or peptide that may be removable by self, enzymatic or chemical cleavage. Therefore, in a particular embodiment this invention provides the proteins according to the invention wherein said polypetides are part of a fusion protein.

It is often observed in the production of certain peptides in recombinant systems that expression as a fusion protein prolongs the life span, increases the yield of the desired peptide, or provides a convenient means of purifying the protein. This is particularly relevant when expressing mammalian proteins in prokaryotic hosts. A variety of peptidases (e.g. enterokinase and thrombin), which cleave a polypeptide at specific sites or digest, the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See e.g., P.Carter, "Site Specific Proteolysis of Fusion Proteins", Chapter 13, in Protein Purification: From Molecular Mechanisms to Large Scale Processes. American Chemical Society, Washington, D.C. (1990).

Furthermore, one could use, e.g., a mammalian cell that already comprises in its genome a nucleic acid molecule encoding a protein that modulates CRH signalling as described above, but does not express the same or not in an appropriate manner due to, e.g., a weak promoter, and introduce into the mammalian cell a regulatory sequence such as a strong promoter in close proximity to the endogenous nucleic acid molecule encoding said purine permease polypeptide so as to induce expression of the same.

As such a recombinant host cell containing a polynucleotide encoding a protein which modulates CRH signalling under the control of a heterologous transcription and/or regulatory sequence or protein, would be another embodiment of this invention.

In this context the term "regulatory sequence" denotes a nucleic acid molecule that can be used to increase the expression of the purine permease polypeptide, due to its integration into the genome of a cell in close proximity to the CRH modulating protein-encoding gene. Such regulatory sequences comprise promoters, enhancers, inactivated silencer intron sequences, 3'UTR and/or 5'UTR coding regions, protein and/or RNA stabilizing elements, nucleic acid molecules encoding a regulatory protein, e.g., a transcription factor, capable of inducing or triggering the expression of the CRH modulating protein-encoding gene or other gene expression control elements which are known to activate gene expression and/or increase the amount of the gene product. The introduction of said regulatory sequence leads to increase and/or induction of expression of polypeptides, which modulate CRH signalling, resulting in the end in an increased amount of said polypeptides in the cell. Thus, the present invention is aiming at providing *de novo* and/or increased expression of polypeptides that modulate CRH signalling.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis, Basic Methods In Molecular Biology (1986). It is specifically contemplated that polypeptides, which modulate CRH signalling may in fact be expressed by a host cell lacking a recombinant vector.

In addition, expression of polynucleotides according to the invention may also be performed using *in vitro* produced synthetic mRNA. Synthetic mRNA or mRNA isolated from cells capable of modulating CRH signaling can be efficiently translated in various cell-free systems, including but not limited to wheat germ extracts and reticulocyte extracts, as well as efficiently translated in cell based systems, including but not limited to microinjection into frog oocytes, with microinjection into frog oocytes being generally preferred.

*Transgenic non-human animals*

The present invention also relates to a method for the production of a transgenic non-human animal, preferably transgenic mouse, comprising introduction of a polynucleotide or vector of the invention into a germ cell, an embryonic cell, stem cell or an egg or a cell derived therefrom. The non-human animal can be used in accordance with a screening method of the invention described herein and may be a non-transgenic healthy animal, or may have a phosphate uptake or reabsorption disorder, preferably a disorder caused by at least one mutation in the protein that modulates CRH signalling. Such transgenic animals are well suited for, e.g., pharmacological studies of drugs in connection with mutant forms of the above described polypeptides. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe; see supra.

Preferably, the transgenic non-human animal of the invention further comprises at least one inactivated wild type allele of the corresponding mammalian CRH modulating protein-encoding gene; see supra. This embodiment allows for example, the study of the interaction of various mutant forms of polypeptides according to the invention on the onset of the clinical symtoms of disease related to disorders in CRH metabolism. All the applications that have been herein before discussed with regard to a transgenic animal also apply to animals carrying two, three or more transgenes; e.g. encoding neutral endopeptidase (NEP). It might be also desirable to inactivate protein that modulates CRH signalling expression or function at a certain stage of development and/or life of the transgenic animal. This can be achieved by using, for example, tissue specific, developmental and/or cell regulated and/or inducible promoters which drive the expression of, e.g., an antisense or ribozyme directed against the RNA transcript encoding the protein capable of modulating CRH signalling; see also supra. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of the mutant protein that modulates CRH signalling may be controlled by such regulatory elements.

Furthermore, the invention also relates to a transgenic mammalian cell which contains (preferably stably integrated into its genome) a nucleic acid molecule according to the invention or part thereof, wherein the transcription and/or expression of the nucleic acid molecule or part thereof leads to reduction of the synthesis of a protein that modulates CRH signalling.

In a preferred embodiment, the reduction is achieved by an anti-sense, sense, ribozyme, co-suppression and/or dominant mutant effect. "Antisense" and "antisense nucleotide" means DNA or RNA constructs which block the expression of the naturally occurring gene product.

The provision of the polynucleotide according to the invention opens up the possibility to produce transgenic non-human animals with a reduced level of the protein as described above and, thus, with a defect in phosphate metabolism. Techniques how to achieve this are well known to the person skilled in the art. These include, for example, the expression of antisense-RNA, ribozymes, of molecules which combine antisense and ribozyme functions and/or of molecules which provide for a co-suppression effect; see also supra. When using the antisense approach for reduction of the amount of protein that modulates CRH signallings in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a protein that modulates CRH signalling. In this case the homology is preferably higher than 80%, particularly higher than 90% and still more preferably higher than 95%. The reduction of the synthesis of a protein according to the invention in the transgenic mammalian cells can result in an alteration in, e.g., adenine reabsorption. In transgenic animals comprising such cells this can lead to various physiological, developmental and/or morphological changes.

Thus, the present invention also relates to transgenic non-human animals comprising the above-described transgenic cells. These may show, for example, a deficiency in CRH metabolism compared to wild type animals due to the stable or transient presence of a foreign DNA resulting in at least one of the following features:

(a) disruption of (an) endogenous gene(s) encoding a protein capable of modulating CRH signalling;
(b) expression of at least on antisense RNA and/or ribozyme against a transcript comprising a polynucleotide of the invention;
(c) expression of a sense and/or non-translatable mRNA of the polynucleotide of the invention;
(d) expression of an antibody of the invention;
(e) incorporation of a functional or non-functional copy of the regulatory sequence of the invention; or
(f) incorporation of a recombinant DNA molecule or vector of the invention.

With the polypeptides, their encoding polynucleotides and vectors of the invention, it is now possible to study *in vivo* and *in vitro* the efficiency of drugs in relation to particular mutations in protein that modulates CRH signallings of a patient and the affected phenotype. Furthermore, mutant forms of polypeptides of the invention can be used to determine the pharmacological profile of drugs and for the identification and preparation of further drugs which may be effective for the treatment of disorders related to the CRH metabolism, in particular for the amelioration of CRH induced stress or depression.

It will thus be appreciated that the present invention also relates to a method for preventing, treating or ameliorating a medical condition related to a disorder of CRH metabolism including CRH receptor related disorders which comprises administering to a mammalian subject a therapeutically effective amount of the polypeptides, the polynucleotides or the vectors encoding a protein capable of modulating CRH signalling of the present invention.

*Diagnostic Assays*

This invention further relates to the use of polynucleotides of the present invention as diagnostic reagents. Detection of a mutated form of the gene characterised by the polynucleotide of SEQ ID No.1, SEQ ID No.2, SEQ ID No. 3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO. 36, SEQ ID No.38 and SEQ ID No.40 which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

It will thus be appreciated that this invention provides a method of diagnosing a pathological condition or a susceptibility to a pathological condition in a subject related to a disorder of CRH activity comprising:

(a) determining the presence or absence of a mutation in the polynucleotide according to the invention; and

(b) diagnosing a pathological condition or susceptibility to a pathological condition based on the presence or absence of said mutation.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled nucleotide sequences according to the invention.

Perfectly matched sequences can be distinguished from mismatched duplexes by RNase digestion or by differences in melting temperatures. DNA sequence differences may also be detected by alterations in electrophoretic mobility of DNA fragments in capilary electrophoresis columns or gels, with or without denaturing agents, or by direct DNA sequencing (e.g., Myers et al., Science (1985)230:1242). Sequence changes at specific locations may also be revealed by specific restriction endonucleases, nuclease protection assays, such as RNase and S1 protection or a chemical cleavage method (see Cotton et al., Proc Natl Acad Sci USA (1985) 85: 4397-4401). In another embodiment, an array of oligonucleotides probes comprising a nucleotide sequence encoding a protein capable of modulating CRH activity or fragments thereof can be constructed to conduct efficient screening of e.g., genetic mutations. Array technology methods are well known and have general applicability and can be used to address a variety of questions in molecular genetics including gene expression, genetic linkage, and genetic variability (see for example: M.Chee et al., Science, Vol 274, pp 610-613 (1996)).

The diagnostic assays offer a process for diagnosing or determining a susceptibility to the diseases through detection of mutations in the CRH modulating protein-encoding gene by the methods described. In addition, such diseases may be diagnosed by methods comprising determining from a sample derived from a subject an abnormally decreased or increased level of polypeptide or mRNA, as well as by determining from said samples the presence of protein derivatives compared to the normal structure (see above). Decreased or increased expression can be measured at the RNA level using any of the methods well known in the art for the quantitation of polynucleotides, such as, for example; nucleic acid amplification, for instance via PCR, RT-PCR; RNase protection; Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays. Assay techniques that can be used to determine the presence of protein derivatives or variants comprise amongst others mass spectrometry.

Thus in another aspect, the present invention provides a method of diagnosing a pathological condition or a susceptibility to a pathological condition in a subject related to a disorder of prolonged CRH exposure comprising:

(a) determining the presence or amount of expression of the polypeptide or a derivative thereof according to the invention in a biological sample; and
(b) diagnosing a pathological condition or susceptibility to a pathological condition based on the presence or amount of expression of the polypeptide or of a derivative thereof.

In particular the present invention provides a method of diagnosing a CRH induced gene expression profile in an individual, said method comprising;

a) obtaining a biological sample of said individual; and

b) determine the amount of at least one protein that modulates corticotropin releasing hormone (CRH) signaling in said biological sample;

whereby the protein that modulates corticotropin releasing hormone (CRH) signaling is SEQ ID NO.20. In an alternative embodiment the method of diagnosing a CCRH induced expression profile is not limited to at least one protein according to the invention, but requires the simultaneous assessment of the expression levels of the group of proteins identified as being involved in CRH signaling, i.e. the proteins having the amino acid sequences SEQ ID NO.10, SEQ ID 12, SEQ ID NO.14, SEQ ID NO.16, SEQ ID NO.18, SEQ ID NO.20, SEQ ID NO.22, SEQ ID NO.24, SEQ ID NO.33, SEQ ID NO. 35, SEQ ID NO.37, SEQ ID No.39 and SEQ ID No.41.

Preferably the amount of said proteins is determined either at the protein level, preferably using antibodies that bind thereto, or at the gene transcription level, preferably using probes that bind to a polynucleotide encoding the amino acid sequence SEQ ID NO.20.

Alternatively the CRH induced gene expression profile is determined by assessing the level of gene transcription of a gene comprising a nucleic acid sequence selected from the group consisting of SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO.36, SEQ ID No.38 and SEQ ID No.40. Methods to determine the level of gene transcription have been described hereinbefore and comprise in a preferred embodiment the use of a probe which binds, preferably selectively binds to a polynucleotide selected from the group consisting of SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO.13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO.36, SEQ ID No.38 and SEQ ID No.40 or the complement thereof.. In another embodiment, an array of oligonucleotides probes comprising a nucleotide sequence according to the invention or fragments thereof can be constructed to conduct efficient screening of the level of gene transcription in the sample of an individual.

Also described is a diagnostic kit which comprises:

(a) a polynucleotide of the present invention, preferably the nucleotide sequence of SEQ ID No.1, SEQ ID No.2, SEQ ID No.3, SEQ ID No.4, SEQ ID NO.5, SEQ ID NO.6, SEQ ID No.7, SEQ ID No.8, SEQ ID NO.9, SEQ ID 11, SEQ ID NO. 13, SEQ ID NO.15, SEQ ID NO.17, SEQ ID NO.19, SEQ ID NO.21, SEQ ID NO.23, SEQ ID No.25, SEQ ID No.26, SEQ ID No.27, SEQ ID No.28, SEQ ID No.29, SEQ ID No.30, SEQ ID No.31, SEQ ID NO.32, SEQ ID NO.34, SEQ ID NO.36, SEQ ID No.38 and SEQ ID No.40 or a fragment thereof;

(b) a nucleotide sequence complementary to that of (a);

(c) a polypeptide of the present invention, preferably the polypeptide of SEQ ID NO.10, SEQ ID 12, SEQ ID NO.14, SEQ ID NO.16, SEQ ID NO.18, SEQ ID NO.20, SEQ ID NO.22, SEQ ID NO.24, SEQ ID NO.33, SEQ ID NO.35, SEQ ID NO.37, SEQ ID No.39 and SEQ ID No.41 or a fragment thereof; or

(d) an antibody to a polypeptide of the present invention, preferably to the polypeptide of SEQ ID NO.10, SEQ ID 12, SEQ ID NO.14, SEQ ID NO.16, SEQ ID NO.18, SEQ ID NO.20, SEQ ID NO.22, SEQ ID NO.24, SEQ ID NO. 33, SEQ ID NO.35, SEQ ID NO.37, SEQ ID No.39 and SEQ ID No.41 and optionally suitable means for detection.

It will be appreciated that in any such kit, (a), (b), (c) or (d) may comprise a substantial component. Such a kit will be of use in diagnosing a disease or suspectability to a disease, particularly CRH metabolism related disorders such as CRH induced stress or depression.

The nucleotide sequences of the present invention are also valuable for chromosome localisation. These sequences are specifically targeted to, and can hybridize with, a particular location on an individual human chromosome. The mapping of relevant sequences to chromosomes according to the present invention is an important first step in correlating those sequences with gene-associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (coinheritance of physically adjacent genes). The gene of the present invention maps to human chromosome 15.

The differences in the cDNA or genomic sequence between affected and unaffected individuals can also be determined. If a mutation is observed in some or all of the affected individuals but not in any normal individuals, then the mutation is likely to be the causative agent of the disease.

The nucleotide sequences of the present invention are also valuable for tissue localisation. Such techniques allow the determination of expression patterns of the polypeptides according to the invention in tissues by detection of the mRNAs that encode them. These techniques include *in situ* hybridziation techniques and nucleotide amplification techniques, for example PCR. Such techniques are well known in the art. Results from these studies provide an indication of the normal functions of the polypeptides in the organism.

The polypeptides of the invention or their fragments or analogs thereof, or cells expressing them, can also be used as immunogens to produce antibodies immunospecific for polypeptides of the present invention. The term "immunospecific" means that the antibodies have substantially greater affinity for the polypeptides of the invention than their affinity for other related polypeptides in the prior art.

Thus in another embodiment, this application describes a monospecific antibody immunologically reactive with a mammalian purine permease. In a preferred embodiment said antibody is immunologically reactive with a polypeptide having an amino acid sequence selected from a group consisting of: SEQ ID NO.10, SEQ ID 12, SEQ ID NO.14, SEQ ID NO.16, SEQ ID NO.18, SEQ ID NO.20, SEQ ID NO.22, SEQ ID NO.24, SEQ ID NO.33, SEQ ID NO.35, SEQ ID NO. 37, SEQ ID No.39 and SEQ ID No.41; and functional analogs thereof or said antibody blocks activity of a protein that modulates CRH signalling.

Antibodies generated against polypeptides of the present invention may be obtained by administering the polypeptides or epitope-bearing fragments, analogs or cells expressing these to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975)256:495-497), the trioma technique, the human B-cell hybridoma technique (Kozbor et al., Immunology Today (1983)4:72) and the EBV-hybridoma technique (Cole et al., MONOCLONAL ANTIBODIES AND CANCER THERAPY, pp.77-96, Alan R. Liss, Inc., 1985).

Techniques for the production of single chain antibodies, such as those described in U.S. Patent No.4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography.

Antibodies against polypeptides of the present invention may also be employed to treat the CRH metabolism related disorders.

In a further aspect, the present invention relates to genetically engineered soluble fusion proteins comprising a polypeptide of the present invention, or a fragment thereof, and various portions of the constant regions of heavy or light chains of immunoglobulins of various subclasses (IgG, IgM, IgD, IgE). Preferred as an immunoglobulin is the constant part of the heavy chain of human IgG, particularly IgG I, where fusion takes place at the hinge region. In a particular embodiment, the Fc part can be removed simply by incorporation of a cleavage sequence which can be cleaved with for instance blood clotting factor Xa. Furthermore, this invention relates to processes for the preparation of these fusion proteins by genetic engineering, and to the use thereof for drug screening, diagnosis and therapy. A further aspect of the invention also relates to polynucleotides encoding such fusion proteins. Examples of fusion protein technology can be found in International Patent Application Nos. W094/29458 and W094/22914.

*Therapeutic Utility*

A further aspect of the invention relates to an immunological/vaccine formulation (composition) which, when introduced into a mammalian host, induces an immunological response in that mammal to a polypeptide of the present invention wherein the composition comprises a polypeptide or polynucleotide of the present invention. The vaccine formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

In still another approach, expression of the gene encoding proteins which modulate CRH signalling can be inhibited using expression blocking techniques. Known such techniques involve the use of antisense sequences, either internally generated or externally administered (see, for example, O'Connor, J. Neurochem (1991) 56:560 ;Oligodeoxynucleotides as Antisense Inhibitors of Gene Expression, CRC Press, Boca Raton, FL (1988)). Alternatively, oligonucleotides which form triple helices ("triplexes") with the gene can be supplied (see, for example, Lee et al., Nucleic Acids Res (1979)6:3073; Cooney et al.., Science (1988)241:456; Dervan et al., Science (1991)251:1360). These oligomers can be administered per se or the relevant oligomers can be expressed *in vivo.* Synthetic antisense or triplex oligonucleotides may comprise modified bases or modified backbones. Examples of the latter include methylphosphonate, phosphorothioate or peptide nucleic acid backbones. Such backbones are incorporated in the antisense or triplex oligonucleotide in order to provide protection from degradation by nucleases and are well known in the art. Antisense and triplex molecules synthesised with these and/or other modified backbones also form part of the present invention.

In another process for inhibiting expression of a target gene in a cell, RNA with partial or fully double-stranded character is introduced into the cell or into the extracellular environment. Inhibition is specific in that a nucleotide sequence from a portion of the target gene is chosen to produce inhibitory RNA. The RNA may comprise one or more strands of polymerized ribonucleotide; it may include modifications to either the phosphate-sugar backbone or the nucleoside. The double-stranded structure may be formed by a single self-complementary RNA strand or two complementary strands. Inhibition is sequence-specific in that the nucleotide sequences corresponding to the duplex region of the RNA are targeted for genetic inhibition. RNA containing a nucleotide sequence identical to a portion of the target sequence is preferred. Examples of RNA inhibition technology can be found in International Patent Application WO 99/32619.

In addition, expression of the proteins which modulate CRH signalling may be prevented by using ribozymes specific to the mRNA sequence encoding said protein. Ribozymes are catalytically active RNAs that can be natural or synthetic (see for example Usman, N, et al., Curr. Opin. Struct. Biol (1996)6(4), 527-33.) Synthetic ribozymes can be designed to specifically cleave the aforementiond mRNAs at selected positions thereby preventing translation of said mRNAs into functional polypeptide. Ribozymes may be synthesised with a natural ribose phosphate backbone and natural bases, as normally found in RNA molecules. Alternatively the ribozymes may be synthesised with non-natural backbones to provide protection from ribonuclease degradation, for example, 2'-O-methyl RNA, and may contain modified bases.

For treating abnormal conditions related to an under-expression of proteins which modulate CRH signalling, several approaches are also available. One approach comprises administering to a subject a therapeutically effective amount of a compound which activates a polypeptide of the present invention, i.e., an agonist as described above, in combination with a pharmaceutically acceptable carrier, to thereby alleviate the abnormal condition. Alternatively, gene therapy may be employed to effect the endogenous production of mammalian purine permease by the relevant cells in the subject. For example, a polynucleotide of the invention may be engineered for expression in a replication-defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For an overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of a polypeptide of the present invention in combination with a suitable pharmaceutical carrier.

In a further aspect, the present invention provides for pharmaceutical compositions comprising a therapeutically effective amount of a polypeptide, such as the soluble form of a polypeptide of the present invention, agonist/antagonist peptide or small molecule compound, in combination with a pharmaceutically acceptable carrier or excipient. Such carriers include, but are not limited to, saline, buffered saline, dextrose, water, glycerol, ethanol, and combinations thereof. The invention further relates to pharmaceutical packs and kits comprising one or more containers filled with one or more of the ingredients of the aforementioned compositions of the invention. Polypeptides and other compounds of the present invention may be employed alone or in conjunction with other compounds, such as therapeutic compounds.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide or other compounds of the present invention can be formulated in an enteric or an encapsulated formulation, oral administration may also be possible. Administration of these compounds may also be topical and/or localized, in the form of patches, salves, pastes, gels, and the like.

The dosage range required depends on the choice of peptide or other compounds of the present invention, the route of administration, the nature of the formulation, the nature of the subject's condition, and the judgment of the attending practitioner. Suitable dosages, however, are in the range of 0.1-100$\mu$g/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

EXPERIMENTAL PROCEDURES

*Animal housing and treatment* - Male transgenic CRF overexpressing C57BL/6J mice (CRF-OE or TG) and their wild-type littermates (WT) were maintained in an specific pathogen free facility that meets all national and European requirements for animal care. Mice were housed individually 4 weeks before the start of the experiment in a climate-controlled animal colony with a 12h dark-light cycle (light on 7:00 EST) with free access to food and water. Prior to treatment 3 month old mice were randomized over three groups each comprising 4 wild-type and 4 transgenic animals. One group received no treatment, a second group received vehicle injection 5 days, 2 times per day (10% cyclodextran (CD), pH4) and a third group received 10 mg/kg of a CRH-R1 specific antagonist R121919 in 10% CD following the same regime. 16 hrs after last treatment animals were sacrificed for dissection of brain areas and subsequent isolation of RNA.

*Sample preparation* - Macroscopically the following brain areas were dissected from transgenic and wild-type animals; pituitary, cerebellum, temporal area, hippocampus, frontal cortex, and nucleus accumbens. Brain tissue was homogenized in Trizol (Invitrogen Life Technologies) using an Ultra-turrax T25 grinder (IKA-labortechnik). Total RNA was extracted using Trizol according to the instructions of the manufacturer. Total RNA was further purified using Rneasy kit (Qiagen) with DNAseI treatment on column.

*Microarray hybridization* - cRNA was prepared as follows. Reverse transcription was performed on 10μg of total RNA for 1h at 42°C using a T7-oligo(dT)$_{24}$-primer and SuperscriptII RT (Invitrogen Life Technologies). Second strand cDNA synthesis was done for 2 h at 16°C using *Escherichia coli* DNA Polymerase I, DNA ligase and RNAseH (Invitrogen Life Technologies). After phenol-chloroform extraction using phase-lock gel (Eppendorf) in vitro transcription was performed for 6 h at 37°C using the Bioarray high-yield RNA transcript labeling kit with Biotin labeled ribonucleotides (Enzo Diagnostics). cRNA samples were purified on Qiagen Rneasy columns followed by fragmentation for 35 min at 95°C. cRNA yields were between 50 and 100μg. Samples were processed on GeneChips (Affymetrix, Santa Clara, CA). In order to check the quality of each sample, 5μg of labeled cRNA was run on Test2-arrays. Actual experiments were performed on Murine Genome U74Av2 arrays, containing probe sets interrogating approximately 12,000 full-length mouse genes and EST clusters from the UniGene database (Build 74). Hybridization was performed using 15μg cRNA for 16 h at 45°C under continuous rotation. Arrays were stained in Affymetrix Fluidics stations using Streptavidin/Phycoerythrin (SAPE) followed by staining with anti-streptavidin antibody and a second SAPE staining. Subsequently arrays were scanned with a HP-Laserscanner and data were analyzed with the Microarray Suite Software (Affymetrix). No scaling or normalization was performed at this stage. Quality of the experiment was assessed based on the percentages present calls (see table).

| Area | Present calls | 3'/5' β-Actin | 3'/5' GAPDH |
|---|---|---|---|
| temporal area | 46.15±1.98% | 1.96±0.53 | 1.46±0.38 |
| hippocampus | 46.27±1.89% | 1.58±0.39 | 1.22±0.29 |
| cerebellum | 47.14±2.13 | 1.67±0.37 | 0.97±0.08 |
| frontal cortex | 44.68±2.23 | 1.85±0.39 | 1.29±0.26 |
| nucleus accumbens | 44.27±2.11 | 2.69±0.85 | 2.52±1.20 |
| pituitary | 45.33±1.67 | 1.53±0.28 | 1.25±0.21 |

Samples with low yields of RNA or low percentages present calls or deviating 3'/5' ratios were omitted from subsequent analysis. The number of animals that were used for analysis are shown in table.

| | WT untreated | WT vehicle | WT R121919 | TG untreated | TG vehicle | TG R121919 |
|---|---|---|---|---|---|---|
| temp. area | 4 | 4 | 4 | 3 | 4 | 3 |
| hippocampus | 4 | 4 | 4 | 3 | 3 | 4 |
| cerebellum | 3 | 4 | 4 | 3 | 4 | 3 |
| frontal cortex | 3 | 4 | 4 | 3 | 4 | 4 |
| nucleus accumbens | 4 | 3 | 4 | 4 | 3 | 4 |
| pituitary | 4 | 4 | 4 | 4 | 3 | 4 |

*Data analysis and selection of genes*

Raw intensities on each array were normalized using the following algorithm:

$$\log I_{aligned} = \log I_{raw} + \left( \log \frac{\sum_{all\ samples} I_{raw}}{\#\ genes \times \#\ samples} - \log \frac{\sum_{sample} I_{raw}}{\#\ genes} \right)$$

Basically this alignment sets the average intensity of one array to the average measured this normalization compensates for array to array variations in hybridization, washing and staining, ultimately allowing a reasonable comparison between arrays. After normalization, data were analyzed using weighted spectral mapping analysis (SMA) (7). Weighted spectral mapping is an unsupervised multivariate analysis method which includes double-centering of the data combined with a specialized visualization representing the two highest principle components explaining most of the variance within the dataset. Even though double-centering removes the "size" component of the array data, this information is reintroduced in the visualization via the area of the symbols representing the size of the respective samples (depicted by squares) and genes (depicted by circles). What remains are contrasts between the different genes and contrasts between the different samples of the data table. These contrasts can be expressed as ratios due to logarithmic transformation performed by the algorithm. The contrasts can be understood as specificities of the different genes for the different samples. Conversely, they refer also to the specificities or preferences of the different samples for some of the genes. Therefore, one could state that SMA provides a visualization of the interactions between genes and samples. This method allows the reduction of a large microarray dataset and provides means to visually inspect and thereby identify clusters of genes and/or subjects in the data. In the spectral map samples which cluster together can thus be interpreted as being similar. Correspondingly samples located at opposite sites of the axes or the center can be interpreted as being dissimilar.

In addition to this overall analysis the identification of individual genes with a different expression level between groups were identified using significance analysis of microarray data (SAM) (8). SAM identifies genes with statistically significant changes in expression by assimilating a set of gene-specific t-tests. In contrast to the classical t-test, this gene-specific t-tests not only takes into account the variance observed for a particular gene but also includes a so called fudge factor covering the overall variance across all genes measured. Genes with scores greater than a threshold are deemed potentially significant. In order to estimate the number of false positives among these potentially significantly changed genes, SAM uses permutations to estimate the false discovery rate. To this end t-values are calculated iteratively on permutations of the measurements. The threshold can be adjusted to identify smaller or larger sets of genes, and corresponding FDRs are calculated. In addition to the threshold delta that influences the number of significantly changed genes and a corresponding FDR, a fold change threshold can be imposed to discards genes that show only a limited difference in expression between samples. These changes are probably from a biological point of view of little significance.

K-means clustering of expression data obtained on a given brain area was carried out using the OmniViz program. All records which were absent in all samples were removed and all signals less than 20 were set to 20. This allowed the clustering of genes with similar behavior.

*Quantitative RT-PCR* - Microarray data were confirmed using real time PCR analysis. First strand cDNA synthesis was performed on $0.5\mu g$ total RNA using random hexamer primers and SuperscriptII RT (Invitrogen Life Technologies). Quantitative PCR was performed on a ABIPrism 7700 cycler (Applied Biosystems) using a Taqman PCR kit. Serial dilutions of cDNA were used to generate standard curves of threshold cycles versus the logarithms of concentration for β-actin, CRH receptor 1 (Crh-R1), CRH receptor 2 (Crh-R2), neurotensin receptor 1 (Ntsr1), neurotensin receptor 2 (Ntsr2), neurotensin receptor 3 (Ntsr3), 11β-hydroxysteroid dehydrogenase 1 (Hsd11B1), serum/glucocorticoid regulated kinase (Sgk) and the genes of interest (see table for sequences of primers). A linear regression line calculated from the standard curves allowed the determination of transcript levels in RNA samples from the different treatment groups at each brain area.

| β-actin forward | 5'-CATCTTGGCCTCACTGTCCAC-3' |
| β-actin probe | 5'-TGCTTGCTGATCCACATCTGCTGGA-3' |
| β-actin reverse | 5'-GGGCCGGACTCATCGTACT-3' |
| $CRF_2$ forward | 5'-GGGAGAACAGAAGCGCCTG-3' |
| $CRF_2$ probe | 5'-AGAAGGGTGAGGATCCCCCAAATCAGAGT-3' |
| $CRF_2$ reverse | 5'-CCCTTGTTTCAATCACTCCCA-3' |
| $CRF_1$ forward | 5'-TTTCTGAACAGTGAGGTCCGC-3' |
| $CRF_1$ probe | 5'-CCGGAAGAGGTGGCGGCGA-3' |
| $CRF_1$ reverse | 5'-GGGCTCTGATGGAGTGCTTG-3'- |
| Nts1 forward | 5'-CGCCGCCGAAAGAAGAG-3' |
| Nts1 probe | 5'-CCAACGTTCTCCAGGAAGCCAAACAG-3' |
| Nts1 reverse | 5'-ACGCATGGTTGCTGGACAT-3' |
| Nts2 forward | 5'-TGGTGACCAACACGCTCTTCT-3' |
| Nts2 probe | 5'-TCAGCTCGGCAGTGACCCCA-3' |
| Nts2 reverse | 5'-AGGAAGACACGGCGTTGTAGA-3' |
| Nts3 forward | 5'-GGAAGCCGGAGAACAGCAA-3' |
| Nts3 probe | 5'-TGCGACGCTACCGCAAAGAACA-3' |
| Nts3 reverse | 5'GGATATGAAGGCTGCACTCGTT-3' |

(continued)

| Sgk forward | 5'-TGGACCAATGCCCCAGTT-3' |
|---|---|
| Sgk probe | 5'-TCAGTCAAAGCCGTTGGTGTTTTCATTG-3' |
| Sgk reverse | 5'-GCCCGTTTTATAGGTGACATTTTAA-3' |
| Hsd11b1 forward | 5'-GGGATAATTAACGCCCAAGCTT-3' |
| Hsd11b1 probe | 5'-CCCAAGGAGGAGTGCGCCCT-3' |
| Hsd11b1 reverse | 5'-AGAGCTGTGCCTTTGATGATCTC-3' |

*Autoradiography* - [125I]Neurotensin binding and film radiographs on brains of adult male transgenic C57BL/6J mice and their wild-type littermates, was performed as described previously by Moyse et al. (12). Twenty-micron-thick sections were incubated with 0.1 nM monoiodo [125I]Tyr$_3$-neurotensin (2200 Ci/mmol, Perkin Elmer) in 50 mM Tris-HCl, pH 7.4 containing 0.1 % bovine serum albumin, 1 mM EDTA, 5 x 10$^{-5}$ M bacitracin, 2 $\mu$g/ml chymostatin and 4 $\mu$g/ml leupeptin. Incubating additional sections in the presence of 1 $\mu$M native neurotensin assessed the non-specific staining. [125I]Neurotensin binding with blockade of the Nts2 was achieved on hippocampal sections in the presence of 1 $\mu$M Levocabastine, an Nts2 antagonist. Densiometric analysis of the film radiographs was performed using an MCID M4 digital analyzer (Imaging Research, St Catharines, ON, Canada). On each section structures were delineated interactively and pixel gray levels were measured in the outlined areas. The pixel gray levels were converted to radioactivity per mg of tissue using the co-exposed 125I-standards and averaged over the measured area. Data were analyzed statistically by a two-way ANOVA and concomitant Bonferroni's Post-hoc test using SPSS 11.0 software.

## RESULTS & DISCUSSION

In order to study the effects of prolonged CRH administration on the central nervous system we compared gene expression profiles in brain regions derived from CRH overexpressing mice to those of their wild-type littermates. In addition we studied the effects of CRH-R1 antagonist administration on these expression profiles. The brain regions investigated in this study include pituitary, nucleus accumbens, frontal cortex, temporal area, hippocampus and cerebellum. Expression of CRH receptor 1 (CRH-R1) and 2 (CRH-R2) was measured in brain areas using quantitative real time RT-PCR (RTq). Although CRH-R1 was readily detectable, no reliable measurements could be obtained for CRH-R2 in these brain areas. The observed expression levels of CRH receptors in these brain areas correspond with previous reports of high levels of CRH-R1 in cerebellum and frontal cortex and lower levels of expression of CRH-R2 which is mainly a peripheral receptor (see figure 1)(9).

RNA isolated from the different brain areas derived from different animals were processed separately on arrays containing 12000 murine EST. After normalization data were analyzed per brain area. First pass examination of data was performed using spectral map analysis (SMA), allowing an overall graphical interpretation of gene expression responses in relation to the treatment group and genotype. SMA indicated no gross changes in expression between treatment groups for all areas except pituitary. In all brain areas most samples derived from transgenic animals (Tg) are clustered at a different location than those derived from wild-type animals (WT). This is most obvious at the level of the pituitary where a clear difference in expression pattern is observed between Tg and WT animals with samples clustered at opposite sites in the spectral map. Taken together these data indicate that life long exposure to CRH induces changes in gene expression profiles in all studied brain areas, with the most prominent effects observed in pituitary. These changes in expression profile are not affected (or only to a limited degree) by a 5 day treatment with CRH-R1 antagonist as exemplified by the pituitary spectral map.

In order to identify genes that were significantly changed, the significance analysis of microarray data (SAM) algorithm was applied. In agreement with the SMA only a limited number of genes was found significantly changed in most brain areas (see supplementary information). In pituitary however more than 300 genes were differently expressed between WT and TG animals. Gene expression profiles in the studied brain areas of CRH overexpressing mice elucidated previously unrecognized homeostatic mechanisms present in these animals. In general the observed expression profiles point towards a few pathways that are affected in transgenic animals (TG).

TG animals have plasma glucocorticoid levels that are 6-8 times higher than in WT animals (see figure 3). As a consequence gene expression profiles show an adaptation to these high levels of corticosterone. This is achieved both by downregulation of enhancers of glucocorticoid receptor signaling such as 11β-hydroxysteroid dehydrogenase type 1 (11β-HSD1) in hippocampus and upregulation of putative repressors of this signaling pathway such as FK506 binding protein 5 (Fkbp5) (see figure 4).

Tissue glucocorticoid concentrations are determined not only by plasma hormone levels, but also by two intracellular 11β-hydroxysteroid dehydrogenases (type 1 and 2), which locally interconvert active glucocorticoids and inert 11-keto forms. 11β-HSD1, the predominant isoform in the brain, appears to function as predominant 11β-reductase, regenerating active glucocorticoids from 11-keto forms. By studying 11β-HSD1 deficient mice it was demonstrated that the intracellular regeneration of glucocorticoids by 11β-HSD1 plays an important role in HPA-axis control. In response to restraint stress these mice show exaggerated ACTH and corticosterone levels. In addition 11β-HSD1 deficient mice were less sensitive to exogenous cortisol suppression of HPA activation, suggesting a diminished glucocorticoid feedback in these animals. In view of the significant downregulation of 11β-HSD1 in hippocampus of CRH overexpressors it can be envisaged that these mice have an altered glucocorticoid feedback.

Fbp5 was found upregulated in all brain areas tested, although only significant in a few, all other showed a similar tendency (figure 4). The observation that the exchange of the immunophilin Fkbp5 for Fkbp4 is an important first step of for activation of the glucocorticoid receptor (GR) is of interest. In squirrel monkey and New World primate genera in general, Fkbp5 has been identified a potent inhibitor of glucocorticoid receptor binding. Our observation of induction of Fkbp5 could be explained as an attenuation of the GR in response persistent high levels of circulating glucocorticoid. Another indication of altered glucocorticoid signaling is the upregulation of serum/glucocorticoid kinase (Sgk) in cerebellum, nucleus accumbens and temporal area (figure 4). Sgk is a serine/threonine protein kinase of which transcription is induced by glucocorticoid and serum and is regulated by anisotonic and isotonic cell volume changes. In rat brain it has been shown that Sgk mRNA levels were increased by dehydration in the temporal lobe, including hippocampus. In the same study it was demonstrated that Sgk markedly increased the activity of the neuronal $K^+$ channel Kv1.3 suggesting that Sgk might participate in the regulation of neuronal excitability. Of interest is a recent report on facilitation by Sgk of memory consolidation of spatial learning in rats. Sgk was shown to be differently expressed in fast learners versus slow learners, moreover it was demonstrated that transfection of Sgk into the CA1 region of hippocampus leads to increased water maze performance (10). In that respect it is striking that while this study demonstrates that CRH overexpressors show high levels of Sgk mRNA, these animals have been reported to exhibit a profound learning deficit (11).

Neurotensin receptor 2 (Ntsr2) was found downregulated in several brain tissues including hippocampus and nucleus accumbens except pituitary (see figure 5). Neurotensin (NT) is a tridecapeptide which is found in high concentration in numerous areas of the CNS where it plays a role as neurotransmitter and neuromodulator. When NT is administered by intracerebroventricular injection a variety of CNS effects are observed, including hypolocomotion, hypothermia, analgesia, and reduced food consumption. Three receptors for NT have been identified (Ntsr1, 2 and 3). Studies of Ntsr1 deficient mice demonstrated this receptor to be responsible for the effects of NT on body temperature, feeding behavior and hypolocomotion. Ntsr2 on the other hand seems to account for the analgesic effects of NT as demonstrated by applying an antisense strategy in mice brain. The observation of Ntsr2 downregulation prompted us to investigate expression levels of other members of the NT receptor family by RTq. Ntsr3 did not show any change in expression in all areas tested. In contrast Ntsr1 was significantly downregulated in CRH overexpressors with the effect being the most pronounced in hippocampus (figure 5). Level of downregulation was higher for Ntsr1 than for Ntsr2. Striking was the upregulation of both Ntsr1 and Ntsr2 in vehicle treated animals (both tg and Wt) compared to untreated animals. This increase in expression level was abolished in R121919 treated animals, suggesting that repeated stress induced expression of Nstr1 (and to a lesser extent Nstr2) and that this induction could be reverted by a CRH-R1 antagonist (even 16 hrs after last treatment). Our data demonstrate for the first time a link between CRH and NT systems in CRH overexpressing animals and suggest a role for these NT receptors in repeated stress.

In order to evaluate the changes in Nts1 and Nts2 mRNA at the protein expression level, we assessed the receptor expression (Nts1-3) by autoradiography of [$^{125}$I]NT binding on brain sections. The generated data demonstrated an overall non-selective (Nts1-3), genetically determined downregulation of the [$^{125}$I]NT binding capacity in the *stratum radiatum* bordering the CA1-CA2 region of the hippocampus (-21.8 $\pm$ 3.0%, p<0.05) and the anterior cingulate cortex (-22.2 $\pm$ 2.7%, p<0.05%) in CRF-OE versus WT animals (Fig 6). No changes were seen in the olfactory bulb, nucleus accumbens and septum.

The [$^{125}$I]NT binding capacity of the vehicle treated animals, in both WT and CRF-OE animals, was not altered compared to the untreated animals. However, subchronic administration of $CRF_1$ antagonist R121919, resulted in an upward trend (p=0.057) of the [$^{125}$I]NT binding capacity for the *Stratum radiatum* bordering the CA1-CA2 region compared to the vehicle treated animals, in both WT and CRF-OE animals. Moreover the level of [$^{125}$I]NT binding in the R121919 treated CRF-OE animals equaled the level measured in untreated WT- animals (Fig 6).

To discriminate between the different receptors [$^{125}$I]NT binding experiments were performed in the presence of a saturating concentration of Levocabastine, a Nts2 specific antagonist. Based on the results of the previous binding experiments, we focused on hippocampal brain sections (Fig 7). [$^{125}$I]NT binding capacity was downregulated in the CRF-OE as compared to the WT animals, in the *stratum radiatum* (-63.8 $\pm$ 6.5%, p<0.001%), the retroslenial granular cortex (-49.4 $\pm$ 7.5%, p<0.001%) and the temporal cortex (-34.8 $\pm$ 3.5%, p<0.001%).

Although in the hippocampus of WT and CRF-OE vehicle-treated mice, the Nts1 mRNA levels were upregulated (Fig 5), no such alterations were observed in the vehicle-treated animals at the level of [$^{125}$I]NT binding (Fig 6 & 7). Overall the [$^{125}$I]NT binding capacity to Nts1-3 was downregulated in the *stratum radiatum* of the hippocampus and the anterior cingulate cortex in CRF-OE when compared against WT animals (Fig. 6). Moreover, when we blocked the Nts2 receptors with the Nts 2 specific antagonist Levocabastine, the downregulation of the [125I]NT binding capacity was even more pronounced in the *stratum radiatum* of the hippocampus, the retrosplenial granular cortex and the temporal cortex in CRF-OE animals versus WT-littermates (Fig 7). Although Nts3 is also expressed in the mentioned regions, the affinity for NT of Nts3 is much lower then that of Nts1 suggesting that [125I]NT primarily bound to Nts1. Furthermore, since no alterations in Nts3 mRNA were seen in the Rtq we attribute this downregulation entirely to the Nts1.

Strikingly, Nts1 (less pronounced for Nts2) mRNA expression was upregulated in vehicle-treated CRF-OE and WT animals. This mRNA increase might be induced by repeated injection stress and was counteracted by subchronic administration of the $CRF_1$ antagonist R121919. Yet, this is not translated at the receptor level. [125I]NT displayed equal binding to Nts1 in the hippocampus of untreated and vehicle-treated WT and CRF-OE mice (Fig 6, 7). However, at total NT binding level, R121919 counteracted the downregulation of [125I]NT binding. In CRF-OE R121919 increased the [125I]NT binding level in the *stratum radiatum* to an extent that equaled that in untreated animals (Fig 6). This could implicate $CRF_1$ receptors in the regulation of NT receptor expression.

Previous reports show that glucocorticoids increase NT mRNA expression (+92%) and NT-release (+100%) in primary hypothalamic neuron cultures and periventricular hypothalamic nuclei (PVN) (Nicot et al., 1995;Scarceriaux et al., 1995;Souaze et al., 1997). Interestingly, different stressors like forced swim in cold water, immobilization stress and tail shock stress also elicit increases in NT mRNA in the PVN and central amygdaloid nucleus of rats. On its turn, NT can lead to HPA-axis activation as demonstrated by intracerebroventricular administration. This activation can be blocked by both alpha-helical CRF, a non-specific CRF antagonist and by SR-48450, a Nts1-2 antagonist (Azzi et al., 1998;Lepee-Lorgeoux et al., 2000;Nicot et al., 1994;Rowe et al., 1995). High NT levels also downregulate Nts1 and Nts2 (Hermans and Maloteaux, 1998). It could be argued that the observed downregulation of Nts1-2 levels in CRF-OE relates to their increased glucocorticoids levels. The observations that hypercortisolism or dexamethasone administration results in a selective downregulation of Nts1 mRNA (40-70%) in the PVN (Nicot et al., 1995) and that dexamethasone decreases the [125I]NT binding in primary hypothalamic neuron cultures (Scarceriaux et al., 1996), support this idea. Thus, the Nts1-2 downregulation might theirfore prevent a further NT-induced CRF release. Taken together, these data demonstrate a clear link between the HPA-axis and the NT-system in limbic brain areas. The NT-system might therefore have a regulatory role in repeated stress and anxiety besides previous proposed roles in schizophrenia and pain suppression.

Other pathways affected include intracellular calcium signaling/sensing (hippocalcin like 1, calcyclin, ...), myelination (myelin, myelin-associated glycoprotein, ...), cell proliferation and neurogenesis (Edg2, Id2, GAB1, ...) and extracellular matrix formation (decorin, brevican, ...).

Hippocalcin like 1 (Hpca11) belongs to the neuronal calcium sensor family family of $Ca^{2+}$-binding proteins which play a role in diverse processes, including modulation of neurotransmitter release, control of cyclic nucleotide metabolism, biosynthesis of phosphoinositides and indirect regulation of ion channels. It has been demonstrated that hippocalcin might potentiate the protection from apoptosis exerted by neuronal inhibitor-of-apoptosis (IAPs) proteins.

The changes of expression observed in genes encoding proteins involved in myelination, cell proliferation and extracellular matrix formation suggest a change in the dynamics of neurogenesis in CRH overexpressors. In support of this are the changes in expression observed mainly in nucleus accumbens involving the endothelial differentiation lysophosphatidic acid G-protein-coupled receptor 2 (Edg2), growth factor receptor bound protein 2-associated protein 1 (Gab1), Inhibitor of DNA binding 2 (Id2) and fibroblast growth factor receptor 2 (Fgfr2).

As the pituitary is the primary target organ for CRH it is expected that prolonged elevated levels of CRH as present in CRH expressors induce major changes in expression profiles in the pituitary of transgenic animals. The activated state of the pituitary is exemplified by high expression levels of several kallikreins, which play a role as proprotein concerting enzymes in the processing of diverse hormones secreted from the pituitary. Striking are the elevated levels of preproenkephalin A (10 times higher in Tg versus Wt) and to a lesser extent of prodynorphin (2 times higher in Tg versus Wt). This finding of elevated levels of endogenous opioids in CRH overexpressors is in line with the notion that these opioids represent a major modulatory system in the adaptation of an organism to chronic stress, balancing the response that the stressor places on the brain with the potentially detrimental effects that a sustained stress response may produce. Other interesting findings in the pituitary include elevation of Bdnf mRNA levels, one of the most prevalent neurotrophic factors in the brain, and of the vesicular inhibitory amino acid transporter (Viaat). This increase in Bdnf is in contrast to reports of acute and chronic stress decreasing levels of Bdnf in hippocampus (11).

In conclusion gene expression profiling of several brain areas of CRH overexpressing mice elucidated previously unrecognized homeostatic mechanisms present in these animals.

The identification of genes altered in CRH overexpressing mice compared to wild-type littermates allow further molecular characterization of this animal model of chronic pituitary-adrenal activation. We provide the tools to evaluate at the level of gene expression the consequences of prolonged exposure to CRH, which could eventually lead to the use of novel markers in stress, anxiety or depression both in animals and in humans. In addition we demonstrate that administration of a CRH-R1 antagonist does not lead to a general 'normalization' of expression levels in transgenic animals, although at the pituitary level some effect of the compound is measured. In addition repeated stress inflicted

# EP 1 558 760 B1

by the treatment seems to elicit a response in gene expression of specific genes such as neurotensin receptor 1. This effect can be reversed by CRH-R1 specific antagonists, suggesting a close link between neurotensin and CRH.

PREFERENCES

1. De Souza EB 1995 Corticotropin-releasing factor receptors: physiology, pharmacology, biochemistry and role in central nervous system and immune disorders. Psychoneuroendocrinology 20:789-819

2. Holsboer F 2001 Stress, hypercortisolism and corticosteroid receptors in depression: implications for therapy. J Affect Disord 62:77-91

3. Holsboer F, Gerken A, Stalla GK, Muller OA 1987 Blunted aldosterone and ACTH release after human CRH administration in depressed patients. Am J Psychiatry 144:229-231

4. Holsboer F, Gerken A, von Bardeleben U, Grimm W, Beyer H, Muller OA, Stalla GK 1986 Human corticotropin-releasing hormone in depression--correlation with thyrotropin secretion following thyrotropin-releasing hormone. Biol Psychiatry 21:601-611

5. Nemeroff CB, Owens MJ, Bissette G, Andorn AC, Stanley M 1988 Reduced corticotropin releasing factor binding sites in the frontal cortex of suicide victims. Arch Gen Psychiatry 45:577-579

6. Raadsheer FC, Hoogendijk WJ, Stam FC, Tilders FJ, Swaab DF 1994 Increased numbers of corticotropin-releasing hormone expressing neurons in the hypothalamic paraventricular nucleus of depressed patients. Neuroendocrinology 60:436-444

7. Wouters L, Göhlmann HW, Bijnens L, Kass SU, Molenberghs G, Lewi PJ 2002 Graphical exploration of gene expression data:a comparative study of three multivariate methods. Biometrics

8. Tusher VG, Tibshirani R, Chu G 2001 Significance analysis of microarrays applied to the ionizing radiation response. Proc Natl Acad Sci U S A 98:5116-5121

9. Van Pett K, Viau V, Bittencourt JC, Chan RK, Li HY, Arias C, Prins GS, Perrin M, Vale W, Sawchenko PE 2000 Distribution of mRNAs encoding CRF receptors in brain and pituitary of rat and mouse. J Comp Neurol 428:191-212

10. Tsai KJ, Chen SK, Ma YL, Hsu WL, Lee EH 2002 sgk, a primary glucocorticoid-induced gene, facilitates memory consolidation of spatial learning in rats. Proc Natl Acad Sci U S A % 19;99:3990-3995

11. Heinrichs SC, Stenzel-Poore MP, Gold LH, Battenberg E, Bloom FE, Koob GF, Vale WW, Pich EM 1996 Learning impairment in transgenic mice with central overexpression of corticotropin-releasing factor. Neuroscience 74: 303-311

12. Moyse E, Rostene W, Vial M, Leonard K, Mazella J, Kitabgi P, Vincent JP, Beaudet A (1987) Distribution of neurotensin binding sites in rat brain: a light microscopic radioautographic study using monoiodo [125I]Tyr3-neurotensin. Neuroscience 22: 525-536.

13. Nicot A, Berod A, Gully D, Rowe W, Quirion R, de Kloet ER, Rostene W (1994) Blockade of neurotensin binding in the rat hypothalamus and of the central action of neurotensin on the hypothalamic-pituitary-adrenal axis with non-peptide receptor antagonists. Neuroendocrinology 59: 572-578.

14. Scarceriaux V, Pelaprat D, Forgez P, Lhiaubet AM, Rostene W (1995) Effects of dexamethasone and forskolin on neurotensin production in rat hypothalamic cultures. Endocrinology 136: 2554-2560.

15. Souaze F, Rostene W, Forgez P (1997) Neurotensin agonist induces differential regulation of neurotensin receptor mRNA. Identification of distinct transcriptional and post-transcriptional mechanisms. J Biol Chem 272: 10087-10094.

16. Azzi M, Betancur C, Sillaber I, Spangel R, Rostene W, Berod A (1998) Repeated administration of the neurotensin receptor antagonist SR 48692 differentially regulates mesocortical and mesolimbic dopaminergic systems. J Neurochem 71: 1158-1167.

17. Lepee-Lorgeoux I, Betancur C, Souaze F, Rostene W, Berod A, Pelaprat D (2000) Regulation of the neurotensin NT(1) receptor in the developing rat brain following chronic treatment with the antagonist SR 48692. J Neurosci Res 60: 362-369.

18. Nicot A, Berod A, Gully D, Rowe W, Quirion R, de Kloet ER, Rostene W (1994) Blockade of neurotensin binding in the rat hypothalamus and of the central action of neurotensin on the hypothalamic-pituitary-adrenal axis with non-peptide receptor antagonists. Neuroendocrinology 59: 572-578.

19. Rowe W, Viau V, Meaney MJ, Quirion R (1995) Stimulation of CRH-mediated ACTH secretion by central administration of neurotensin: evidence for the participation of the paraventricular nucleus. J Neuroendocrinol 7: 109-117.

SEQUENCE LISTING

SEQ ID NO.1

tttttttttttttttttaccaaaaatatcattttattctttaatggaaaaaggcattaatattcaaacaaaggaatcacattttaataaccctatagataagaaacaggc
tccaggaacattcaagcagcagtcatgagggaaaaatgtttcaatagcccagttttcttca

SEQ ID No.2

ttttttttttttttttccaacttaacgtttttttttaatttagaatataatttttaacacaaaagcagttcattttattcagcaaaataaatttaacaagttcatttaaataagg
taaattctagactctgtaacttttttttttccttagctacttgcttttctgccccttggaatccatagctgctatactaggttgctttccaaggtaacacagctgtaagg
gaaggaactgttcattcattcatatatatatatatatatatatatatatatatatataatttgaatgactcaaatacacccacgttctcattcaaccaccagtgt
gtttagtgaagcagaaggagggagcacaggga

SEQ ID No. 3

ttttttttttttttttttggacatgaagtgtgtttattaggaagaacactccaagcactcacaaatggctttcacaaacacttagcctaggctggaacacaaaagga
tatcacaacagagtccattgggtttacttgcttacatcaccaaagaatgttcatggcagttaattttcaggctgtaaaaactacatctatggcaccaacatgg

SEQ ID No.4

atatttaactcaaggcagaggaaagattatcccagctaataattccattcagctgatgaaatacttgattattagagagcagtcccaataagcagctttaagtt
ttccgtattcattttaaacatcagtgcattaccttcaatatggtaaagatgtttttttcagttgaatggttcatattttggtgcagtgtttgatgttcacaaaaaaatgt
tacaataataaactaacata

SEQ ID No.5

gaaggcaaagatcatttgcatgtttttatttaaaagtaaacagctgttaagatagaagggcagatgtttctttattgtaaagacagcagttacaaaagagttac
aagttacaagtcaaataaatatgacattaagaattttttggttttggttttgttaaaaaaatacaacaccccccctccccaggtatttgtgagaagtcccaagaaact
caagatacaaaaatctgtctcatgggcgagaatacatttctttcagttctctggaactgnttgtcagacacgggaatttggtcatgatttcaaaaga

SEQ ID No.6

```
tttttttttttttttttggnttttatcacatttattgttacatttatttaattttttttgcagtaatagatgaggcacaaacacctcctgcttctccatcactgcaacagaaa
ggacaatagtcaagggtaacagtgaaattaaaaaaaaaaaaaaaaaatacaaacgcctaaggtctgggagaaacctgactacaatctacgtggagaaact
tgtaaatgactccagcctggtcttccgattgcaccacttcacagaccacagaggaggggtgtcccagcagaaaggacacagccaggctcagctcgcca
gggtggtgggcctctgcccatccacctgcgttctgctcagctagctcaagttcacatcttgctactcacatgctgccttctggttttcaaagtgactggtacaa
acggtcagtctcctcgtgccg
```

SEQ ID No.7

```
gtggcaacacaggaaactcacagaccttggtttattagattatttttaatatgccagaccaatagaaaggaaggacggagcacacaggaacacctgtgtttg
caggacagagggcagatgtccacagccttgggcatgcatgtatctcctgtgtaattaggcaaatgaatacttaaataccacacaaccgccagacttgggg
gtggggagaatcacaggccacaggggaccatgctttaaaggcagtcagaggctttaaatacaggggctgaaccttctcactttatacaaaacttatatatta
aaagtaaggataccacggtatatacatgcaagcagctttggttggagaaaaatgaccaagggaggtggagcctgaataggaagggcntgccatataat
gtctatcttcttcatcctcacaaagtcacctgggattatagaataaatcagctggtctcagtaagatattgaaaagtccngtctggtgtcctaagtgcttaaan
gccnccgtnggcagggaagggnccttttncgagatactgaaacctngaaggaacagctggactngattgtgggtgact
```

SEQ ID No.8

```
tttatttgatcattttaatatgccaggtcaatggaaaggaaggacagagcacacaggaacacctgtatttgcaggacggagggcagatgtccacagccttg
ggcatgcatgtatctcctgtgtaattaggcaaataaatacttaaataccacagcacagccagacttgggggtgggggggaatcacaggccacaggggac
catgctttaag
```

SEQ ID No.9

ttaaataagaagtcaagatctgtgggactgtgataggcagcctattttagttgaatggggcttgctctggtgacccaatagagaaatctacaagggatgaaa
aggtgagccatattcccagaatcaggagcctgtccataattctgttactatcagtcgtgcagacaatgtcccaagcttctggcattctggttagactccaccc
ccacagccacctgactatagctcactataagagggggctgcttggcccctcctcgctctttagccctcaggtttcttactctctagcctcctcctcctctctct
ctctctctcccttccccatctctccacatggccatggctggtttctctctctttctaccttctttcctttctccctgcctttctacaataaagctctaaaaccataaa
ctgtctctgatcatgaaggcctgccatgcttgaactgccatgcttaggcttttctcctaaggagccgagtctaacctcccaccagaaggccatcctgtgctcc
agccacagacctgaccaaggactctcgcccgagtgggaaccatccagtgccctctcccccagccctctcctcctttcatccctgggggccaagtgccacc
ccgggcccctattctgttctctgttcctttgctgtatccagcctgggatgtcggaggccgagaatctggtacctgggggctgccccttgtctaccacccgctgt
gtggggttagtggcttcctacagcagacacccaccggggggccgtgtggaaagcgtgaacagtcctcctccgaccacccgcccagagcactggatctct
ggcgggacacgggttctctcccactcccctctttcccccacatccacagccctgcaaagatcaggctttgcagacaccactgttgcttttttgccgcttgctg
aagcaatgatcaaccccaccgcgttcttccttgggccacgtctcctttgagctgtttgcagacaaagttccaaagacagcagaaaactttcatgctctgagc
actgaagagaaaaggattggctataaggggttcctcctttcacagaattgtcccaggattcatgtgccaggtggtgacttcacacgccgtaatggcactgat
ggcaggtccatctacggagagaaatctgaggatgacaatttcatcctgaagcatacaggtcctggcatcttgtccatggcaaatgctggaccaaacacaa
atgggtcccagttttttatctgcactgccaagactgaatggctcgatggcaagcatttggtctttggaaaggtgaaagaaggcaatgaacattgtcgaagcc
atggagcgttttgggtccaagaatggcaagaccagcaagaagatcaccatttgtgactgtggacaagtctaatttctttttgactttcggccattttacccatca
aaccattctttctgtagctcataagaacaccccctaccccatctgctctcaatgtcctgtaatctctgctctcactgaagttctttgggttccatatttcctcattcc
ccttcaagtctagctggattgcaaagttaagcttatgattatgaataaaaactaaataagaaaaaaaagtcaagatcagatttagaatttaaaacaactataca
gaggtaccattaactaaattcaatttatatgttcttaagatgtatacaaaataaagtttgatatattcgtgaaatttttgtttaaccaagaaaagaaaaaaaaaat
aaaaaggattttttgcaaaaacacatatccaccaagtagcttatctaaaatatacaaagaatatgtatatagcacagcagaaagggaaaaaaaaacccca
actttaaagtaggaaaatatttacatagtcactgcatgaacggtattcaaaacccagaaaatcacattaaaaaaatgttcagtgtccttagtagtgaggggaa
tgcaaatttgtgtttgaaaggttgctggtttccaagagatcctttgagaacagtgtggtagctgctgctaaagaatcaggcaaagccagaagttgctcacatt
taggaggcttgtgtgctggggggttgggaggatgactgctaaatactggggaagcaaaatgagctaagtctttacagcaactgtcatttgtgagagtaaacc
attagaaacggacggcaggtttgtggtagattccacaaggtggtggaactagtcgcatatgtagttcagacttgaaggcacacacgtatggggacagttct
ggtgtttacgataaacaggtatgctgccatccagctagcacagcactaggctaaagcgtactgagcccttgtcttccgtgggagctgcagagtgggatgc
atgcgttgtgagctgaggctcaagctgcgctggcagaagagcaggggttgccttgtcagactccagggtctctttctctctgagcctgggaaagtgccact
ttattggatctataaagccggggggggggggggggggggaggaatctcaaggtgaagaggaagttcacagacccctctaacgcctcattagaaccttcca
gctattctctcatacttgtacactgagctggcacacagtataggcaagttctattcgcatcaccccctcagttcctgtctccctggttatgcaagcctcatattta
ggtagatgtgacccttaggaaaccaaaatatcctttaagatcttactaactggttgcctgttcagcttttccacattgatcctgtagcccctcgaggaggtgaa
ggaaaaaaatctcctctttgtttctctaactcattaatgaattttaagggcactctgtaaggttcctttcccattctggtctggttcgtacattctgagaaacacact
gtgtttgtgttgagagttggctccctagctacactgtctgtcacattgatgctctgagtagggacagggttcatctaggaaatatattttcactcacactctgtat
cttttcctagtttggcatattctagtctgcatttggctctctgtttaaatataaaagaaaactaaaacacacccttcagacgcctatgtctgaaaaatctggcattt
ccgtgggtttttctttaaggaggccttcatttgtaaccaacaccatgctctccttaaggaaatcaatctcaatgccctattatccttccctttctttcctcccagttt
gaggctgcagttgcctttttttttcttatcccctgctgaacctgaaaaaccctctctttttctacagttttctgttcccaggccccgctgacttcctttagagcatgg
gggggggggggggatcaggattgtgatgtgtgaactgggaggatcttgacctactccgctaacccagtggcctgagcaaatcacaaggaggattggagc
catctgcccagcccctcccccacggcagcctgctgaaagagacaagttagtcattcaaatgattggcttttgcccgcttcttctctaaataagaaggcag
cagcttctgctgaggtgcaaagggttttgtgaattacggtggtgtgggtaagacccgagttcctccagttcctgcctattttcagctgactaggcggttaagga
tggtagatgctttctgcgcaacctggaagctgacagacagtcagaattttgatgagtacatgaaagctctgggtaggtaaaaataagacgtactgttttcttct
taactctgtattttgatccttagaaaccccccgattttacttaccaggtttctgaaagacaggctgagcacattgtgtcaattatactaagactgtgcatagccca
ctcaaataattcttactgtggtattttcctgccttcttgcgaagaatcaaataaagggaccatttgatatcctgtggataatgtgaagcttctgtaacttttggaga
agggagtgagaaggtccttatttgtgtcttaccttgtccatggctagtggagtaaccattaatcaggtttggacgaaatctacacttttagaatccaattctggatt
cttagagtgacaagcagatttctgaactattttggaagttgctgttaactgcgctggctccctttgctgtttcaggcgtgggctttgccactaggcaagtggga
aacgtgaccaaaccaactgtgattatcagtcaggaaggtggcaaagtggtgatccggacacaatgcacattcaagaacacagagatcaatttccagctg
ggagaagagtttgaagaaaccagcatagatgacagaaactgtaaggtaagaggcagctttatccggtgaggatgggacagaggattcccaatctctcca
cagacatggagaaggggaacaagctttcagaatcccccttcaggaagaaggagccagagctaacattctagggtttactggttgtatcccagacactga
gtaatttccagagccaaacttgcaggctatttatcctttttcacatttttatagcaacttttaccagagagtattgaaccatgtttatttgaaagaaagaccacac
cttgttccttctctctgatcatctcagcttgaccaattagtccgtcgcacagcaaagctttgcttaagcaagaagagtgactagttctgtattttatttctgatcta
tgctggttttttgcaactggctgtatacatggcagctgaatcataaatggccacattttagaaaacagagattcttgaaagtaacagaggatgaagtgctgct
ggaataattatgagtgatgtactcagttatggccctctctgataatgctgtcgtttgacttctgtgaaaagtggacttctgcctgttaacccccctttgattggttgt
tgctctcagtctgtggttcggttggatggagacaagctcattcatgtgcagaagtgggatggcaaagaaacaaattgtaccagagaaattaaggatggca
agatggtcgtggtaagtcctaaggagtctgcctttctgtccctgcttcccttctaagcatcattcttttgcactcccctctggactcttcttccttagcccccact
tttgctctcccccaaccccttattttcatctcagtttctcctgccgttcaaagtcccttaatatgtaaaacaggaggaaggaaaaactcagaactagaagaagc
ctcaggttggcacctgtagagtgtttaaaattatacatcaatttaccccaaaagtgtgtgtcaagaaccgtggtcatgggattgctgtgaatgtccagccttcaa
ctggaacttaaatgtaagctactcattaggaaaatatttgaagataactcataggaaaacataaagtaacgaaatatcagagtagttacctattagagactgt
aattaaggcattttaaacatttttaaattttagtttataagagctcagtgcaaataattttaacaaaaggtgaagctttaattttttactagatctgtttgatgttactccc
ttacagttgtttttcatatttaatgaatcagctaagttttcaagatatgtccagtgacagctaggcatataaatatcctaagatagaaaataattttataaataatttc
cctcatgatagaagttaggccttgatctagacctgtagagacaattttaaaaatagaaatatttagatgatagtgatatgtggtaagtttgattattactcttgagg
atgtctggggttcccttacaaactcctgctaagattttctagctgcctttctagtgcctttctggctgctggtacagaatagattgctcacttctttactatcacagt
cagaactattctgtacttaaatacacttcttctcacatgtagaatgttggagagcagggagcagagattttcttgaataatcaacaaagaaaagagttcatttc

taacaattatcctaaagttatcttttatgagtagccttgattcctttcttgtttatacaaagctagagcaagctcatctttagaaatcctgactgtggtcaggcaa gactgagttctgcaagaactcagattacagaaaggcatactgaattctgagggtttaccaatgcttggaacgtcttgggtataaaccttaatactttttttttgta gtaattcttcactttcagagtaagagcatcaaaattcagtgcccctgccacctaagcatctagattatgttcacatgagctaaacatgacagtcaaagtagag agaacaccgtactggcatgtccttgctgagcttaaaataagtcactcctgataccatgagctcaaaggcttgtattttctaaagaaacccatcaggattgtgg atagaaaactgttatgaaaatctatgaaaaatcgaaatccctttcaaagcagacaattgaggccacagtgacatgatgcgtttttcttggatccaatttcttctg gttcccttagcaattttacaggaaaggaatttttaaaccatagtttccaattcagtttctacttcctttttcatcttcataaatttgaataagatgagcatgcttttcac ttattaaacatgagcatcttttttgtttggttaggaaacaatgaatatatgcaacatttaatgcttgcctatgtcctcattctttggtctaatatttaagtcatataaatg acaatgtttaaaatttcagcagtgaatggtcttaggtaaaacgattcaattatctttggactatttcagactcttacctttggggatatcgttgctgttcgctgttat gaaaaggcatagaagagatgacaggacttttgacaacttggtttcctggagtcccagagttagccagctaatgaagcactggtcactaattataagtttgtct tcggtgtggaggtagaaaattataattctaagatgtgttactccaagcaattcatgggattttgatatgcaaatttactgttttccattttttttaaataattttacaat actttttatagaaatgggaatatacttatgactttctttggaatgcaaaccaaattgaaataaaaaatacacacacatatgatagctggttgtcattttaataccg tcaatctatcttgaattttccatacagtagcctttcttatagcataaagtaaggctgggaaaaaagtcttgtatcttgtataagatactcattcataagatagcttgat gacccagaaaaatatggagatgcccacatatttgagactatgatgaagaacctggaattagtaagttcatctggtcaggacctaaggaagttggttatagtaa gactacaaaaatgatcttttgctattaaaaacagtataaaggaaaaaaaaacaggcttcaacatgtgaccacaggtaaatataatcaagtgccatggaatca cagtgccatttatcaaagttttatagcaaagacaatttgaaaaaagaagtcaatttatcctccactcttagcacgagtctccaaacactgcactacactgaac tttacaaagtcaagaatggtacaatgaagcctatacagagaatccatgaaaatgttccaattccactttttcagggggtaggaagaacaggtaattagaac agagct

SEQ ID No.10


MVDAFCATWKLTDSQNFDEYMKALGVGFATRQVGNVTKPTVIISQEGGKVVIRTQCTFKNTEI
NFQLGEEFEETSIDDRNCKSVVRLDGDKLIHVQKWDGKETNCTREIKDGKMVVTLTFGDIVAVR
CYKA


SEQ ID No.11

EP 1 558 760 B1

agcacagtgctgccctccgtaggctccgggttgtgctgggtgaggcttgggttgggttggcccggcggctgcgtgaactgcggagctggacctagcag
gcttacagttcctcctagcatgaccgagatctgatcagccaacccgcgcattgcttttgtgcctggcactgcagtgcaggggggcctcttcatcgccccaaa
ctacagcactgtcatggcagctgcctctacttccagccctgtaatttcacagccccagttcacagccatgaacgaacaacagtgcttctacaatgagtctat
cgccttctttataaccggagtgggaaatatctagccacagaatggaacacagtgagcaagctggtgatgggactgggcatcactgtttgcgtgttcatcat
gttggccaatctcctggtcatggtggcaatctacgtcaaccgccgcttccatttccctatttattacttgatggccaacctggctgctgcagacttcttcgctgg
attggcctacttctacctgatgttcaatacaggacctaatacccggagactgactgttaacacgtggctcctccggcagggcctcattgacaccagcctga
cagcttctgtggccaacctgctggctattgctatcgagaggcacatcacggtttttccgcatgcagctccatacacgaatgagcaaccggcgcgtggtggt
ggtgattgtagtcatctggactatggccattgtgatgggtgctatgcccactgtgggctggaactgcatctgtgatatcgatcactgttccaacatggcaccc
ctctacagtgactcctacttagtcttctgggccattttcaacctggtgacctttgtggtcatggtggttctctacgctcacatctttggctatgttcgccagagga
ctatgaggatgtctcggcatagttctggacccaggaggaatcgggacaccatgatgagccttctgaagactgtggtcattgtgcttggtgccttattgtctg
ctggactccgggattggtcttgttattgctggatgtgtgctgcccgcagtgcgatgtcctggcctatgagaagttcttcctcctcctggccgagttcaactctg
ctatgaaccccatcatctactcctaccgcgacaaagagatgagcgccaccttcaggcagatcctgtgttgccagcgcaacgagaaccctaatggccccca
cggaaggctctgaccgctctgcctcctccctcaacccacaccattctggctggagttcacagcaacgaccactctgtggttagaaggaagcagccggcct
ctgtggatctgtgaaccccaccctacccccccattgccagggcaaggtggggagccagaggagatgaggacactcctgtacttaacactaaccaatggc
agtatttgtccctagacccaagagacttgaggatgaatttatttggcaggccccatcttctcctttggaaaacagaaggggggaccgtcttgtggtggaattgag
aaatggactctggggtgaccgtgtagcattcactaactagacttaaaaagattttatgtggtttggcttaagccaggaaaaaaaaaatctgctgaattgagtata
caatcgagtatacacaggcttccccctttaaagaacaaacaatacattgcatttattaatgagtatgtttatgcctgacagcatgtttgtgatcgaaaagactgct
aaactgacatagatgagttgttttttttttttttgttttttgttttttttacatgatggaggaaaagtataaaattagaatgattttttgtgtttgtttagaaagcaagcatgtg
gtgtgtgtattcagtatgcctttctttaaagataaaaaggccactattttaaatcttctagggaatagaagaatctagtaaaaaccagtattcatttaggctacagg
aaaaaccatatcctaatcaattaccttttaattaaagtaatgaaatatacatgaaaggcaaagtaatgtgagcttgtcacccaaagagtgtgtgctctccaaac
gctggaggagatgaagctgtagcgttgtccctgcatagtgaagatacccacgtgcgttctcagtgccagaccctcagtgggacttgttttaaagcctgtggt
tttccaagttagaaaataatacctacttactatagaaaacttgaaaattgcagaactgtgtgaaaaaagaaaaaagaaagaaagattgctgatatacgacct
ggaagtagccatctttccctgctcacccacgtatgcctatagacatatattatataccttttttttttttttacataatcgggttgatattgtaaaatgttttgtaactttt
ctttcagaattgccaggttttctatggcatttttttttcaaaaacgttagcggctatataatattccatttaatggatgcagctcagtttatttagtaccattcctgtatt
gttgactatgcttttggcttttttttttcataatattggaagaatcttgtgtatataaaactttgccagtatcattgatgatttacttgggctccattcccatgaaggacct
acttttaaaatgtgaaatgtcctcatgtgttgtcacttttataaaaagggatattctaccctcaaactgcagggggtgaccatgctgacaaaaagctcagaaatta
cctttttacaaaagaaacacagtggctactttagttgcgaatgggttcttgacaagatgtttcccaataacccagacccttaacataactagcaaatttacttta
gtaggaactggtgatccctttctggacacaagaaaaacaaagacatcaagcatcagagtgaatccagcaatgcagtaccaagcagcctgggtggggt
gtgctagcaactggctgctgtgtggtctccagcaatcacccaatggacaccctgtgttcagagatgcccagtacagtgtcctggagactaaggtctctaat
gtcatgctatgaggaacaaggaggaagaagaatagtcctgaagcaggtgtggagaggtcaggaggacgtctgagagaaggcagtcctgccaagagt
ggcattctagggaggaggaaggcagggtgtggcttgtgtctggacagtcagtggtaggcatgtgacatttgcctggtggaaaaaaaataagtaggcgga

gatccagttagcaggacaaagattttgctcgaggattcccccaatccaagaaatttaaactggaaatgagtgaaccgaacttggactttattgattcccttt
tatgggggaggtaaggactatttgaattgaaaagcatactaattgaacccttaataaaatcattctcaatcagtgtttggcgatgtgtggttctggtctgtgctttttt
tctattttcgagaagcctctggctgtgctgtgatgagcttctctctctgaccttctttaacagtctgaggcagcccaacaactgttcctttagctcagatactggtt
cctcatccatgagattcatgagagacgtgttacctcaatggaatgagtactagagcaaggtatttagagagatttttttttattatttatttatttattttgaataaaat
gtatgtataaataagataaaataaa

SEQ ID No.12

MAAASTSSPVISQPQFTAMNEQQCFYNESIAFFYNRSGKYLATEWNTVSKLVMGLGITVCVFIM
LANLLVMVAIYVNRRFHFPIYYLMANLAAADFFAGLAYFYLMFNTGPNTRRLTVNTWLLRQGLI
DTSLTASVANLLAIAIERHITVFRMQLHTRMSNRRVVVVIVVIWTMAIVMGAMPTVGWNCICDI
DHCSNMAPLYSDSYLVFWAIFNLVTFVVMVVLYAHIFGYVRQRTMRMSRHSSGPRRNRDTMM
SLLKTVVIVLGAFIVCWTPGLVLLLLDVCCPQCDVLAYEKFFLLLAEFNSAMNPIIYSYRDKEMS
ATFRQILCCQRNENPNGPTEGSDRSASSLNHTILAGVHSNDHSVV

SEQ ID No. 13

31

cccagagataagctgacgctgggcaaacccctggggggaaggttgcttcgggcaagtagtcatggctgaagcagtgggaatcgataaagacaaaccca
aggaggcggtcaccgtggcagtgaagatgttgaaagatgatgccacagagaaggacctgtctgatctggtatcagagatggagatgatgaagatgattg
ggaaacataagaacattatcaacctcctggggggcctgcacgcaggatggacctctctacgtcatagttgaatatgcatcgaaaggcaacctccgggaata
cctccgagcccggaggccacctggcatggagtactcctatgacattaaccgtgtccccgaggagcagatgaccttcaaggacttggtgtcctgcaccta
ccagctggctagaggcatggagtacttggcttcccaaaaatgtatccatcgagatttggctgccagaaacgtgttggtaacagaaaacaatgtgatgaag
atagcagactttggcctggccaggatatcaacaacatagactactataaaaagaccacaaatgggcgacttccagtcaagtggatggctcctgaagcc
cttttgatagagtttacactcatcagagcgatgtctggtccttcggggtgttaatgtgggagatctttactttaggggggctcaccctacccagggattcccgt
ggaggaacttttaagctgctcaaagagggacacaggatggacaagcccaccaactgcaccaatgaactgtacatgatgatgagggattgctggcatgc
tgtaccctcacagagacccacattcaagcagttggtcgaagacttggatcgaattctgactctcacaaccaatgaggaatacttggatctcacccagcctct
cgaacagtattctcctagttaccccgacacaagtagctcttgttcttcaggggacgattctgtgtttctccagaccccatgccttatgaaccctgtctgcctca
gtatccacacataaacggcagtgttaaaacatga

<br>

SEQ ID No.14

PRDKLTLGKPLGEGCFGQVVMAEAVGIDKDKPKEAVTVAVKMLKDDATEKDLSDLVSEMEM
MKMIGKHKNIINLLGACTQDGPLYVIVEYASKGNLREYLRARRPPGMEYSYDINRVPEEQMTFK
DLVSCTYQLARGMEYLASQKCIHRDLAARNVLVTENNVMKIADFGLARDINNIDYYKKTTNGR
LPVKWMAPEALFDRVYTHQSDVWSFGVLMWEIFTLGGSPYPGIPVEELFKLLKEGHRMDKPTN
CTNELYMMMRDCWHAVPSQRPTFKQLVEDLDRILTLTTNEEYLDLTQPLEQYSPSYPDTSSSCS
SGDDSVFSPDPMPYEPCLPQYPHINGSVKT

<br>

SEQ ID No.15

ctcgagtcctcacggcgtgcaccatgagcggcggcgaagtggtttgctcgggatggctccgcaagtcgcccccggagaagaagttgaagcgttatgcg
tggaagagaaggtggtttgtgttgcgcagtggccgtttgactggagacccggatgtcctggagtattacaaaaacgatcatgccaagaagcctattcggat
tattgatttaaatttatgtcagcaagttgatgctgggttgacattcaacaaaaaggagtttgaaaacagctatatctttgatatcaacaccatcgaccggatttc
tacttggtggcagatagtgaggaagacatgaacaagtgggtccgttgtatctgtgacatctgtggattcaatcccacagaagaagatcctgtgaagccgct
gactggctcctcacaagcaccgtcgattcacctttcgctataagtacagcaccagcctccagtcagatggaagcttcttcagtcgcgctacctcctcctta
ccaggtcatcagccttccgccacacccagacaccctcggcctccaggacgatccacaagactacctcttgctgatcaactgtcaaagcaagaagcctga
acctaacagaaccctctttgactctgccaagcccacctttctgagacagactgcaatgacaacgtcccttcccaccagactcctgcttcctcccagagca
aacacggaatgaatggcttttttgcagcaacaaatgatgtatgactgcccaccgtcccggctgacatctgtctcgggagagtccagcctctataacctgccc
aggagctattcccatgacgtgttgccaaaggaatccccatcaagcacggaggccgacggggagctgtacacctttaacaccccatctgggactgcaggt
gtagaaacgcagatgagacatgtatccatcagttacgacattccgccaacacctggcaacacttaccagatcccacggacatttccagaaagcacactg
ggacagtcatcaaagctggacaccattcctgatatcccccccacctcggccaccaaagccacatccaagtcatgaccggtctcctgtgaaacgtgtgga
gtcccacgcacggcctcggacactgacagcagttactgtatccctcctccagtaggcatgacgccctcccggagtaataccatttccaccgtggatttgaa
caagttgcggaaagatgctagttctcaagattgctatgatattccacggacctttccgagcgatagatctagttccctggaaggcttccatagccagtataaa
atcaaaagcgtgttgacagcgggaggtgtctcgggtgaagagctggatgagaactacgttcccatgaaccccaactcgccacctcgacaacattccggc
agctttaccgagccaatccaggagccaaactatgtgccaatgaccccagggaccctttgacttttcttcctttggaatgcaagtccctcctcctgctcatatgg

<br>

gcttcaggtccagcccaaagacccctcccaggaggccagttcctgttgctgactgtgaaccaccccggtggataggaatctcaagccagacagaaaagtcaagccggcacctttagacataaaaacctctgtcagaatgggaagagctgcaagcccccagtcagatctcccatcaccaggagcttcgctcgggactcctctaggtttcccatgtcccctcggcctgattctgtgcacagtacgacatcgagcagcgactctcatgacagtgaagagaactatgtccccatgaatccaaatctgtctggcgaagacccgaatctctttgccagcaacagccttgatggggggaagcagcccgatgaataaacccaaaggagacaaacaagtcgaataccctggatttagacctagattctgggaagtccacgccaccacggaagcaaaagagcagtggttctggcagcagcatggcagacgagagggtggattacgttgtggtggaccaacagaagactctggccctgaagagtaccagagaagcttggacggatgggaggcagtccacagagtccgagacacccacaaagaatgtgaagtgaagacatgccgtcgcctctgcgggaagacgagatctgagtggaaagagagatgccaagtgaagatgttcccactctcagtggagcctcgagcagaggggaagagagaaggatctctcacacatgttcaagcaaattaggttgtgaatggtgctgtgtggtattggatttataacgtgtaaataacccgggggaaatagtgttttagttcacagagaagctttctgtccctaattaacacacctgtagtattactatactgatgcacgttttcatttaaaaaccttggtttgggtcttcccgatctaccttaacagactttccttggggaggtcttttggcctcctcacactactctatataacaatactaagtgaactgagctacttgtaattctggaaattccagttgaagctacagggctaacaccattaaaacaagaagtaagttgacacattcgcttttctcttgaaggtggtagccattagcttaagctgtagaacatagttggccg

SEQ ID No.16

MSGGEVVCSGWLRKSPPEKKLKRYAWKRRWFVLRSGRLTGDPDVLEYYKNDHAKKPIRIIDLN
LCQQVDAGLTFNKKEFENSYIFDINTIDRIFYLVADSEEDMNKWVRCICDICGFNPTEEDPVKPLT
GSSQAPVDSPFAISTAPASSQMEASSVALPPPYQVISLPPHPDTLGLQDDPQDYLLLINCQSKKPEP
NRTLFDSAKPTFSETDCNDNVPSHQTPASSQSKHGMNGFLQQQMMYDCPPSRLTSVSGESSLYN
LPRSYSHDVLPKESPSSTEADGELYTFNTPSGTAGVETQMRHVSISYDIPPTPGNTYQIPRTFPEST
LGQSSKLDTIPDIPPPRPPKPHPSHDRSPVETCGVPRTASDTDSSYCIPPPVGMTPSRSNTISTVDLN
KLRKDASSQDCYDIPRTFPSDRSSSLEGFHSQYKIKSVLTAGGVSGEELDENYVPMNPNSPPRQH
SGSFTEPIQEPNYVPMTPGTFDFSSFGMQVPPPAHMGFRSSPKTPPRRPVPVADCEPPPVDRNLKP
DRKVKPAPLDIKPLSEWEELQAPVRSPITRSFARDSSRFPMSPRPDSVHSTTSSSDSHDSEENYVP
MNPNLSGEDPNLFASNSLDGGSSPMNKPKGDKQVEYLDLDLDSGKSTPPRKQKSSGSGSSMADE
RVDYVVVDQQKTLALKSTREAWTDGRQSTESETPTKNVK

SEQ ID No.17

gtcgacccacgcgtccgggcggcggctgcgggtggtggacctgcagacggccgtcgcccacacctcgctctctcctgggccggcggatccttttgtctctccgagcggcgaaggtcgcagagcaggcgcggcggctgcgggacgcaaacgcccgctctctcggtcacctccccagcctcggccaccgcagaacttgggctcgagaagccagcggaccgcgtccagctccagagccggtttagtctccactgccagctgccatgggcaagcagaacagcaagctgcgtcctgaggtgctgcaggacctgcgggaacacacggaattcaccgaccatgagcttcaggagtggtacaagggcttcctcaaggactgccccactggccacctgactgtggacgagttcaagaagatctacgccaacttcttcccctacggcgatgcctccaagttcgctgaacacgtcttccgcaccttcgacaccaacagcgatggcaccatcgacttccgggagttcatcattgctctgagtgtgacctctcggggcaagctggaacagaagctcaagtgggcctttagcatgtacgacctggacggcaatggctacatcagccgcagtgaaatgctggagatagtgcaggccatctacaagatggtgtcctccgtgatgaagatgcctgaggacgagtccacgccccgagaagcgaacagacaagatcttcaggcagatggacacaaacaatgatggcaaactgtccctggaagaattcatcaaaggtgccaagagcgacccatccattgtccggttgctgcagtgtgatcccagcagtgctagccagttctgaggggaatgggcttttggacatttgtgagaaacacaggcttggcctgccatcttcagctttgcttgtaaaagtggatttctccgtgatctctcctgctctcccaggacctgggtccaggcagcaatcgcaccctcctgacctggccaactgtggcttcctcctcctccagtccaggctggctccttccaatggttcaagtgttctcggctgtcgggctccttcctgcccacagagggcctggcggcccttaaggacaccagacaggacttttcagtatcttaccaagaccagctaatttattctatgatcccaggtgactttgtgaggatgaaaaagatggagagtagacaggcaagcccttcctgtgcatggctccacctcccttgttcatttctctgaccaaagccgcttgcatgtatataccgtggtatccttacttttaatatatgagttatttgtatggtgagatggactataatgttcagcccctgtatttaatgcctctgactgcctttgaagcatggtccctgtggcctgtgattccccccaccccccaagtctggctgtgctgtggtatttatgcattcatgatttgcctgattccacaggagatacatgcatgcccagggctgtggacatctgccctccgtcctgtaaatgcaggtgtttctgtgtgctctgtccttcctgtcgattggtatggcatattaaaatgatcaaat

SEQ ID No.18

MGKQNSKLRPEVLQDLREHTEFTDHELQEWYKGFLKDCPTGHLTVDEFKKIYANFFPYGDASK
FAEHVFRTFDTNSDGTIDFREFIIALSVTSRGKLEQKLKWAFSMYDLDGNGYISRSEMLEIVQAIY
KMVSSVMKMPEDESTPEKRTDKIFRQMDTNNDGKLSLEEFIKGAKSDPSIVRLLQCDPSSASQF


SEQ ID No.19


ggatgagacagaaggatagagaggaggagagagagagagaagagaagcaaccagaaataggcagccaataaaaaggagccgcacttatctga
agcctcaagggggcctgagccaggtccctgtttgatggcagttatgaaaaattacctcctcccgatcctggtgctctccctggcctactactactattctacaa
atgaagagttcagaccagaaatgctccagggaaagaaagtgattgtcactggggccagcaaagggattggaagagaaatggcatatcatctgtcaaaa
atgggagcccatgtggtattgactgccaggtcggaggaaggtctccagaaggtagtgtctcgctgccttgaactcggagcagcctctgctcactacattg
ctggcactatggaagacatgacatttgcggagcaatttattgtcaaggcgggaaagctcatgggcggactggacatgcttattctaaaccacatcactcag
acctcgctgtctctcttccatgacgacatccactctgtgcgaagagtcatggaggtcaacttcctcagctacgtggtcatgagcacagccgccttgcccatg
ctgaagcagagcaatggcagcattgccgtcatctcctccttggctgggaaaatgacccagcctatgattgctccctactctgcaagcaagtttgctctggat
gggttctttccaccattagaacagaactctacataaccaaggtcaacgtgtccatcactctctgtgtccttggcctcatagacacagaaacagctatgaag
gaaatctctgggataattgacgccctagcttctcccaaggaggagtgcgccctggagatcatcaaaggcacagctctacgcaaaagcgaggtgtactat
gacaaattgcctttgactccaatcctgcttgggaacccaggaaggaagatcatggaatttttttcattacgatattataataaggacatgtttgtaagtaactag
gaactcctgagccctggtgagtggtcttagaacagtcctgcctcatacttcagtaagccctacccacaaaagtatctttccagagatacacaaattttggggt
acacctcatcatgagaaattcttgcaacacttgcacagtgaaaatgtaattgtaataaatgtcacaaaccactttgggcctgcagttgtgaacttgattgtaac
tatggatataaacacatagtggttgtatcggctttaacctcacactgaatgaaacaatgataactaatgtaacattaaatataataaaggtaatatcaacttcgta
aatgcaaaaaaaaaaaaaaaaaaaaaaaaaaa


SEQ ID No.20


MAVMKNYLLPILVLSLAYYYYSTNEEFRPEMLQGKKVIVTGASKGIGREMAYHLSKMGAHVVL
TARSEEGLQKVVSRCLELGAASAHYIAGTMEDMTFAEQFIVKAGKLMGGLDMLILNHITQTSLS
LFHDDIHSVRRVMEVNFLSYVVMSTAALPMLKQSNGSIAVISSLAGKMTQPMIAPYSASKFALD
GFFSTIRTELYITKVNVSITLCVLGLIDTETAMKEISGIIDALASPKEECALEIIKGTALRKSEVYYD
KLPLTPILLGNPGRKIMEFFSLRYYNKDMFVSN


SEQ ID No.21


ggagatcaggccaccacgagacagagatggagaccagcagcctgtggcccccgaggcccagccccagtgcagggctgagcctggaggcgcggct
gggcgtggacactcgcctctgggccaaggtgctgttcaccgcgctctattcgctcatcttcgcgcttggcacagccggcaatgcgctgtccgtgcacgtg
gtgctgaaggcgcggacgggtcgccccgggcgcctgcgctaccacgtgctcagcctggcactgtcagccctgctgctactgctgatcagcgtgcccat
ggagctctacaacttcgtgtggtcccactaccctgggtcttcggcgatctcggctgtcgtggctattacttcgtgcgcgagctgtgcgcctacgccacggt
gctgagcgtggccagcctgagcgcagagcgctgcctggccgtgtgccagccgctgcgcgccccgccgcctgctcaccccgcgccgcacctgccgcct
gctgtcactggtctgggtcgcctctctgggccttgccctgcccatggcgggtatcatgggacagaagcacgaaatggagagggccgacggggagcctg
agcctgcctcgcgtgtgtgcacggtgctagtaagtcgcgccagctccaggtctacattccaggtgaaacgtgctggtctccttcgttctcccctttgggaac
tcactgctattctgaatgggatcactgtcaaccacctggtggccctctactcccaggtaccatcagcttctgcccaagtcaactccatcccagccgcctgg
agctcctgagtgaggaaggcctcctgggcttcatcacatggagaaagaccctctccctggggggtccaagccagcctggtgagacacaaggatgccag
ccagatccgcagcctccagcacagcgcccaggttctcagagccatcgtggctgtgtatgtcatctgctggctgccgtaccatgcccgcaggctcatgtac
tgctacatccccgatgatggatggactgatgagctctatgacttctatcactatttctacatggtgaccaacacgctcttctatgtcagctcggcagtgacccc
agtcctctacaatgccgtgtcttcctccttcagaaagctctttctggaatctctcagttccctgtgtggtgaacagcgctccgtggtgcccttaccccaagaag
ccccagagtcaactactagtacgtacagtttccggctttggggatccccaagaaaccccagcctgggtgaaatacaagtgtgaagagaacaaacaatgg
ctgcttgggacacacccatcagataagccatgccattactaacagtctaagcggacctactgacccagcgcagtcattgaccagtgcatactgcaggcaa
gccacgtaacacctcctgccctcagcttcccacctgtgcaaccaaggtgtagaataggacaaattgcctagtgatgaaggtgcccagtgccagcctggta
cagaaccagtactccataaattttagctggtgctattttttaaaaaaaaaaaaaaaaaa


SEQ ID No.22

METSSLWPPRPSPSAGLSLEARLGVDTRLWAKVLFTALYSLIFALGTAGNALSVHVVLKARTGR
PGRLRYHVLSLALSALLLLLISVPMELYNFVWSHYPWVFGDLGCRGYYFVRELCAYATVLSVAS
LSAERCLAVCQPLRARRLLTPRRTCRLLSLVWVASLGLALPMAVIMGQKHEMERADGEPEPASR
VCTVLVSRASSRSTFQVKRAGLLRSPLWELTAILNGITVNHLVALYSQVPSASAQVNSIPSRLELL
SEEGLLGFITWRKTLSLGVQASLVRHKDASQIRSLQHSAQVLRAIVAVYVICWLPYHARRLMYC
YIPDDGWTDELYDFYHYFYMVTNTLFYVSSAVTPVLYNAVSSSFRKLFLESLSSLCGEQRSVVPL
PQEAPESTTSTYSFRLWGSPRNPSLGEIQV

SEQ ID No.23

ggcgctcgccctcgctcgccatggagaagaccgagctgatccagaaggccaagctggccgagcaggccgagcgctacgacgacatggccacctgc
atgaaagccgtgacggagcaggcgccgagctgtccaacgaggagcgcaacctgctgtcggtggcctacaaaaacgtggtaggggggccgcaggtc
cgcctggagggtcatctcgagcattgagcagaagaccgacacctctgacaagaagttgcagctgatcaaggactatcgggagaaagtggagtcggag
ctgaggtccatctgcaccacggtcctggaattgttggataagtatttaatagccaatgcaactaatccagagagtaaggtcttctatctgaaaatgaaggga
gattatttccggtatcttgctgaagtagcttgtggcgatgatcgaaaacaaacaatagaaaattcccaaggagcctaccaagaggcgtttgatataagcaag
aaggagatgcagcctacgcatccaatccgcctggggctggctcttaacttttctgtattttactatgagatccttaataatccagagcttgcctgcacactggc
taaaacggcttttgatgaggccatcgcagagcttgatacactgaacgaagactcctacaaagacagcaccctcatcatgcagttgcttagagacaacttaa
cattatggacatcagacagtgcaggagaagaatgtgatgcagcagagggggccgaaaactaaacc

SEQ ID No.24

MEKTELIQKAKLAEQAERYDDMATCMKAVTEQGAELSNEERNLLSVAYKNVVGGRRSAWRVI
SSIEQKTDTSDKKLQLIKDYREKVESELRSICTTVLELLDKYLIANATNPESKVFYLKMKGDYFR
YLAEVACGDDRKQTIENSQGAYQEAFDISKKEMQPTHPIRLGLALNFSVFYYEILNNPELACTLA
KTAFDEAIAELDTLNEDSYKDSTLIMQLLRDNLTLWTSDSAGEECDAAEGAEN

SEQ ID No.25

ttttttttttttttttttcccatcccctgctggctttattactgggcaggggaaacagatccttggcagtggtaggactgagagtacaagagttggaagccctggg
gctcagctttcactaggcagggtgggcacactagcaagtgcagagaggaggcctgtatatatgtcaaccccacggagaaatatatcctcatgcagccgtt
cattatggtcatgtagcaggacaggtgtgcggttcatgggcgagaagcccagagctgggatccccaccgcccggataaagcggctgtcagtggcagc
aggaaagatctctggctccagagtgaggttcattgccttgcaggctccgctgaaagctgcccaccagggatctgaatcatccgtgggtgtcattcgaggc
tctgtaaacttctga

SEQ ID No.26

gccatcaatgctttattccttcatctctgtcctgtccaacactagtgaacacacacctctggctcattaaggaagtgacatgcagatgagcaactaggataga
aacatcattcacacactcttctcaggtgttcctccggtgcacactcacacgagtttgcacatactataagcctgaataatgatgcccattatctgcacagggt
aaaactgattccatactggtgcaacctggtggaagtcttcaggacacttaacaaaattatattctgattattcattttctagatttggggatgaaaatagaaaag
aatataaacaaagcatgctttttgaggtttcttgggtcagttagtagaaaaaataggtgtttctttgagaggtttttttaattgtttatcttattttattttttaagtggag
aactgtgaaactggaattcctgaggtttttttttttccccacttcatcagtctttaagagagtggtaggctttggcagtcccgctgttgacaaagtctgttttcttaa
actgagccttcttataggcgtgatgcagctctgtatgtgcccacagagagtaaaggaggacagaagctgctttgcttgccttngtggggcatagcctttccc
tatgctgatgtcatattttctgcaaaacctcgggtccagagggctcgtgcattctggtaactattttgcattaaatgttcaac

SEQ ID No.27

gtgacaggacgcctcccccacacccatattctaagctttatcttctagtagcctgagggctggagagaggtagtttctgccggattgtgtcatctggagtcgt
tcctgtggcctccttttctctggtttttgcatttttcttggtccgatgaaagcatttccttttctaaccaataaagtgatcgctttcagcaatgg

SEQ ID No.28

agcaatgaatgtggaacatgaagttaacctcctggtggaggaaattcatcgcctgggttccagaaatgctgatggaaaattaagtgtgaagtttggggtcct
cttccaggatgacagatgtgccaatctctttgaagcgttggtaggaactctgaaagctgcaaaacgaaggaagattgttacatacgcaggggaactactttt
gcaaggtgttcatgatgatgttgacattgtattgctgcaagattaatgtggtttgcatggcttggtgtatctgataaactggaataactaagttaagagactagc
gtgaatttccttatgtattttatagaactttgtaaacaaagggggggcttgttgagaagtcctgtttttataccttgaagcaaaacattacaatgtaaaatgagac
aaacctattattttttcttaagaaggtaatttgggaaatgtaggtaatgaaacatttttgggaggtgtgaaaaagc

SEQ ID No.29

attggtgcatgtgtgtttgctagctcacttgtccatgagaatattttatgatattaaagaaaacctttttgaaatggctgcttttcaaagaagataattcatggcttct
cattttcagtctcttcaaaagtgtggctggcctgtttttatgactgcagagttgttatgtttttttaattttgaatattgcctattaaagataggacaaacttggagatt
atgatgttgcttggcacagactgtattaaaacaacactcccgtga

SEQ ID No.30

ctggcacggacatggctgtcttctgtctgctgtgtgggaaacgctttcaggcacaaagcgcactccagcagcacatggaggtccacgcaggcgtgcgc
agctatatttgcagtgagtgcaaccgcaccttccccagccacacggctctcaagcgccaccttcgctcacatacaggttttttttctccatgtgtcaccaagtg
aagtttgtgccttctatagcaaagagaatatttttttacatcctactaacagtagatttttttgtagtgaacatttttttgtattttttatttataagtctcataagaaaaata
gcgatgttcagttgtataccttgaatctgcagttagaagagaataaagttaactc

SEQ ID No.31

tttttttttttttttttttgcaagagtaaaagtctttaatttactgtgcacctcagaaagctacaagtaaaatgtttgtaagaatatacacaaagaattcagagtataaaa
ttctccatgtaattagtagtcatattaatattagaactacagtagaaaaaaatagctgtctcctagattctcccaggagcctaactgtgtggctttgagaaggtg
gagttgttctgagtgagcgggaagtcaagctcactcctccagttctcccagcagctccacgaagtcagtgatgtaccacttggcgttgtccttaacctgctg
cctgatcacattgcctccaaagccaatgaaagcatcag

SEQ ID No.32

ggggcgggacgacggcgggcgctgcccggcgcactagccggcttgcgggcgctgccagtctccggcggcggtgtccggcgcgcggctgagcgac
cgagcgtgcggacggagcggcggcctgctgggcgggctgagcggcgcgcgcggcggcggagagacgcggagcgagggacgcggcggcggcg
gacgcggcgacaggtcttctacttacaaaggacaatgactactgatgagggcaccagtaacaatggagagaacccagcagccaccatgactgagcag
ggtgaagatatcactacgaagaaagacagaggagtattaaagattgtcaaaagagtggggactagtgacgaggccccaatgtttggtgacaaagtttatg
tccactacaaagggatgttgtcagatggaaagaagtttgattccagtcatgacagaaagaagccatttgcctttagccttggccaaggccaggttatcaaa
gcctgggacattggggtgtctactatgaagaaaggcgagatctgccattttattatgtaaaccagaatatgcttatggctcggctggccacctccaaaaaatt
ccatcaaatgcaactctcttttttgagattgagctccttgatttcaaaggtgaggatttatttgaagattcaggcgttatccgtagaatcaaacggaaaggcga
gggatactcaaacccaaacgaaggagcaacggtaaaagtccacctggaaggctgctgtggtggaaggacatttgattgccgagatgtggtgttcgttgtt
ggggaaggagaagaccacgacattccgattgggatcgacaaagccctggtgaagatgcagagagaagaacagtgtattctatatcttggaccacgctat
ggttttggagaagccgggaagcctaagtttggcattgaccccaatgctgagcttatgtacgaggtcacccttaagagcttcgagaaggccaaagaatcttg
ggagatggacaccaaagaaaagctgacgcaggctgccatcgtgaaagagaagggaactgtgtacttcaagggaggcaagtacacgcaggccgtgat
tcagtacaggaagatagtgtcctggctggagatggaatacggcctgtcagagaaggagtccaaagcctcagagtcgttcctcctcgcagccttcctgaac
ctggccatgtgctacctgaagctccgagagtacaacaaagccgtggagtgctgcgacaaggcccttggactggacagtgccaatgagaaaggcttgta
cagaagggggcgaggcccagctgctcatgaatgacttt gagtcggccaagggcgacttcgagaaggtgttggcagtcaatcctcagaacagggccgctc
gcctgcagatctccatgtgccagaggaaggcgaaggagcacaacgagcgggaccgcagggtgtacgccaacatgttcaagaagttcgcagagcggg
acgcaaaggaggaagccagcaaagctgggagcaagaaggctgtagaaggagccgctggcaaacaacacgagagtcaggccatggaagaaggaa
aggccaaaggccatgtatgacgctgcgccacgtgagggaagagagtcctaatgaactcggccctcctcgctgggctcgcctccaactcaggactgaac
agtgtttagtgtaaggtttgttacagtctctgtgattctggaagcaaatggcataccagtagcttcccaaatgaccacctgctgctgcggggggggtgggggt
ggggggacatgccaggaaacagcagagaaggccgctggtgtgaagagaccaggccagcagctcagtccagcccatttcagtttgtcacctttcagtgtc
cagcacagcatccctgtgaacctaggcccagctgctgtgggttctacatcggcactagggtcacactgcagaaaccgttgataaaacaaactcagtgat
ctctgctttcctattggtgggcatggcaggggcgggtgatgagatttgcttagcactgactgactggcctgctaagaacacaagcccacagccaggggct
ccctggtccacagctgggtctcaggcccctt acctgccttccaagtcctttcgcagactcttgagtgtggctttctgtcctagccagcatgtcccacagactc
tgttgttcctccaacgcccgtcattagtgacagctttctctctgagtttctgtggtgtggagagtgggtagaagtaggtttatctttcccgctgtctgccccactc
aaggacgat

SEQ ID No.33

MTTDEGTSNNGENPAATMTEQGEDITTKKDRGVLKIVKRVGTSDEAPMFGDKVYVHYKGMLS
DGKKFDSSHDRKKPFAFSLGQGQVIKAWDIGVSTMKKGEICHLLCKPEYAYGSAGHLQKIPSNA
TLFFEIELLDFKGEDLFEDSGVIRRIKRKGEGYSNPNEGATVKVHLEGCCGGRTFDCRDVVFVVG
EGEDHDIPIGIDKALVKMQREEQCILYLGPRYGFGEAGKPKFGIDPNAELMYEVTLKSFEKAKES
WEMDTKEKLTQAAIVKEKGTVYFKGGKYTQAVIQYRKIVSWLEMEYGLSEKESKASESFLLAA
FLNLAMCYLKLREYNKAVECCDKALGLDSANEKGLYRRGEAQLLMNDFESAKGDFEKVLAVN
PQNRAARLQISMCQRKAKEHNERDRRVYANMFKKFAERDAKEEASKAGSKKAVEGAAGKQHE
SQAMEEGKAKGHV

SEQ ID No.34

atggcgtggtccaggctgatgctggcagcttgcctcctcgtgatgccctctaatgttatggcggactgcctgtccctgtgctccctgtgtgcagtgaggattc
aggatgggccccgtcccatcaaccccctgatttgctccctggagtgccaggacctggtgccgccctcagaggagtgggagacatgccggggcttctca
tcttttctcaccctgacggtctctgggctccgtggcaaggatgacttggaagatgaggttgctttggaagaaggcatcagtgcacatgccaagtcttggaa
cccgtcctgaaggagctggagaaaagccgactccttaccagcgtcccagaggaaaagttcaggggtctctccagcagctttggcaacggaaaagaatc

tgagctggcgggtgctgaccggatgaatgatgaagccgcacaggggcgcaccgtccatttta atgaggaggacttgagaaaacaggccaaacgctatg
gcggcttttgcgcaaataccccaagaggagttccgagatggcccgggatgaggacggggccaggatggggatcaggtaggcatgaggacctgt
acaaacgctatggggggcttcctgcggcgcattcgccccaagctgaagtgggacaaccagaagcgctatggtggtttcctgcggcgtcagttcaaggtgg
tgacgcggtcccaggagaaccccaatacctattctgaagatttagatgtttga

SEQ ID No.35

MAWSRLMLAACLLVMPSNVMADCLSLCSLCAVRIQDGPRPINPLICSLECQDLVPPSEEWETCR
GFSSFLTLTVSGLRGKDDLEDEVALEEGISAHAKLLEPVLKELEKSRLLTSVPEEKFRGLSSSFGN
GKESELAGADRMNDEAAQGRTVHFNEEDLRKQAKRYGGFLRKYPKRSSEMARDEDGGQDGD
QVGHEDLYKRYGGFLRRIRPKLKWDNQKRYGGFLRRQFKVVTRSQENPNTYSEDLDV


SEQ ID No.36


gacagggcgccctcggaaggacaggatgtcatcaggaaaacaggactccccgtgggaagataggatacctccaggaagatagaatagtcccaggca
tcactagaacagcaggaaacactagggtccaagctctcattgaggcacccggggaggttgttgggttgtgggcggggctcaggaaagactgtccctgct
ggtcctgatccacgaccacccacccggcaaggttctctctaagagaaccttgtcagagacagaacgggtccctacaggcgcgttcttctctcctacagcc
catggcgcggttcctgaggctttgcacctggctgctggcgcttgggtcctgcctcctggctacagtgcaggcggaatgcagccaggactgcgctaaatg
cacgtaccgcctggttcgcccaggcgacatcaatttcctggcgtgcacactggaatgtgaaggtcagctgccttctttcaaaatctgggagacctgcaag
gatctcctgcaggtgtccaggccccgagttcccttgggataacatcgacatgtacaaagacagcagcaaacaggatgagagccacttgctagccaagaa
gtacggaggcttcatgaaacggtacggaggcttcatgaagaagatggacgagctatatcccatggagccagaagaagaagcgaacggaggagagat
ccttgccaagaggtatggcggcttcatgaagaaggatgcagatgagggagacaccttggccaactcctccgatctgctgaaagagctactgggaacgg
gagacaaccgtgcgaaagacagccaccaacaagagagcaccaacaatgacgaagacatgagcagcaagaggtatggggggcttcatgagaagcctc
aaaagaagcccccaactggaagatgaagcaaaagagctgcagaagcgctacggggggcttcatgagaagggtgggacgccccgagtggtggatgga
ctaccagaagaggtatggggggcttcctgaagcgctttgctgagtctctgccctccgatgaagaaggcgaaaattactcgaaagaagttcctgagatagag
aaaagatacgggggctttatgcggttctgaagcccttttccagcagtgaccccgaccccactagcctgctccatccccatgagcaactgccttgtcaat
gatgtttcttgtcacatgctgctttgtgctgtacagttgcccccgtggtctagataactacactgcctgaaagctgtgattttagggtctgtgttcttttgagtcttg
aagctcagtattggtctcttatggctatgttgttatcaatagtttgttacctcatctctcctgacgaaacatcaataaatgcttatttgtatataaatataataaaccc
gtgaccccaactgc


SEQ ID No.37


MARFLRLCTWLLALGSCLLATVQAECSQDCAKCTYRLVRPGDINFLACTLECEGQLPSFKIWET
CKDLLQVSRPEFPWDNIDMYKDSSKQDESHLLAKKYGGFMKRYGGFMKKMDELYPMEPEEEA
NGGEILAKRYGGFMKKDADEGDTLANSSDLLKELLGTGDNRAKDSHQQESTNNDEDMSSKRY
GGFMRSLKRSPQLEDEAKELQKRYGGFMRRVGRPEWWMDYQKRYGGFLKRFAESLPSDEEGE
NYSKEVPEIEKRYGGFMRF


SEQ ID No.38


acccacgcgtccggccggtttcactgctcccctcagtctcttttgggctctttccgggcatcgggacgatgaccgtcaaagccgaggctgctcgaagcac
ccttacctactccagaatgaggggaatggtagcgattctcatcgctctttatgaaacagagaaggatgggcctgaacgattttattcagaagattgccagcaa
cacctatgcatgcaaacacgctgaagttcagtccattttgaaaatgtcccatcctcaggagccggagcttatgaacgctaaccctctcctccgccaagtc
cctctcaacaaatcaacctgggtccgtcctccaaccctcacgccaaacctccgactttcacttcttgaaagtgatcggaaagggcagttttggaaaggttc
ttctggctaggcacaaggcagaagaagtattctatgcagtcaaagttttacagaagaaagccatcctgaagaagaaagaggagaagcatattatgtcaga
gcggaatgttctgttgaagaatgtgaagcaccctttcctggtggggccttcacttctcattccagaccgctgacaaactctactttgtcctggactacattaatg
gtggagagctgttctaccatctccagagggagcgctgcttcctggaaccacgggctcgattctacgcagctgaaatagccagtgccttgggctatctgca
ctccctaaacatcgtttatagagacttaaaacctgagaatattctcctagactcccaggggcacatcgtcctcactgactttgggctctgcaaagagaatatt
gagcataacgggacaacatctaccttctgtggcacgcctgagtatctggctcctgaggtcctccataagcagccgtatgaccggacggtggactggtggt
gtcttggggctgtcctgtatgagatgctctacggcctgccccccgttttatagccggaacacggctgagatgtacgacaatattctgaacaagcctctccagt
tgaaaccaaatattacaaactcggcaaggcacctcctggaaggcctcctgcagaaggaccggaccaagaggctgggtgccaaggatgacttttatggag
attaagagtcatatttcttctctttaattaactgggatgatctcatcaataagaagattacacccccatttaacccaaatgtgagtgggcccagtgaccttcgg
cactttgatcccgagtttaccgaggagccggtccccagctccatcggcaggtcccctgacagcatccttgtcacggccagtgtgaaggaagcagcagaa
gccttcctcggcttctcctatgcacctcctgtggattccttcctctgagtgctcccgggatggtctgaaggacttcctcagcgtttcctaaagtgttttccttac
cctttggtggaggttgccagctgacagaacattttaaaagaatttgcacacctggaagcttggcagtctcgcctgcccggcgtggcgcgacgcagcgcg

cgctgcttgatgggagctttccgaagagcacaccctcctctcaatgagcttgtgaggtcttcttttcttctcttccttccaacgtggtgctagctccaggcgag
cgagcgtgagagtgccgcctgagacagacaccttggtctcagttagaaggaagatgcaggtctaagaggaatccccgcagtctgtctgagctgtgatca
agaatattctgcaatgtgcctttctgagatcgtgttagctccaaagcttttcctatcgcagagtgttcagtttgtgtttgtttgtttttgttttgttttgttttcccttg
gcggatttcccgtgtgtgcagtggcgtgagtgtgctatgcctgatcacagacggttttgttgtgagcatcaatgtgacacttgcaggacactacaatgtggg
acattgtttgtttcttccacatttggaagataaatttatgtgtagactgttttgtaagatatagttaataactaaaacctattgaaacggtcttgcaatgacgagcat
tcagatgcttaaggaaagcattgctgctacaaatatttctatttttagaaagggtttttatggaccaatgccccagttgtcagtcaaagccgttggtgtttttcattg
tttaaaatgtcacctataaaacgggcattatttatgtttttttccctttgttcatattcttttgcattcctgattattgtatgtatcgtgtaaaggaagtctgtacattgg
gttataacactagatatttaaacttacaggcttatttgtaaaccatcattttaatgtactgtaattaacatgggttataatatgtacaattcctcctccttaccacaca
acttttttttgtgtgcgataaaccaattttggtttgcaataaaatcttgaaacct


SEQ ID No.39


MTVKAEAARSTLTYSRMRGMVAILIAFMKQRRMGLNDFIQKIASNTYACKHAEVQSILKMSHP
QEPELMNANPSPPPSPSQQINLGPSSNPHAKPSDFHFLKVIGKGSFGKVLLARHKAEEVFYAVKV
LQKKAILKKKEEKHIMSERNVLLKNVKHPFLVGLHFSFQTADKLYFVLDYINGGELFYHLQRER
CFLEPRARFYAAEIASALGYLHSLNIVYRDLKPENILLDSQGHIVLTDFGLCKENIEHNGTTSTFCG
TPEYLAPEVLHKQPYDRTVDWWCLGAVLYEMLYGLPPFYSRNTAEMYDNILNKPLQLKPNITN
SARHLLEGLLQKDRTKRLGAKDDFMEIKSHIFFSLINWDDLINKKITPPFNPNVSGPSDLRHFDPE
FTEEPVPSSIGRSPDSILVTASVKEAAEAFLGFSYAPPVDSFL


SEQ ID No.40

gaagcagaagagggagaacgagggtgagcacgctgggcagccgtgcgtgcgctcctctctgcgccacaggctcgcccaacgaagcacaagccag
cagggtcgcggtcctgaggacatcgctgccacccaggagtgcagagaaccaaccacagaatctggaggtcggtagcagccccgatctaggcaggct
cggagccaggggggggtgccgaggaagaaccggaaagcagatcccagactctgaaggagaaagaagagtggatccctgagccggcacgtctcgag
ccccagagctcccaggatctcctggatagctggcttcgccgccggacggcacgccccccccttagcttccacaccctctcctctgagcgcctcctcatctgc
ccctggagggactttcaccttcaaaggtgcaggacaaagccgcatatctggcgcccaccatgcacctcaacagctccgtgcagcagggagcccccaagt
gaacccggtgcccagcccttcccacatccacagttcggactggagacgatgctcctggcgctcagcttgagcaatggtctggcaattcctcagaatcca
tcctggagcccaacagcaacctggacgtgaacactgacatttattccaaggtgctggtgaccgctgtatacctggcactttttgtggtgggcactgtgggc
aactcggtgacagccttcactctagcgcggaagaagtcgctgcagagcctgcagagcactgtgcattaccacctggggtagcctggcactgtctgacctg
ctcatcctgctgctggccatgcccgtggagctgtacaacttcatctgggtgcaccatccctgggcctttggggatgctggctgccgtggctactatttcctg
cgagatgcctgcacctatgccacagccctcaatgtagccagcctgagtgtggagcgctacttggccatctgccatcccttcaaggccaagaccctcatgt
cccgcagccgcaccaagaaattcatcagtgccatatggctagcctcggcgctgctggctgtacccatgcttttcaccatgggcctgcagaaccgcagtgc
cgatggccagcaccctggtggcctggtgtgcacacccacggtggacacagccaccgtcaaggtcgtcatccaggttaacaccttcatgtccttcctgtttc
ccatgctgatcatctccatcctaaacactgtgatagccaacaaactgaccgtcatggtgcaccaggctgccgagcagggccgtggtgtgtgcaccgtgg
gcacccacaacagtttagagcacagcacgttcaacatgtccatcgagccaggccgtgtccaggccctgcgccatggagtcctcgtcttacgtgctgtggt
aatcgcctttgtggtctgctggctgccctaccacgtgcggcgcctcatgttctgctatatctcagatgaacagtggactacgttcctcttcgacttctaccact
atttctatatgctgaccaacgctctcttctacgtcagctcagccatcaatcccatcctttacaacctcgtctccgccaacttccgccaggtcttcctgtccacac
tggcttgcctctgtcctgggtggcgccgccgccgaaagaagaggccaacgttctccaggaagccaaacagcatgtccagcaaccatgcgttttccacca
gcgccaccgggaaaccctgtactaggccatgaggaggtagcctgtgcacagggcagcaactcccccaaccccccaccccccaccccaaagcctccc
aaggttaagtagtggcccatctaagccatgccggtgaggctggggcaccctcagcggaggcttctttcttcccagtttctctttcttcccttcctctggttctc
ccctcctagtcctttattcttgcccctatccctctcctacctccacctttaaaaacagaaaagaggcggtttctctcctggccctacaaaaggcctttaacaa
gaagaaattagcactcaccaaggacggtctcctttgttcccagactaatggatgttttagaagcaagaaatgaaagcaccacattgggcctggacagatg
agctgttgtaaccataacagcaccttggaactgcactggcagggcgagacagtgtctgatgttggacttgggttcagagacacaggctccatctcagccc
ctttatgcctctactcctgccctggtccagcagataccatagcactctctgagccttatgtccagaccctttcttggcaggctggtacactgcccttcccaga
gtggtccagagaagcccccaaaattactgtgtaaggtccagggcccacagctggaagctgtgggaatccatgccacacctggatggctatggcccacta
aagagaccccaattcccacatgcccaggaaggataaaagggctggcctggaatcaacacaggacaagcttcaagtggctttgcacggggaccttcacc
tcttggatctgcagaaggatggaagataagtggggtccagcctccccagtccaggtggctttgctggggacatgcatggttctgcgtctcatatacagatg
tatagactaaggtctgacagcagctggcttcaggctgggacttctgagaaggggcactgagccaggtcctcagcaagatgccagtgtctgagactccac
aggtaaggggaggccaagcaccccagcatctagctggccagcagccctggactgaggcatatgtcaatttgttaaccagccgccaagcagccaccct
ggcccaggttctaggcgcctggaaaagcaggcacccttcatctctgggagctgtctccaaacaagacaggcccattcagcctccagccaacaagctgg
ctgggcacttggagacagtcccaggatgccatgcaccaacaaggtagaagtagcaggagagtcaagtgtagcctttgcaaaagagccttagctctgtt
gtctgggagagagatggctggacatgcagctgctctgtaaggatgaaattgccacgggacatggaggctcagtcacagcccaggctagcctctgctc
cctctgaactataaaacaatgggtgaaaagtcagggtatctgtggtcacccagagaaatggggagggtgttcaagtacttgaaccccacaaaacactgct
actagtaatgtgattcctcaggccatcaagggcaggacctgggtgagtccggatcaaggctgacaccaggaagatgtgatcacccaagagggaagcc
cctcctcctaagaaatgtctgcatgggagccctcacagccaccttgcccagtgagtgggtcaaaggctagcggttggtcatgaggattttgacttagact

caagccaggcagcccagccccaggactctaccctgggtttctagaccttaagccattcttctgagccttgatttcctcatctcggcggaccgggactgtctt
gaaggactcagcctggagtacacagaagtgggacacctgaggctgggtagagccaatgccctcttacatgattcctcagaaatgtggtggcctcattata
ggggtggtccaggcagtcaccagaagtctctgctgtagtctatagcatcttgtctgtgtgcttggtgtaattaggctacatacatccatgcaccaccagacac
acctgtgaccctggctgggtatcctatgctctgggccaagtgaagatgaaaatagttctggaggctgtcctgagcatcctggctagagtctcatgagccca
gtcgtgatgtggaaacaagtcgtgtgcccccaaatatctgtcagaatgccacccacccctccacacttcaataaaagaacaccctcctcatatgtctcaataaa
gaataaaggaagctgtgtatatcg

SEQ ID No.41

MHLNSSVQQGAPSEPGAQPFPHPQFGLETMLLALSLSNGSGNSSESILEPNSNLDVNTDIYSKVLV
TAVYLALFVVGTVGNSVTAFTLARKKSLQSLQSTVHYHLGSLALSDLLILLLAMPVELYNFIWV
HHPWAFGDAGCRGYYFLRDACTYATALNVASLSVERYLAICHPFKAKTLMSRSRTKKFISAIWL
ASALLAVPMLFTMGLQNRSADGQHPGGLVCTPTVDTATVKVVIQVNTFMSFLFPMLIISILNTVI
ANKLTVMVHQAAEQGRGVCTVGTHNSLEHSTFNMSIEPGRVQALRHGVLVLRAVVIAFVVCW
LPYHVRRLMFCYISDEQWTTFLFDFYHYFYMLTNALFYVSSAINPILYNLVSANFRQVFLSTLAC
LCPGWRRRRKKRPTFSRKPNSMSSNHAFSTSATRETLY

40

**Claims**

1. A method for identifying a compound capable to alter prolonged CRH exposure response in a cell, said method comprising;

    a) contacting said cell with CRH in the presence and absence of said compound;
    b) determine the amount of at least one protein that modulates corticotropin releasing hormone (CRH) signaling in said cell; and
    c) compare the amount of said protein in the presence and absence of said compound;

    whereby the protein that modulates corticotropin releasing hormone (CRH) signaling is SEQ ID NO.20.

2. A method according to claim 1 wherein the cell is a eukaryotic cell such as the murine pituitary corticotroph-derived adenoma cell line AtT-20.

3. A method according to claims 1 or 2 wherein the amount of protein that modulates CRH signaling is being determined using an antibody which binds to a polypeptide comprising an amino acid sequence SEQ ID NO.20.

4. A method according to claims 1 or 2 wherein the amount of protein that modulates CRH signaling is being determined by assessing the level of gene transcription of a gene encoding an amino acid sequence SEQ ID NO.20.

5. A method according to claim 4 wherein the level of gene transcription is being assessed using a probe which binds to a polynucleotide encoding amino acid sequence SEQ ID NO.20.

6. A method according to claims 4 or 5 wherein the level of gene expression is analysed using microarray technology.

7. A method according to claim 6 wherein the level of gene transcription is analysed using an array of oligonucleotide probes that bind to the polynucleotides encoding the group of polypeptides having the amino acid sequence SEQ ID NO.20.

8. A method according to claim 6 wherein the level of gene transcription is analysed using an array of oligonucleotide probes that bind to the polynucleotides having the nucleic acid sequence SEQ ID NO.19.

9. A method for identifying a compound capable to alter prolonged CRH exposure response in a cell, said method comprising;

    a) contacting a cell which expresses at least one protein comprising amino acid sequence SEQ ID NO.20, with said test compound; and
    b) compare the CRH response of said cell in the presence and absence of said compound.

10. A method according to claim 9 wherein the cell is a host cell capable to express at least one protein having amino acid sequence SEQ ID NO.20.

11. A method according to claim 10 wherein the host cell is transfected with at least one vector comprising a regulatory sequence.

12. A method according to claim 10 wherein the host cell is transfected with at least one vector comprising a polynucleotide sequence encoding amino acid sequence SEQ ID NO.20.

**Patentansprüche**

1. Verfahren zur Identifizierung einer Verbindung, die die Reaktion auf einen langen Kontakt mit CRH in einer Zelle verändern kann, wobei man in dem Verfahren

    a) die Zelle mit CRH in Gegenwart bzw. Abwesenheit der Verbindung in Kontakt bringt,
    b) die Menge wenigstens eines Proteins bestimmt, das die Signalgebung von CRH (Corticotropin Releasing Hormone) in der Zelle moduliert, und

c) die Menge des Proteins in Gegenwart bzw. Abwesenheit der Verbindung vergleicht;

womit es sich bei dem die Signalgebung von CRH (Corticotropin Releasing Hormone) modulierenden Protein um SEQ ID NO.20 handelt.

2. Verfahren nach Anspruch 1, wobei es sich bei der Zelle um eine eukaryontische Zelle, wie etwa die von ACTH-produzierenden Zellen der Hypophyse abgeleitete Adenom-Mauszelllinie AtT-20, handelt.

3. Verfahren nach Anspruch 1 oder 2, wobei die Menge an die CRH-Signalgebung modulierendem Protein unter Verwendung eines Antikörpers bestimmt wird, der an ein eine Aminosäuresequenz SEQ ID NO.20 umfassendes Polypeptid bindet.

4. Verfahren nach Anspruch 1 oder 2, wobei die Menge an die CRH-Signalgebung modulierendem Protein durch Beurteilen des Niveaus der Gentranskription eines für eine Aminosäuresequenz SEQ ID NO.20 codierenden Gens bestimmt wird.

5. Verfahren nach Anspruch 4, wobei das Niveau der Gentranskription unter Verwendung einer Sonde, die an ein für die Aminosäuresequenz SEQ ID NO.20 codierendes Polynukleotid bindet, beurteilt wird.

6. Verfahren nach Anspruch 4 oder 5, wobei das Niveau der Genexpression mittels Mikroarray-Technologie analysiert wird.

7. Verfahren nach Anspruch 6, wobei das Niveau der Gentranskription unter Verwendung eines Arrays von Oligonukleotidsonden, die an die für die Gruppe von Polypeptiden mit der Aminosäuresequenz SEQ ID NO. 20 codierenden Polynukleotide binden, analysiert wird.

8. Verfahren nach Anspruch 6, wobei das Niveau der Gentranskription unter Verwendung eines Arrays von Oligonukleotidsonden, die an die Polynukleotide mit der Nukleinsäuresequenz SEQ ID NO. 19 binden, analysiert wird.

9. Verfahren zur Identifizierung einer Verbindung, die die Reaktion auf einen langen Kontakt mit CRH in einer Zelle verändern kann, wobei man in dem Verfahren

a) eine Zelle, die wenigstens ein die Aminosäuresequenz SEQ ID NO.20 umfassendes Protein exprimiert, mit der Testverbindung in Kontakt bringt,
b) die CRH-Reaktion der Zelle in Gegenwart bzw. Abwesenheit der Verbindung vergleicht.

10. Verfahren nach Anspruch 9, wobei es sich bei der Zelle um eine Wirtszelle handelt, die in der Lage ist, wenigstens ein Protein mit der Aminosäuresequenz SEQ ID NO.20 zu exprimieren.

11. Verfahren nach Anspruch 10, wobei die Wirtszelle mit wenigstens einem eine regulatorische Sequenz umfassenden Vektor transfiziert wird.

12. Verfahren nach Anspruch 10, wobei die Wirtszelle mit wenigstens einem eine für die Aminosäuresequenz SEQ ID NO.20 codierende Polynukleotidsequenz umfassenden Vektor transfiziert wird.

**Revendications**

1. Méthode d'identification d'un composé susceptible de modifier la réponse à une exposition prolongée à la CRH dans une cellule, ladite méthode comprenant :

a) la mise en contact de ladite cellule avec la CRH en présence ou en l'absence dudit composé ;
b) la détermination de la quantité d'au moins une protéine qui module la signalisation de l'hormone de libération de la corticotropine (CRH) dans ladite cellule ; et
c) la comparaison de la quantité de ladite protéine en présence ou en l'absence dudit composé ;

la protéine qui module la signalisation de l'hormone de libération de la corticotropine (CRH) étant SEQ ID NO :20.

**2.** Méthode selon la revendication 1, **caractérisée en ce que** la cellule est une cellule eucaryote telle que la lignée cellulaire d'adénome AtT-20 hypophysaire corticotrope murin.

**3.** Méthode selon la revendication 1 ou 2, **caractérisée en ce que** la quantité de protéine qui module la signalisation de CRH est déterminée en utilisant un anticorps qui se lie à un polypeptide comprenant une séquence d'acides aminés SEQ ID NO : 20.

**4.** Méthode selon la revendication 1 ou 2, **caractérisée en ce que** la quantité de protéine qui module la signalisation de CRH est déterminée en évaluant le taux de transcription génique d'un gène codant pour une séquence d'acides aminés SEQ ID NO : 20.

**5.** Méthode selon la revendication 4, **caractérisée en ce que** le taux de transcription génique est évalué en utilisant une sonde qui se lie à un polynucléotide codant pour une séquence d'acides aminés SEQ ID NO : 20.

**6.** Méthode selon la revendication 4 ou 5, **caractérisée en ce que** le taux d'expression génique est analysé en utilisant la technologie de puce à ADN.

**7.** Méthode selon la revendication 6, **caractérisée en ce que** le taux de transcription génique est analysé en utilisant une matrice de sondes oligonucléotidiques qui se lient aux polynucléotides codant pour le groupe de polypeptides ayant les séquences d'acides aminés SEQ ID NO : 20.

**8.** Méthode selon la revendication 6, **caractérisée en ce que** le taux de transcription génique est analysé en utilisant une matrice de sondes oligonucléotidiques qui se lient aux polynucléotides ayant la séquence d'acides nucléiques SEQ ID NO : 19.

**9.** Méthode d'identification d'un composé susceptible de modifier la réponse à une exposition prolongée à la CRH dans une cellule, ladite méthode comprenant :

a) la mise en contact d'une cellule qui exprime au moins une protéine comprenant la séquence d'acides aminés SEQ ID NO : 20, avec ledit composé à tester ; et
b) la comparaison de la réponse à la CRH de ladite cellule en présence ou en l'absence dudit composé.

**10.** Méthode selon la revendication 9, **caractérisé en ce que** la cellule est une cellule hôte susceptible d'exprimer au moins une protéine ayant la séquence d'acides aminés SEQ ID NO : 20.

**11.** Méthode selon la revendication 10, **caractérisée en ce que** la cellule hôte est transfectée avec au moins un vecteur comprenant une séquence de régulation.

**12.** Méthode selon la revendication 10, **caractérisée en ce que** la cellule hôte est transfectée avec au moins un vecteur comprenant une séquence polynucléotidique codant pour la séquence d'acides aminés SEQ ID NO : 20.

Table 1

| | Gene Name | CDS | SEQ ID | | Title | Sequence Derived from |
|---|---|---|---|---|---|---|
| | | | NA | AA | | |
| down | 1700015E05Rik | | 1 | | RIKEN cDNA 1700015E05 gene | AB42377 |
| down | 2300002F06Rik | | 2 | | RIKEN cDNA 2300002F06 gene | AW123564 |
| down | 2610008LD4Rik | | 3 | | RIKEN cDNA 2610008LD4 gene | AB46337 |
| down | 2610511G02Rik | | 4 | | RIKEN cDNA 2610511G02 gene | AV221664 |
| down | 4931430ID1Rik | | 5 | | RIKEN cDNA 4931430ID1 gene | AB26942 |
| down | AI414047 | | 6 | | expressed sequence AI414047 | AW125649 |
| down | Genera:47634 | | 7 | | ESTs, Moderately similar to VIS3 MOUSE VISININ-LIKE PROTEIN 3 [M.musculus] | C80148 |
| down | | | 8 | | ESTs, Moderately similar to VIS3 MOUSE VISININ-LIKE PROTEIN 3 [M.musculus] | AB43393 |
| | | | | | | |
| down | B-FABP | | 9 | | Brain Fatty acid-binding protein gene | U04827 |
| down | B-FABP | | | 10 | Brain Fatty acid-binding protein | AAA81904 |
| down | Edg2 | 214-1308 | 11 | 12 | endothelial differentiation, lysophosphatidic acid G-protein-coupled receptor, 2 | U13370 |
| down | Fgfr2 | 1-1038 | 13 | 14 | fibroblast growth factor receptor 2 | M23362 |
| down | Gab1 | 24-2111 | 15 | 16 | growth factor receptor bound protein 2-associated protein 1 | AID46826 |
| down | Hpcal1 | 261-842 | 17 | 18 | hippocalcin-like 1 | AF085192 |
| down | Hsd11b1 | 128-1006 | 19 | 20 | hydroxysteroid 11-beta dehydrogenase 1 | X83202 |
| down | Ntsr2 | 27-1280 | 21 | 22 | neurotensin receptor 2 | U51908 |
| down | Ntsr1 | 444-1718 | 40 | 41 | neurotensin receptor 1 | NM_01876 |
| down | Ywhaq | 21-758 | 23 | 24 | tyrosine 3-monooxygenase/tryptophan 5-monooxygenase activation protein, theta polypeptide | U56243 |
| | | | | | | |
| up | 1110014J22Rik | | 25 | | RIKEN cDNA 1110014J22 gene | AB52646 |
| up | 1200008D14Rik | | 26 | | RIKEN cDNA 1200008D14 gene | AW208938 |
| up | 1600023A02Rik | | 27 | | RIKEN cDNA 1600023A02 gene | AV019597 |
| up | 3110003A17Rik | | 28 | | RIKEN cDNA 3110003A17 gene | AA833425 |
| up | 3110023F10Rik | | 29 | | RIKEN cDNA 3110023F10 gene | AI195392 |
| up | AI467657 | | 30 | | expressed sequence AI467657 | AI553024 |
| up | AI480570 | | 31 | | expressed sequence AI480570 | AB46545 |
| | | | | | | |
| up | Fkbp5 | 220-1590 | 32 | 33 | FK506 binding protein 5 (51 kDa) | U16959 |
| up | Pdyn | 1-747 | 34 | 35 | prodynorphin | AV382264 |
| up | Penk1 | 297-1106 | 36 | 37 | preproenkephalin 1 | M55181 |
| up | Sgk | 69-1364 | 38 | 39 | serum/glucocorticoid regulated kinase | AW046181 |

Fig. 1

Fig. 2A

Spectralmap Cerebellum

Repres. = 47 % + 12%, Closure = none; Center = double; Norm. = global, Scale = uvc, RW = mean, CW = mean

PC 1

PC2

Fig. 2B

Fig. 2C

EP 1 558 760 B1

**Spectralmap Hippocampus**

PC 1

Repres. = 25% + 13%, Closure = none; Center = double; Norm. = global, Scale = uvc, R/W = mean, C/W = mean

Fig. 2D

EP 1 558 760 B1

**D**

### Spectralmap Nucleus Accumbens

PC 1

Repres. = 22 % + 12%; Closure = none; Center = double; Norm. = global, Scale = uvc, RW = mean, CW = mean

PC2

Fig. 2E

EP 1 558 760 B1

**E**

## Spectralmap Temporal Area

PC 1

Repres. = 19 % + 13%, Closure = none, Center = double; Norm. = global, Scale = uvc, R/W = mean, C/W = mean

Fig. 2F

Spectralmap Pituitary

PC 1

Repres. = 28 % + 10%, Closure = none, Center= double, Norm = global, Scale = uic, Row = mean, Col = mean

PC2

Fig. 3

Fig. 4

Fig.5

Ntsr2 (array data hippocampus)

Ntsr2 (RTq data hippocampus)

Ntsr1 (RTq data hippocampus)

Fig.6

Fig.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4946778 A
- WO 9429458 A
- WO 9422914 A
- WO 9932619 A

### Non-patent literature cited in the description

- **Stenzel-Poore et al.** *Endocrinology,* 1992, vol. 130, 3378-3386
- **Stenzel-Poore et al.** *J.Neurosci.,* 1994, vol. 14, 2579-2584
- **M. Dayhoff.** Atlas of Protein Sequence and Structure. 1978, vol. 5, 345-352
- **M.Chee et al.** *Science,* 1996, vol. 274, 610-613
- PCR Protocols: A Guide to Method and Application. Academic Press, 1990
- **Kohler, G. ; Milstein, C.** *Nature,* 1975, vol. 256, 495-497
- **Kozbor et al.** *Immunology Today,* 1983, vol. 4 (72
- **Cole et al.** MONOCLONAL ANTIBODIES AND CANCER THERAPY. Alan R. Liss, Inc, 1985, 77-96
- **Sambrook et al.** Molecular Cloning: A Laboratory Manual. Cold Spring Harbor Laboratory, 1989
- Methods Enzymol. Academic Press, Inc, 1990, vol. 185
- Computational Molecular Biology. Oxford University Press, 1988
- Biocomputing: Informatics and Genome Projects. Academic Press, 1993
- Computer Analysis of Sequence Data, Part I. Humana Press, 1994
- **Heinje, G.** Sequence Analysis in Molecular Biology. Academic Press, 1987
- Sequence Analysis Primer. M Stockton Press, 1991
- **Carillo, H. ; Lipman, D.** *SIAM J. Applied Math.,* 1988, vol. 48, 1073
- **Devereux, J. et al.** *Nucleic Acids Research,* 1984, vol. 12 (1), 387
- **Atschul, S. F. et al.** *J. Molec. Biol.,* 1990, vol. 215, 403
- **T. Maniatis et al.** Molecular Cloning: A Laboratory Manual. 1989
- **Frohman et al.** *PNAS USA,* 1988, vol. 85, 8998-9002
- Site Specific Proteolysis of Fusion Proteins. **P.Carter.** Protein Purification: From Molecular Mechanisms to Large Scale Processes. American Chemical Society, 1990
- **Davis.** *Basic Methods In Molecular Biology,* 1986
- Gene Targeting, A Practical Approach. Oxford University Press, 1993
- **Gossen ; Bujard.** *Proc. Natl. Acad. Sci. 89 USA,* 1992, vol. 89, 5547-5551
- **Gossen et al.** *Trends Biotech.,* 1994, vol. 12, 58-62
- **Myers et al.** *Science,* 1985, vol. 230, 1242
- **Cotton et al.** *Proc Natl Acad Sci USA,* 1985, vol. 85, 4397-4401
- **Kozbor et al.** *Immunology Today,* 1983, vol. 4, 72
- **O'Connor.** *J. Neurochem,* 1991, vol. 56, 560
- Oligodeoxynucleotides as Antisense Inhibitors of Gene Expression. CRC Press, 1988
- **Lee et al.** *Nucleic Acids Res,* 1979, vol. 6, 3073
- **Cooney et al.** *Science,* 1988, vol. 241, 456
- **Dervan et al.** *Science,* 1991, vol. 251, 1360
- **Usman, N et al.** *Curr. Opin. Struct. Biol,* 1996, vol. 6 (4), 527-33
- **T Strachan ; A P Read.** Human Molecular Genetics. BIOS Scientific Publishers Ltd, 1996
- **De Souza EB.** Corticotropin-releasing factor receptors: physiology, pharmacology, biochemistry and role in central nervous system and immune disorders. *Psychoneuroendocrinology,* 1995, vol. 20, 789-819
- **Holsboer F.** Stress, hypercortisolism and corticosteroid receptors in depression: implications for therapy. *J Affect Disord,* 2001, vol. 62, 77-91
- **Holsboer F ; Gerken A ; Stalla GK ; Muller OA.** Blunted aldosterone and ACTH release after human CRH administration in depressed patients. *Am J Psychiatry,* 1987, vol. 144, 229-231

- **Holsboer F ; Gerken A ; von Bardeleben U ; Grimm W ; Beyer H ; Muller OA ; Stalla GK.** Human corticotropin-releasing hormone in depression--correlation with thyrotropin secretion following thyrotropin-releasing hormone. *Biol Psychiatry,* 1986, vol. 21, 601-611
- **Nemeroff CB ; Owens MJ ; Bissette G ; Andorn AC ; Stanley M.** Reduced corticotropin releasing factor binding sites in the frontal cortex of suicide victims. *Arch Gen Psychiatry,* 1988, vol. 45, 577-579
- **Raadsheer FC ; Hoogendijk WJ ; Stam FC ; Tilders FJ ; Swaab DF.** Increased numbers of corticotropin-releasing hormone expressing neurons in the hypothalamic paraventricular nucleus of depressed patients. *Neuroendocrinology,* 1994, vol. 60, 436-444
- **Wouters L ; Göhlmann HW ; Bijnens L ; Kass SU ; Molenberghs G ; Lewi PJ.** Graphical exploration of gene expression data:a comparative study of three multivariate methods. *Biometrics*
- **Tusher VG ; Tibshirani R ; Chu G.** Significance analysis of microarrays applied to the ionizing radiation response. *Proc Natl Acad Sci U S A,* 2001, vol. 98, 5116-5121
- **Van Pett K ; Viau V ; Bittencourt JC ; Chan RK ; Li HY ; Arias C ; Prins GS ; Perrin M ; Vale W ; Sawchenko PE.** Distribution of mRNAs encoding CRF receptors in brain and pituitary of rat and mouse. *J Comp Neurol,* 2000, vol. 428, 191-212
- **Tsai KJ ; Chen SK ; Ma YL ; Hsu WL ; Lee EH.** sgk, a primary glucocorticoid-induced gene, facilitates memory consolidation of spatial learning in rats. *Proc Natl Acad Sci U S A %,* vol. 19 (99), 3990-3995
- **Heinrichs SC ; Stenzel-Poore MP ; Gold LH ; Battenberg E ; Bloom FE ; Koob GF ; Vale WW ; Pich EM.** Learning impairment in transgenic mice with central overexpression of corticotropin-releasing factor. *Neuroscience,* 1996, vol. 74, 303-311
- **Moyse E ; Rostene W ; Vial M ; Leonard K ; Mazella J ; Kitabgi P ; Vincent JP ; Beaudet A.** Distribution of neurotensin binding sites in rat brain: a light microscopic radioautographic study using monoiodo [125I]Tyr3-neurotensin. *Neuroscience,* 1987, vol. 22, 525-536
- **Nicot A ; Berod A ; Gully D ; Rowe W ; Quirion R ; de Kloet ER ; Rostene W.** Blockade of neurotensin binding in the rat hypothalamus and of the central action of neurotensin on the hypothalamic-pituitary-adrenal axis with non-peptide receptor antagonists. *Neuroendocrinology,* 1994, vol. 59, 572-578
- **Scarceriaux V ; Pelaprat D ; Forgez P ; Lhiaubet AM ; Rostene W.** Effects of dexamethasone and forskolin on neurotensin production in rat hypothalamic cultures. *Endocrinology,* 1995, vol. 136, 2554-2560
- **Souaze F ; Rostene W ; Forgez P.** Neurotensin agonist induces differential regulation of neurotensin receptor mRNA. Identification of distinct transcriptional and post-transcriptional mechanisms. *J Biol Chem,* 1997, vol. 272, 10087-10094
- **Azzi M ; Betancur C ; Sillaber I ; Spangel R ; Rostene W ; Berod A.** Repeated administration of the neurotensin receptor antagonist SR 48692 differentially regulates mesocortical and mesolimbic dopaminergic systems. *J Neurochem,* 1998, vol. 71, 1158-1167
- **Lepee-Lorgeoux I ; Betancur C ; Souaze F ; Rostene W ; Berod A ; Pelaprat D.** Regulation of the neurotensin NT(1) receptor in the developing rat brain following chronic treatment with the antagonist SR 48692. *J Neurosci Res,* 2000, vol. 60, 362-369
- **Rowe W ; Viau V ; Meaney MJ ; Quirion R.** Stimulation of CRH-mediated ACTH secretion by central administration of neurotensin: evidence for the participation of the paraventricular nucleus. *J Neuroendocrinol,* 1995, vol. 7, 109-117